(19) Europäisches Patentamt
European Patent Office
Office européen des brevets

(11) EP 4 789 671 A1

(12) EUROPEAN PATENT APPLICATION
published in accordance with Art. 153(4) EPC

(43) Date of publication:
**12.08.2026 Bulletin 2026/33**

(21) Application number: **24832161.4**

(22) Date of filing: **04.10.2024**

(51) International Patent Classification (IPC):
***A61K 31/513*** *(2006.01)* ***A61K 31/506*** *(2006.01)*
***A61P 31/14*** *(2006.01)* ***A61P 43/00*** *(2006.01)*
***C07D 401/14*** *(2006.01)*

(52) Cooperative Patent Classification (CPC):
**A61K 31/506; A61K 31/513; A61P 31/14; A61P 43/00; C07D 401/14**

(86) International application number:
**PCT/JP2024/035560**

(87) International publication number:
**WO 2025/005306 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.10.2023 JP 2023173420**
**14.06.2024 JP 2024096866**

(71) Applicant: **Shionogi & Co., Ltd**
**Osaka-shi, Osaka 541-0045 (JP)**

(72) Inventors:
- **SATO, Jun**
**Osaka-shi, Osaka 541-0045 (JP)**
- **SHIBAYAMA, Hiromitsu**
**Osaka-shi, Osaka 541-0045 (JP)**
- **UEHARA, Shota**
**Osaka-shi, Osaka 541-0045 (JP)**
- **UNOH, Yuto**
**Osaka-shi, Osaka 541-0045 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) PHARMACEUTICAL COMPOSITION CONTAINING URACIL DERIVATIVE

(57) The present invention provides a pharmaceutical composition comprising a compound exhibiting coronavirus proliferation inhibitory activity.

A pharmaceutical composition comprising a compound represented by Formula (I-1):

CN
O
Cl
N
N
N
O
F
F
Cl
F

(I-1)

, or a pharmaceutically acceptable salt thereof.

Fig. 1

VIRAL TITER IN MOUSE LUNG HOMOGENATE
Lung viral titter ($log_{10}$ $TCID_{50}$/mL)
8.00
7.00
6.00
5.00
4.00
3.00
2.00
1.00
0.00
**
***
***
***
Vehicle
0.1 mg/kg
0.3 mg/kg
1 mg/kg
3 mg/kg
10 mg/kg
COMPOUND (I-1)
p** p < 0.01, *** p < 0.0001 vs vehicle (Dunnett's test)



Processed by Luminess, 75001 PARIS (FR)

## Description

Technical Field

The present invention provides a pharmaceutical composition comprising a compound exhibiting coronavirus 3CL protease inhibitory activity.

Background Art

Nidovirales order, Coronaviridae family, and Orthocoronavirinae subfamily coronaviruses have a genome size of about 30 kilobases and are the largest single-stranded positive-sense RNA viruses among known RNA viruses. Coronaviruses are classified into four genera: Alphacoronavirus, Betacoronavirus, Gammacoronavirus, and Deltacoronavirus, and seven coronaviruses are known to infect humans, including two alphacoronaviruses (HCoV-229E, and HCoV-NL63) and five betacoronaviruses (HCoV-HKU1, HCoV-OC43, SARS-CoV, MERS-CoV, and SARS-CoV-2). Of these, four coronaviruses (HCoV-229E, HCoV-NL63, HCoV-HKU1, and HCoV-OC43) are causative agents of the common cold, whereas the remaining three are severe acute respiratory syndrome (SARS) coronavirus (SARS-CoV), Middle East respiratory syndrome (MERS) coronavirus (MERS-CoV), and novel coronavirus (SARS-CoV-2), which are known to cause severe pneumonia.

Novel coronavirus infection (COVID-19), which emerged in December 2019, rapidly spread throughout the international community, and the WHO declared it a pandemic on March 11, 2020. The main routes of transmission of SARS-CoV-2 have been reported to include droplet transmission, contact transmission, and aerosol transmission, and it has been confirmed that SARS-CoV-2 can remain suspended in the air together with aerosols for approximately three hours while retaining infectivity (Non Patent Document 1). The incubation period is approximately 2 to 14 days, and typical symptoms include fever (87.9%), dry cough (67.7%), fatigue (38.1%), and sputum production (33.4%), which are common cold-like symptoms (Non Patent Document 2). In severe cases, respiratory failure due to acute respiratory distress syndrome, acute lung injury, interstitial pneumonia, and the like occurs. Furthermore, multiple organ failure such as renal failure and hepatic failure has also been reported.

In Japan, as a result of drug repositioning of existing drugs, remdesivir, which is an antiviral drug, dexamethasone, which is an anti-inflammatory drug, and baricitinib, which is an antirheumatic drug, have been approved as therapeutic drugs against COVID-19, and in January 2022, tocilizumab, which is an anti-IL-6 receptor antibody, has received additional approval. Furthermore, in July 2021, Ronapreve (casirivimab/imdevimab), an antibody cocktail therapy, was granted special approval, followed by the special approval of sotrovimab in September 2021 and molnupiravir in December 2021. Sufficient evidence has not been obtained on the efficacy and safety of these drugs. Accordingly, it is imperative to create therapeutic drugs against COVID-19.

When coronaviruses infect cells, they synthesize two polyproteins. These two polyproteins contain a replication complex generating a viral genome, as well as two proteases. The proteases cleave the virally synthesized polyproteins and play an essential role in enabling each protein to function. Among the two proteases, the 3CL protease (main protease) is responsible for most of the polyprotein cleavage (Non Patent Document 3).

As a COVID-19 therapeutic drug targeting 3CL proteases, completion of the Phase 1b trial of Lufotrelvir (PF-07304814), a prodrug of PF-00835231 by Pfizer was published on ClinicalTrials.gov in June 2021 (NCT04535167). In March 2021, Pfizer announced that it will begin a Phase 1 trial of PF-07321332, a therapeutic drug for novel coronavirus infection. The structural formulae of PF-00835231, Lufotrelvir and PF-07321332 are as shown below and differ in chemical structure from the compounds used in the present invention (Non Patent Documents 4, 8 and 9; and Patent Documents 1 and 2).

PF-00835231:

[Chemical formula 1]

Lufotrelvir(PF-07304814):

[Chemical formula 2]

PF-07321332:

[Chemical formula 3]

In December 2021, PAXLOVID (TM) was approved for emergency use in the United States, and on February 10, 2022, the Paxlovid (registered trademark) PACK was approved as special case approval in Japan.

Furthermore, regarding COVID-19 therapeutic drugs targeting 3CL proteases, the start of Phase 1 trials for PBI-0451 by Pardes Biosciences was reported in ClinicalTrials.gov in August 2021 (NCT05011812). The structural formula of PBI-0451 is as shown below, and these agents are different from the compound used in the present invention in chemical structure (Non Patent Document 12).

[Chemical formula 4]

Furthermore, Xocova (registered trademark) was granted emergency approval in Japan on November 22, 2022 as a COVID-19 therapeutic drug targeting 3CL protease and was granted standard approval on March 5, 2024 (Non Patent Documents 17 and 18).

The active ingredient of Xocova is ensitrelvir fumaric acid, and the structural formula thereof is as shown below and is different in chemical structure from the compound of the present invention (Patent Documents 10 to 13).

[Chemical formula 5]

No sufficient evidence has been obtained for mutations resistant to COVID-19 therapeutic drugs targeting 3CL proteases.

Compounds having 3CL protease inhibitory activity are disclosed in Non Patent Documents 4 to 7 and 13 to 16; however, the compounds related to the present invention are neither described nor suggested in any of the documents. Compounds having 3CL protease inhibitory activity are disclosed in Patent Documents 14 and 15; however, the pharmaceutical composition comprising the compound according to the present invention is neither described nor suggested in any of the documents.

Compounds having $P2X_3$ and/or $P2X_{2/3}$ receptor inhibitory activity have been disclosed in Patent Documents 3 to 9; however, the 3CL protease inhibitory activity and the antiviral effect are neither described nor suggested in any of the documents.

Although compounds having an inhibitory action on HIV-1 reverse transcriptase are disclosed in Non Patent Document 11, 3CL protease inhibitory activity and anti-coronavirus effect have neither been described nor suggested.

Prior Art Documents

Patent Documents

Patent Document 1: International Publication WO2021/205298
Patent Document 2: International Publication WO2021/250648
Patent Document 3: International Publication WO2012/020742
Patent Document 4: International Publication WO2013/118855
Patent Document 5: China Patent Application Publication CN113620888
Patent Document 6: China Patent Application Publication CN113666914
Patent Document 7: China Patent Application Publication CN113735838A
Patent Document 8: Chinese Patent Application Publication CN113773300
Patent Document 9: Chinese Patent Application Publication CN113801097
Patent Document 10: International Publication WO2022/138987
Patent Document 11: International Publication WO2022/138988
Patent Document 12: International Publication WO2023/054292
Patent Document 13: International Publication WO2023/054732
Patent Document 14: International Publication WO2023/195529
Patent Document 15: International Publication WO2023/195530

Non Patent Documents

Non Patent Document 1: The NEW ENGLAND JOURNAL of MEDICINE (2020), Vol. 382, pp. 1564-1567
Non Patent Document 2: "Report of the WHO-China Joint Mission on Coronavirus Disease 2019 (COVID-19)", [online], February 28, 2020, WHO, [retrieved on March 16, 2023], Internet <URL: https://www.who.int/docs/default-source/coronaviruse/who-china-joint-mission-on-covid-19-final-report.pdf>
Non Patent Document 3: Science (2003), Vol. 300, pp. 1763-1767 Non Patent Document 4: "A comparative analysis of SARS-CoV-2 antivirals characterizes 3CLpro inhibitor PF-00835231 as a potential new treatment for COVID-19", Journal of Virology, 2021 Mar 10; 95(7), e01819-20
Non Patent Document 5: Cell Research (2020), Vol. 30, pp. 678-692
Non Patent Document 6: Science (2020), Vol. 368, pp. 409-412
Non Patent Document 7: ACS Central Science (2021), Vol. 7, No. 3, pp. 467-475

Non Patent Document 8: 261st Am Chem Soc (ACS) Natl Meet · 2021-04-05 / 2021-04-16 · Virtual, N/A · Abst 243
Non Patent Document 9: Science (2021), Vol. 374, pp. 1586-1593
Non Patent Document 10: "Pfizer's Novel COVID-19 Oral Antiviral Treatment Candidate Reduced Risk Of Hospitalization Or Death By 89% In Interim Analysis Of Phase 2/3 EPIC-HR Study", [online], November 5, 2021, Pfizer Press Release, [retrieved on March 16, 2023], Internet <URL: https://www.pfizer.com/news/press-release/press-release-detail/pfizers-novel-covid-19-oral-antiviral-treatment-candidate>
Non Patent Document 11: Synthetic Communications (2006), Vol. 36, No. 19, pp. 2913-2920
Non Patent Document 12: "Discovery and Development of PBI-0451", [online], March 24, 2022, 35th International Conference on Antiviral Research (ICAR), [retrieved on March 16, 2023], Internet <URL: https://ir.pardesbio.com/static-files/fc7c4f8c-e0bd-4b97-8c9c-eff09bafd4db>
Non Patent Document 13: Molecules (2020), vol. 25, p. 3193
Non Patent Document 14: Molecules (2020), vol. 25, p. 3920
Non Patent Document 15: European Journal of Medicinal Chemistry (2020), Vol. 206, p. 112711
Non Patent Document 16: Journal of the American Chemical Society (2022), vol. 144, pp. 2905-2920
Non Patent Document 17: Shionogi & Co., Ltd. Press Release (November 22, 2022)
Non Patent Document 18 Shionogi & Co., Ltd. Press Release (March 5, 2024)

SUMMARY OF THE INVENTION

TECHNICAL PROBLEMS TO BE SOLVED BY THE INVENTION

An object of the present invention is to provide a pharmaceutical composition comprising a compound having coronavirus 3CL protease inhibitory activity. Preferably, the present invention provides a pharmaceutical composition comprising a compound having an antiviral activity, particularly a coronavirus proliferation inhibitory activity.

MEANS FOR SOLVING THE PROBLEM

The present invention relates to the following.

(1) A pharmaceutical composition comprising a compound represented by Formula (I-1):

[Chemical formula 6]

(I-1)

, or a pharmaceutically acceptable salt thereof.
(2) The pharmaceutical composition according to the above item (1), wherein the pharmaceutical composition is a 3CL protease inhibitor.
(3) The pharmaceutical composition according to the above item (1) or (2), wherein the pharmaceutical composition is for use in inhibition of viral proliferation of SARS-CoV-2.
(4) The pharmaceutical composition according to any one of the above items (1) to (3), wherein the pharmaceutical composition is a therapeutic agent and/or a prophylactic agent for novel coronavirus infection (COVID-19).
(5) The pharmaceutical composition according to any one of the above items (1) to (4), wherein the pharmaceutical composition is for use in suppression of exacerbation of SARS-CoV-2 infection.
(6) The pharmaceutical composition according to any one of the above items (1) to (4), wherein the pharmaceutical composition is used so that administration is initiated within 72 hours after the onset of symptoms of SARS-CoV-2 infection or after a positive diagnosis of SARS-CoV-2.
(7) The pharmaceutical composition according to any one of the above items (1) to (4), wherein the pharmaceutical composition is used so that administration is initiated within 24 hours after the onset of symptoms of SARS-CoV-2

infection or after a positive diagnosis of SARS-CoV-2.

(8) The pharmaceutical composition according to any one of the above items (1) to (4), wherein the pharmaceutical composition is used so that administration is initiated within 120 hours after the onset of symptoms of SARS-CoV-2 infection or after a positive diagnosis of SARS-CoV-2.

(9) The pharmaceutical composition according to any one of the above items (1) to (4), wherein the pharmaceutical composition is used so that administration is initiated within 48 hours after the onset of symptoms of SARS-CoV-2 infection or after a positive diagnosis of SARS-CoV-2.

(10) The pharmaceutical composition according to any one of the above items (1) to (4), wherein the pharmaceutical composition is for use in suppression of viral transmission of SARS-CoV-2.

(11) The pharmaceutical composition according to any one of the above items (1) to (4), wherein the symptoms of the SARS-CoV-2 infection are mild or moderate I symptoms.

(12) A method for inhibiting viral proliferation of SARS-CoV-2, comprising administering a compound represented by Formula (I-1):

[Chemical formula 7]

(I-1)

, or a pharmaceutically acceptable salt thereof, to an individual in need of treatment and/or prevention of novel coronavirus infection (COVID-19).

(13) A method of treating and/or preventing novel coronavirus infection (COVID-19), comprising administering a compound represented by Formula (I-1):

[Chemical formula 8]

(I-1)

, or a pharmaceutically acceptable salt thereof, to an individual in need of treatment and/or prevention of the novel coronavirus infection (COVID-19).

(14) A method of suppressing exacerbation of SARS-CoV-2 infection, comprising administering a compound represented by Formula (I-1):

[Chemical formula 9]

(I-1)

, or a pharmaceutically acceptable salt thereof, to an individual in need of treatment and/or prevention of novel coronavirus infection (COVID-19).

(15) A method of treating novel coronavirus infection (COVID-19), comprising administering a compound represented by Formula (I-1):

[Chemical formula 10]

(I-1)

, or a pharmaceutically acceptable salt thereof, to an individual in need of treatment of the novel coronavirus infection (COVID-19) within 72 hours after the onset of symptoms of SARS-CoV-2 infection or after a positive diagnosis of SARS-CoV-2.

(16) A method of treating novel coronavirus infection (COVID-19), comprising administering a compound represented by Formula (I-1):

[Chemical formula 11]

(I-1)

, or a pharmaceutically acceptable salt thereof, to an individual in need of treatment of the novel coronavirus infection (COVID-19) within 24 hours after the onset of symptoms of SARS-CoV-2 infection or after a positive diagnosis of SARS-CoV-2.

(17) A method of treating novel coronavirus infection (COVID-19), comprising administering a compound represented by Formula (I-1):

[Chemical formula 12]

(I-1)

, or a pharmaceutically acceptable salt thereof, to an individual in need of treatment of the novel coronavirus infection (COVID-19) within 120 hours after the onset of symptoms of SARS-CoV-2 infection or after a positive diagnosis of SARS-CoV-2.

(18) A method of treating novel coronavirus infection (COVID-19), comprising administering a compound represented by Formula (I-1):

[Chemical formula 13]

(I-1)

, or a pharmaceutically acceptable salt thereof, to an individual in need of treatment of the novel coronavirus infection (COVID-19) within 48 hours after the onset of symptoms of SARS-CoV-2 infection or after a positive diagnosis of SARS-CoV-2.

(19) A method of suppressing viral transmission of SARS-CoV-2, comprising administering a compound represented by Formula (I-1):

[Chemical formula 14]

(I-1)

, or a pharmaceutically acceptable salt thereof, to an individual in need of treatment of novel coronavirus infection (COVID-19).

(20) A method of treating novel coronavirus infection (COVID-19), comprising administering a compound represented by Formula (I-1):

[Chemical formula 15]

(I-1)

, or a pharmaceutically acceptable salt thereof, to an individual with mild or moderate I symptoms in need of treatment of novel coronavirus infection (COVID-19).

(21) Use of a compound represented by Formula (I-1):

[Chemical formula 16]

(I-1)

, or a pharmaceutically acceptable salt thereof for inhibition of viral proliferation of SARS-CoV-2.

(22) Use of a compound represented by Formula (I-1):

[Chemical formula 17]

(I-1)

, or a pharmaceutically acceptable salt thereof for manufacturing a medicament for treatment and/or prevention of novel coronavirus infection (COVID-19).

(23) Use of a compound represented by Formula (I-1):

[Chemical formula 18]

(I-1)

, or a pharmaceutically acceptable salt thereof for suppression of exacerbation of SARS-CoV-2 infection.

(23') Use of a compound represented by Formula (I-1):

[Chemical formula 19]

(I-1)

, or a pharmaceutically acceptable salt thereof for manufacturing a medicament for suppression of exacerbation of novel coronavirus infection (COVID-19).

(24) Use of a compound represented by Formula (I-1):

[Chemical formula 20]

(I-1)

, or a pharmaceutically acceptable salt thereof for administration within 72 hours after the onset of symptoms of SARS-CoV-2 infection or after a positive diagnosis of SARS-CoV-2.

(24') Use of a compound represented by Formula (I-1):

[Chemical formula 21]

(I-1)

, or a pharmaceutically acceptable salt thereof for manufacturing a medicament for treatment of novel coronavirus infection (COVID-19), wherein the medicament is administered within 72 hours after the onset of symptoms of SARS-CoV-2 infection or after a positive diagnosis of SARS-CoV-2.

(25) Use of a compound represented by Formula (I-1):

[Chemical formula 22]

(I-1)

, or a pharmaceutically acceptable salt thereof for administration within 24 hours after the onset of symptoms of SARS-CoV-2 infection or after a positive diagnosis of SARS-CoV-2.

(25') Use of a compound represented by Formula (I-1):

[Chemical formula 23]

(I-1)

, or a pharmaceutically acceptable salt thereof for manufacturing a medicament for treatment of novel coronavirus infection (COVID-19), wherein the medicament is administered within 24 hours after the onset of symptoms of SARS-CoV-2 infection or after a positive diagnosis of SARS-CoV-2.

(26) Use of a compound represented by Formula (I-1):

[Chemical formula 24]

(I-1)

, or a pharmaceutically acceptable salt thereof for administration within 120 hours after the onset of symptoms of SARS-CoV-2 infection or after a positive diagnosis of SARS-CoV-2.

(26') Use of a compound represented by Formula (I-1):

[Chemical formula 25]

(I-1)

, or a pharmaceutically acceptable salt thereof for manufacturing a medicament for treatment of novel coronavirus infection (COVID-19), wherein the medicament is administered within 120 hours after the onset of symptoms of SARS-CoV-2 infection or after a positive diagnosis of SARS-CoV-2.

(27) Use of a compound represented by Formula (I-1):

[Chemical formula 26]

(I-1)

, or a pharmaceutically acceptable salt thereof for administration within 48 hours after the onset of symptoms of SARS-CoV-2 infection or after a positive diagnosis of SARS-CoV-2.

(27') Use of a compound represented by Formula (I-1):

[Chemical formula 27]

(I-1)

, or a pharmaceutically acceptable salt thereof for manufacturing a medicament for treatment of novel coronavirus infection (COVID-19), wherein the medicament is administered within 48 hours after the onset of symptoms of SARS-CoV-2 infection or after a positive diagnosis of SARS-CoV-2.

(28) Use of a compound represented by Formula (I-1):

[Chemical formula 28]

(I-1)

, or a pharmaceutically acceptable salt thereof for suppression of viral transmission of SARS-CoV-2.

(28') Use of a compound represented by Formula (I-1):

[Chemical formula 29]

(I-1)

, or a pharmaceutically acceptable salt thereof for manufacturing a medicament for suppression of transmission of novel coronavirus infection (COVID-19).

(29) Use of a compound represented by Formula (I-1):

[Chemical formula 30]

(I-1)

, or a pharmaceutically acceptable salt thereof, for manufacturing a medicament for treatment of novel coronavirus infection (COVID-19), wherein symptoms of an individual in need of treatment of novel coronavirus infection (COVID-19) are mild or moderate I symptoms.

(29') Use of a compound represented by Formula (I-1):

[Chemical formula 31]

(I-1)

, or a pharmaceutically acceptable salt thereof for manufacturing a medicament for treatment of novel coronavirus infection (COVID-19), wherein symptoms of an individual in need of treatment of novel coronavirus infection (COVID-19) are mild or moderate I symptoms.

Furthermore, the present invention relates to the following.

(101) A pharmaceutical composition for prevention of novel coronavirus infection (COVID-19), which is used to suppress the onset after exposure to SARS-CoV-2, comprising a compound represented by Formula (I-1):

[Chemical formula 32]

(I-1)

, or a pharmaceutically acceptable salt thereof.

(102) A method for preventing novel coronavirus infection (COVID-19), comprising administering a compound represented by Formula (I-1):

[Chemical formula 33]

(I-1)

, or a pharmaceutically acceptable salt thereof, to an individual in need of suppression of onset after exposure to SARS-CoV-2.

(103) Use of a compound represented by Formula (I-1):

[Chemical formula 34]

(I-1)

, or a pharmaceutically acceptable salt thereof for suppression of onset after exposure to SARS-CoV-2.

(104) Use of a compound represented by Formula (I-1):

[Chemical formula 35]

(I-1)

, or a pharmaceutically acceptable salt thereof for manufacturing a medicament for suppressing onset after exposure to SARS-CoV-2.

Furthermore, the present invention relates to the following.

(201) A pharmaceutical composition for prevention of novel coronavirus infection (COVID-19), which is used to suppress the onset before exposure to SARS-CoV-2, comprising a compound represented by Formula (I-1):

[Chemical formula 36]

(I-1)

, or a pharmaceutically acceptable salt thereof.

(202) A method of preventing novel coronavirus infection (COVID-19), comprising administering a compound represented by Formula (I-1):

[Chemical formula 37]

(I-1)

, or a pharmaceutically acceptable salt thereof, to an individual in need of prevention before exposure to SARS-CoV-2.

(203) Use of a compound represented by Formula (I-1):

[Chemical formula 38]

(I-1)

, or a pharmaceutically acceptable salt thereof for prevention before exposure to SARS-CoV-2.

(204) Use of a compound represented by Formula (I-1):

[Chemical formula 39]

(I-1)

, or a pharmaceutically acceptable salt thereof for manufacturing a medicament for prevention before exposure to SARS-CoV-2.

One aspect of the present invention further includes the following.

(301) A compound represented by Formula (II):

[Chemical formula 40]

( II )

wherein
X is a single bond or $-CH_2-$;
$R^2$ is substituted or unsubstituted aromatic carbocyclyl;
$R^{3c}$ is substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, halogen, substituted or unsubstituted alkyl, or substituted or unsubstituted amino;
$R^1$ is substituted or unsubstituted aromatic heterocyclyl, or substituted or unsubstituted non-aromatic heterocyclyl;
m is 0 or 1;
$R^{5a}$ is each independently a hydrogen atom, or substituted or unsubstituted alkyl; and
$R^{5b}$ is each independently a hydrogen atom, or substituted or unsubstituted alkyl,
provided that the following compounds are excluded:

[Chemical formula 41]

and

, or a pharmaceutically acceptable salt thereof.

(302) The compound according to the above item (301), wherein

X is a single bond;

$R^2$ is phenyl substituted with halogen;

$R^{3c}$ is non-aromatic heterocyclyl substituted with 1 to 3 substituent(s) selected from a substituent group a or unsubstituted non-aromatic heterocyclyl (substituent group a: halogen, hydroxy, C1-C3 alkyl, C1-C3 alkyloxy, halo C1-C3 alkyl, halo C1-C3 alkyloxy, unsubstituted 5- or 6-membered aromatic heterocyclyl, and 5- or 6-membered aromatic heterocyclyl substituted with halogen), or alkyl substituted with 1 to 3 substituent(s) selected from a substituent group b or unsubstituted alkyl (substituent group b: halogen, hydroxy, C1-C3 alkyloxy, and halo C1-C3 alkyloxy);

$R^1$ is pyridyl substituted with halogen; and

m is 0, or a pharmaceutically acceptable salt thereof.

EFFECT OF THE INVENTION

The compound according to the present invention has inhibitory activity against coronavirus 3CL proteases, and the pharmaceutical composition comprising the compound according to the present invention is useful as a therapeutic agent and/or prophylactic agent for coronavirus infections.

BRIEF DESCRIPTION OF THE DRAWINGS

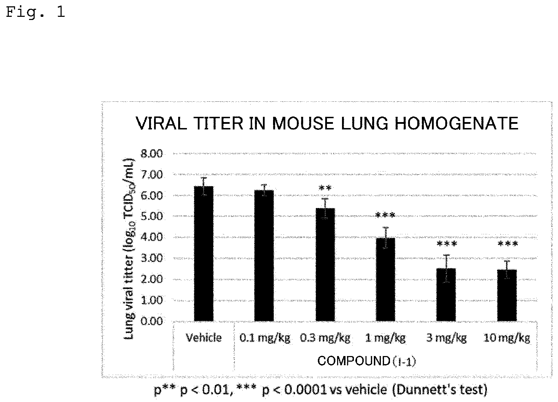

[Fig. 1] Fig. 1 shows viral titers in lung homogenates at 3 days after infection in hCoV-19/Japan/TY7-501/2021-infected mice ($1.00 \times 10^4$ $TCID_{50}$ per mouse, intranasal inoculation) following oral administration of vehicle or the compound represented by Formula (I-1) twice daily for 2 days beginning 1 day after infection. The vertical axis represents viral titers in lung homogenates, and the horizontal axis represents each administration group.

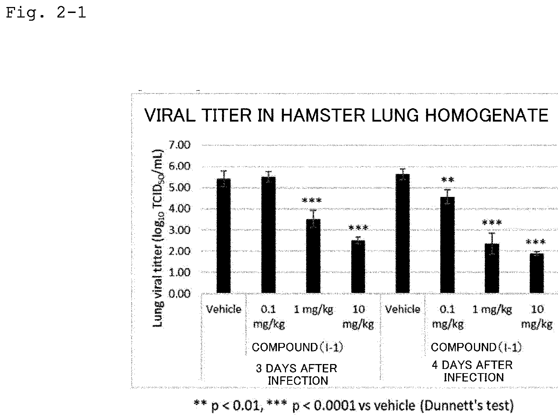

[Fig. 2-1] Fig. 2-1 shows viral titers in lung homogenates at 3 to 4 days after infection in hCoV-19/Japan/TY41-702/2022-infected hamsters ($1.00 \times 10^4$ $TCID_{50}$ per hamster, intranasal inoculation) after oral administration of vehicle or the compound represented by Formula (I-1) twice daily for 2 to 3 days beginning 1 day after infection. The vertical axis represents viral titers in lung homogenates, and the horizontal axis represents each administration group and the number of days after infection.

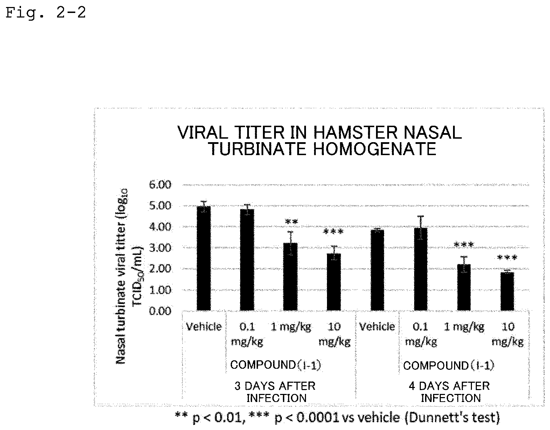

[Fig. 2-2] Fig. 2-2 shows viral titers in nasal turbinate homogenates at 3 to 4 days after infection in hCoV-19/Japan/TY41-702/2022-infected hamsters ($1.00 \times 10^4$ $TCID_{50}$ per hamster, intranasal inoculation) after oral administration of vehicle or the compound represented by Formula (I-1) twice daily for 2 to 3 days beginning 1 day post-infection. The vertical axis represents viral titers in nasal turbinate homogenates, and the horizontal axis represents each administration group and the number of days after infection.

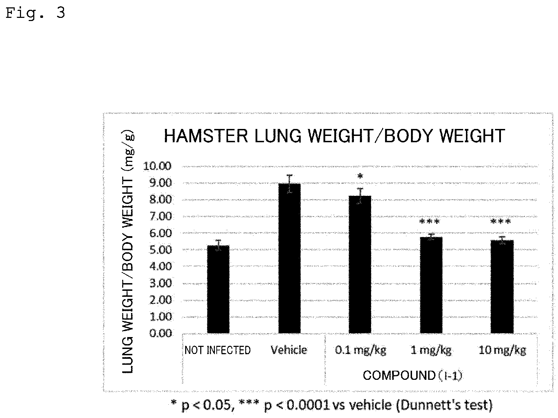

[Fig. 3] Fig. 3 shows lung weight/body weight at 7 days after infection in hCoV-19/Japan/TY41-702/2022-infected hamsters ($1.00 \times 10^4$ $TCID_{50}$ per hamster, intranasal inoculation) after oral administration of vehicle or the compound represented by Formula (I-1) twice daily for 5 days beginning 1 day after infection. The vertical axis represents the lung

weight/body weight, and the horizontal axis represents each administration group.

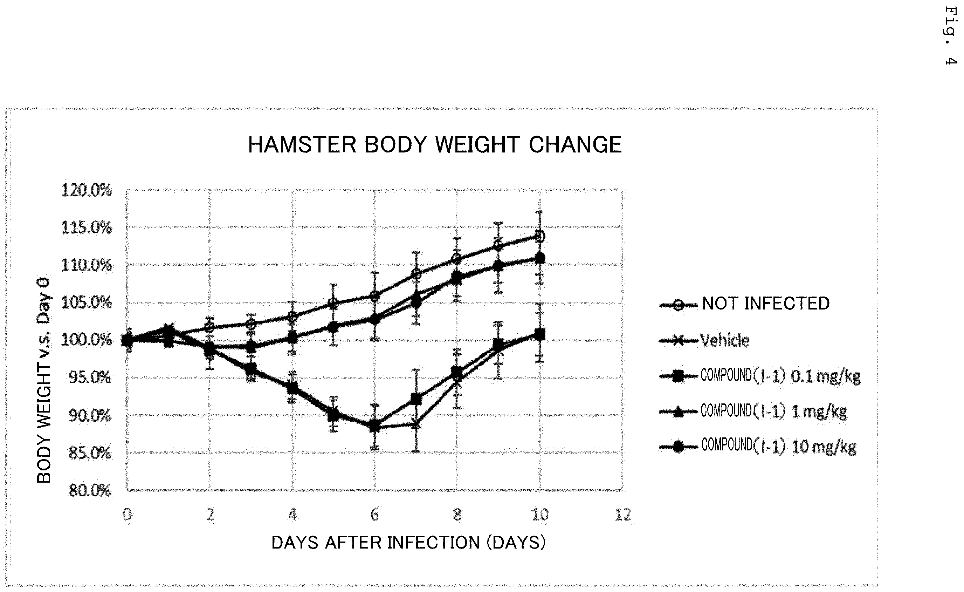

[Fig. 4] Fig. 4 shows body weight changes through 10 days after infection in hCoV-19/Japan/TY41-702/2022-infected hamsters ($1.00 \times 10^4$ $TCID_{50}$ per hamster, intranasal inoculation) after oral administration of vehicle or the compound represented by Formula (I-1) twice daily for 5 days beginning 1 day after infection. The vertical axis indicates the body weight change (%) relative to body weight on the day of infection defined as 100%, and the horizontal axis indicates the number of days after infection.

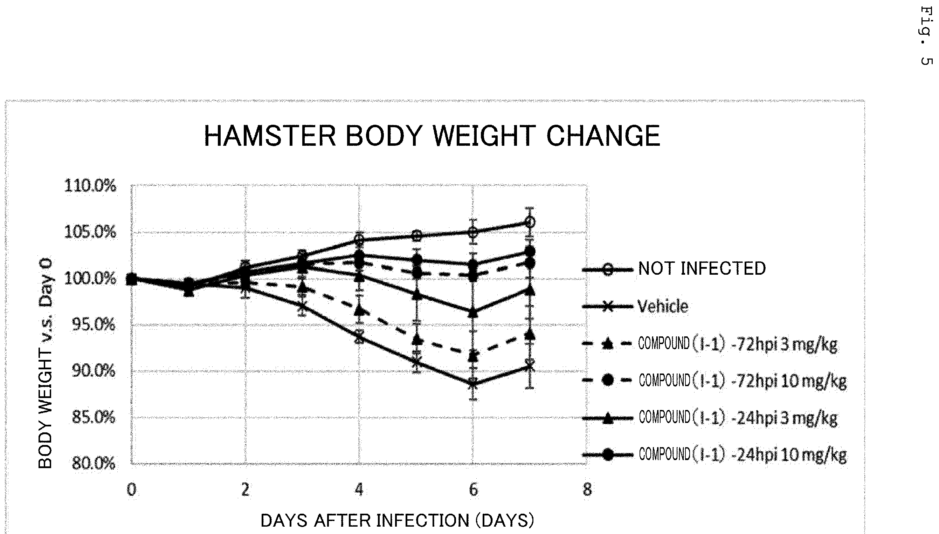

[Fig. 5] Fig. 5 shows body weight changes through 7 days after infection in hCoV-19/Japan/TY41-702/2022-infected hamsters ($1.00 \times 10^4$ $TCID_{50}$ per hamster, intranasal inoculation) after a single subcutaneous administration of vehicle or the compound represented by Formula (I-1) administered 3 days or 1 day before infection. The vertical axis indicates the body weight change (%) relative to body weight on the day of infection defined as 100%, and the horizontal axis indicates the number of days after infection.

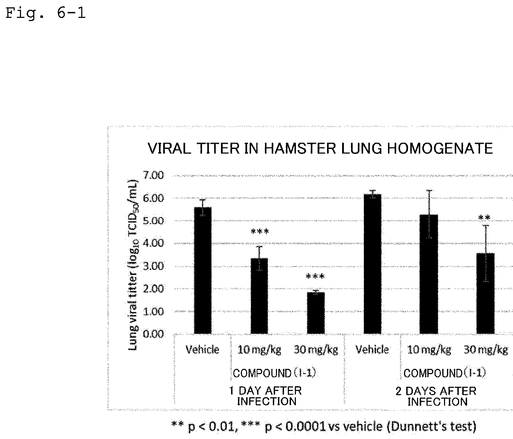

[Fig. 6-1] Fig. 6-1 shows viral titers in lung homogenates at 1 to 2 days after infection in hCoV-19/Japan/TY41-702/2022-infected hamsters ($1.00 \times 10^4$ $TCID_{50}$ per hamster, intranasal inoculation) after a single subcutaneous administration of vehicle or the compound represented by Formula (I-1) administered 1 day before infection. The vertical axis represents viral titers in lung homogenates, and the horizontal axis represents each administration group and the number of days after infection.

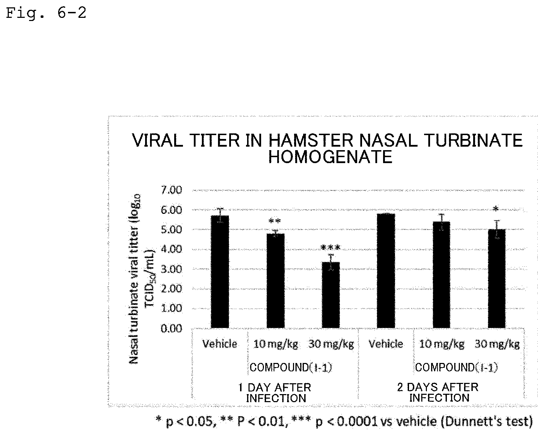

[Fig. 6-2] Fig. 6-2 shows viral titers in nasal turbinate homogenates at 1 to 2 days after infection in hCoV-19/Japan/TY41-702/2022-infected hamsters ($1.00 \times 10^4$ $TCID_{50}$ per hamster, intranasal inoculation) after a single subcutaneous administration of vehicle or the compound represented by Formula (I-1) administered 1 day before infection. The vertical axis represents viral titers in nasal turbinate homogenates, and the horizontal axis represents each administration group and the number of days after infection.

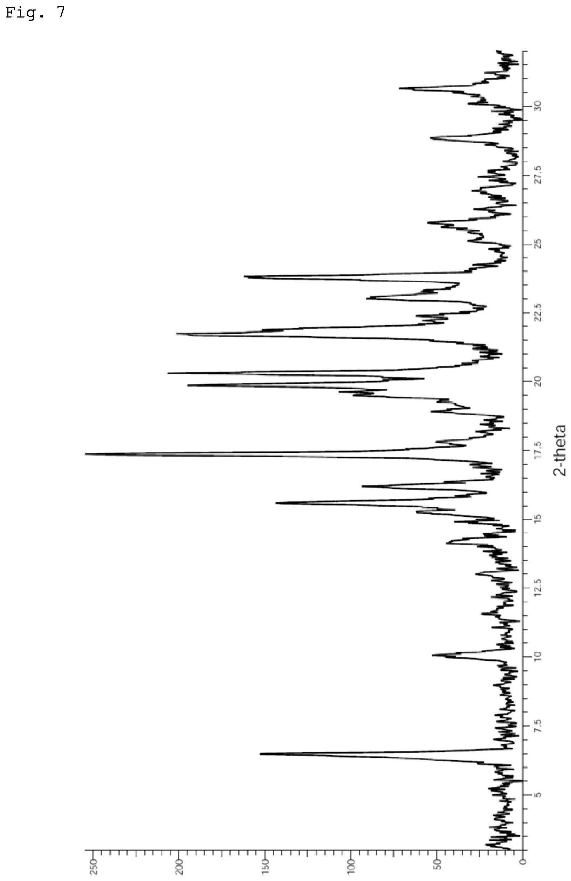

[Fig. 7] Fig. 7 shows an X-ray powder diffraction pattern of an anhydrous crystal of the compound represented by Formula (I-1). The horizontal axis represents $2\theta$ (°), and the vertical axis represents Intensity.

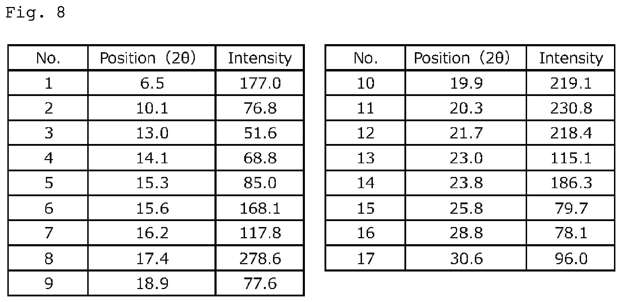

[Fig. 8] Fig. 8 shows a peak list of the X-ray powder diffraction pattern of Fig. 7. In the list, Position represents $2\theta$ (°), and the Intensity represents the intensity.

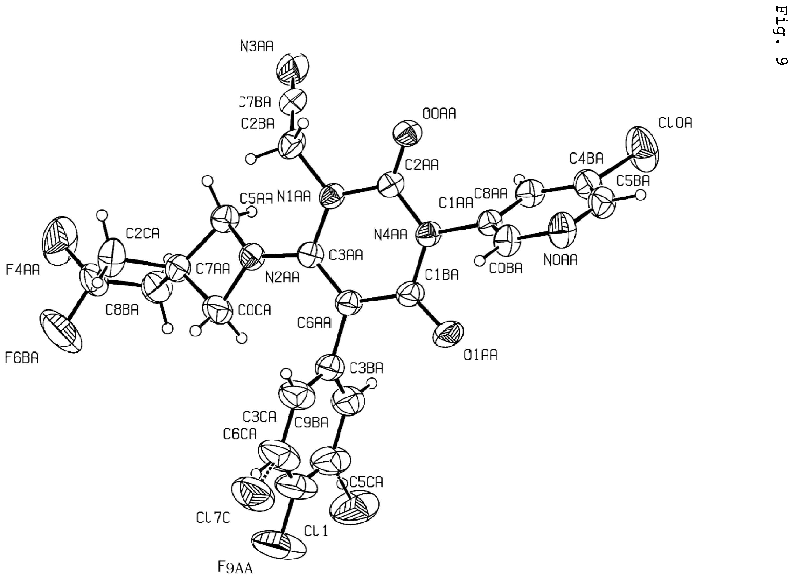

[Fig. 9] Fig. 9 shows a crystal structure (structure in an asymmetric unit) of an anhydrous crystal of the compound represented by Formula (I-1).

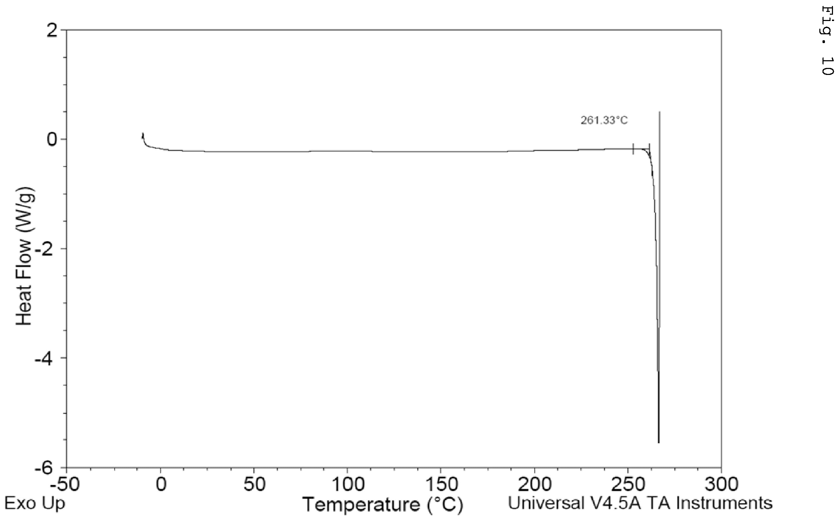

[Fig. 10] Fig. 10 shows differential scanning calorimetry (DSC) results of the anhydrous crystal of the compound represented by Formula (I-1). The horizontal axis represents temperature (°C), and the vertical axis represents heat flow (W/g).

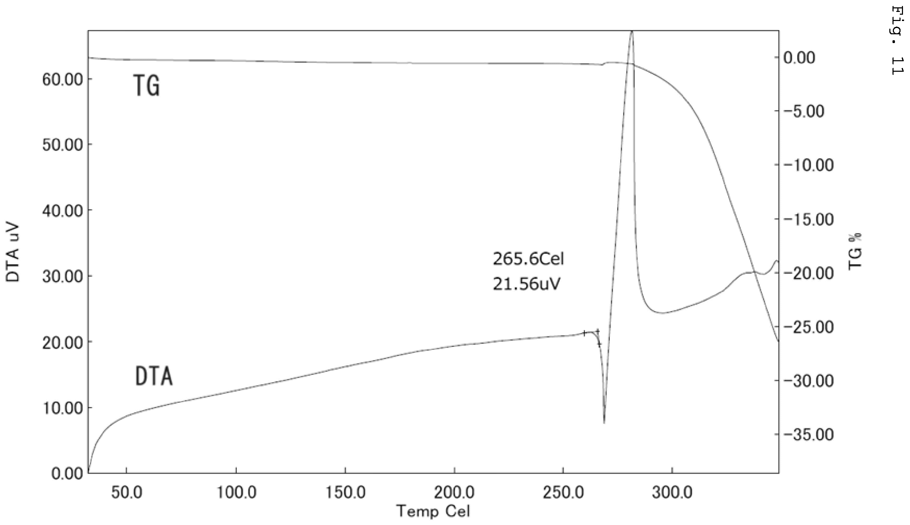

[Fig. 11] Fig. 11 shows simultaneous thermogravimetric-differential thermal analysis (TG/DTA) results of the anhydrous crystal of the compound represented by Formula (I-1). The vertical axis represents heat flow ($\mu$V) or weight change (%), and the horizontal axis represents temperature (°C). In Fig. 11, Cel denotes degrees Celsius (°C).

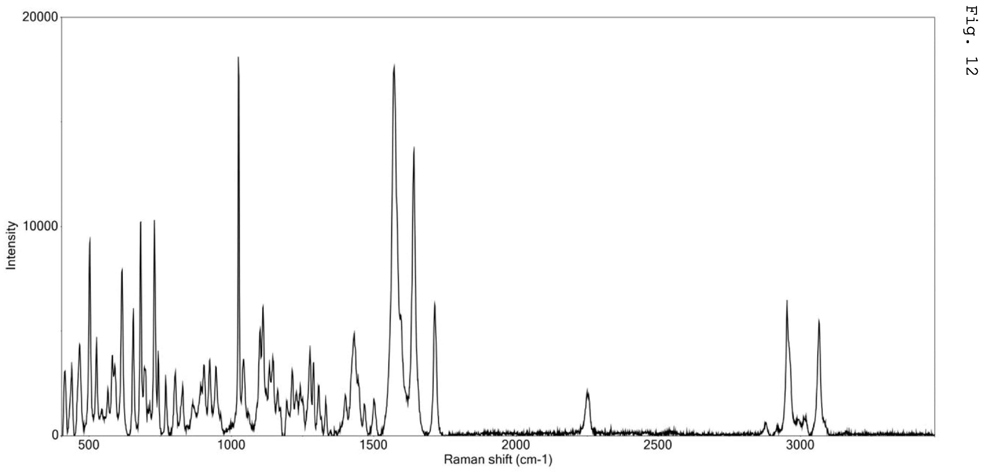

[Fig. 12] Fig. 12 shows a Raman spectrum of the anhydrous crystal of the compound represented by Formula (I-1). The horizontal axis represents Raman shift ($cm^{-1}$), and the vertical axis represents peak intensity.

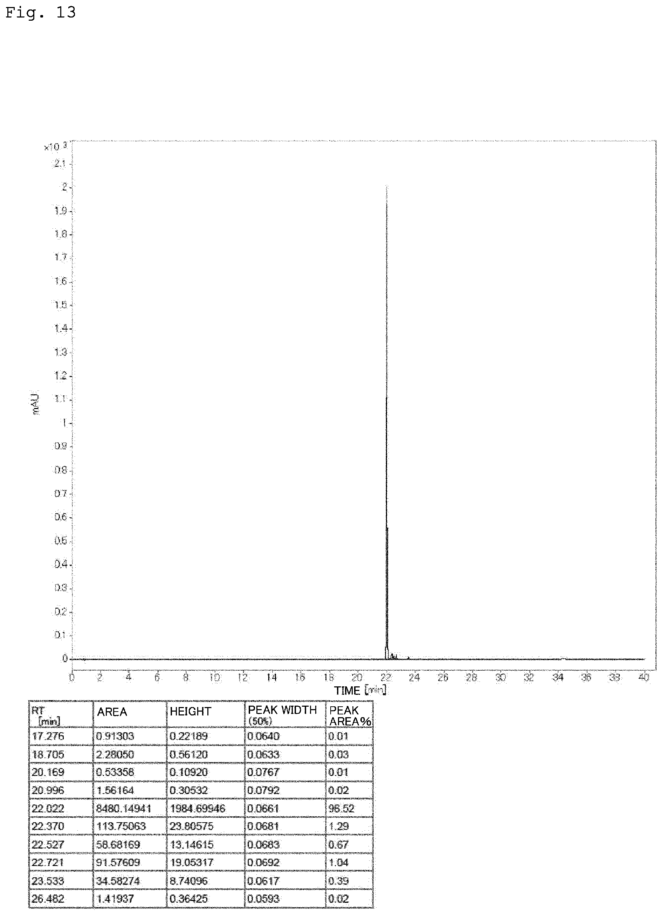

[Fig. 13] Fig. 13 shows HPLC measurement results of the anhydrous crystal of the compound represented by Formula (I-1) in an upper portion. HPLC peak table is shown in a lower portion.

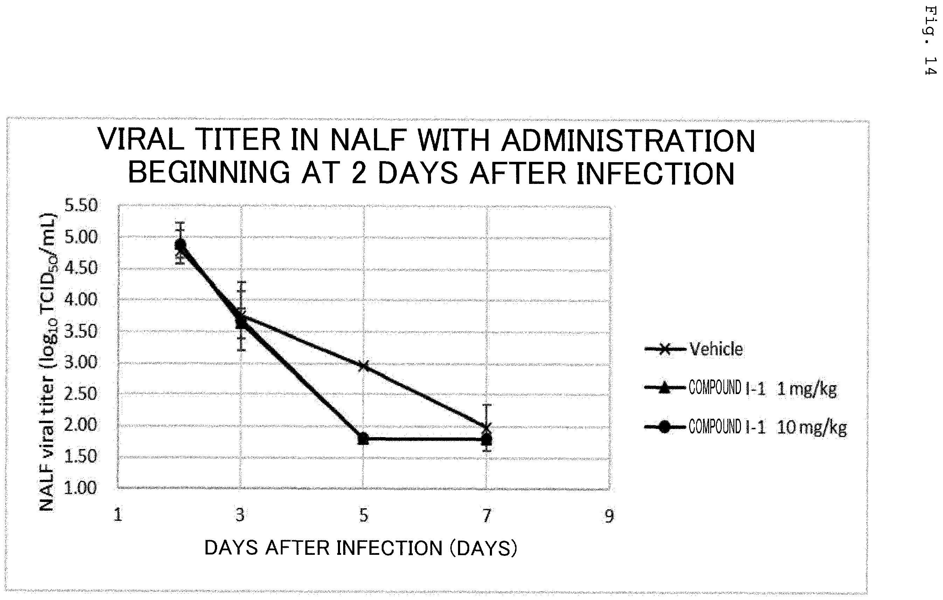

[Fig. 14] Fig. 14 shows viral titers in nasal lavage fluid (NALF) at 3 to 7 days after infection in hamsters infected with hCoV-19/Japan/TY41-702/2022 ($1.00 \times 10^4$ $TCID_{50}$ per hamster, intranasally inoculated), following oral administration of vehicle or the compound represented by Formula (I-1) twice daily for 1 to 5 days beginning 2 days after infection. The vertical axis represents viral titers in NALF, and the horizontal axis represents the number of days after infection.

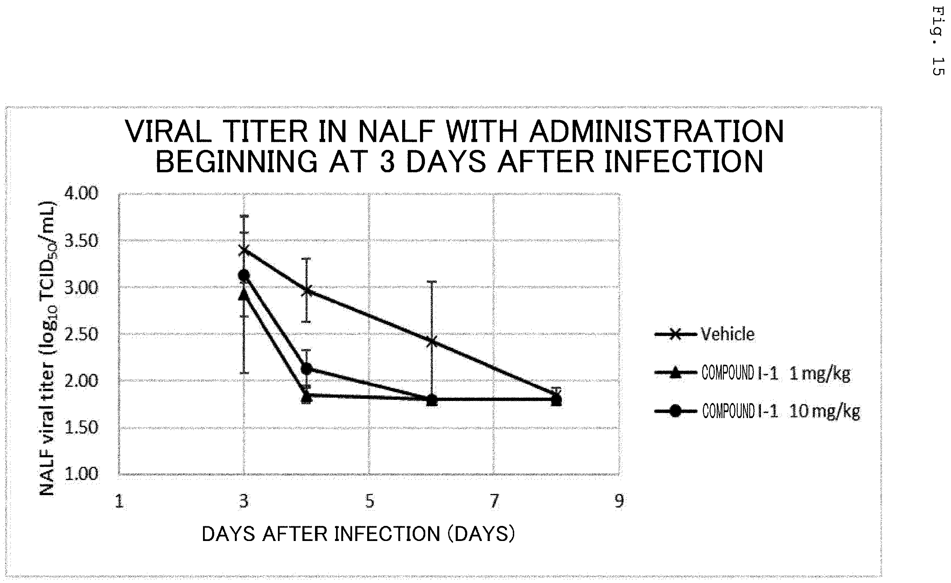

[Fig. 15] Fig. 15 shows viral titers in nasal lavage fluid (NALF) at 4 to 8 days after infection in hamsters infected with hCoV-19/Japan/TY41-702/2022 ($1.00 \times 10^4$ $TCID_{50}$ per hamster, intranasally inoculated), following oral administration of vehicle or the compound represented by Formula (I-1) twice daily for 1 to 5 days beginning 3 days after infection. The vertical axis represents viral titers in NALF, and the horizontal axis represents the number of days after infection.

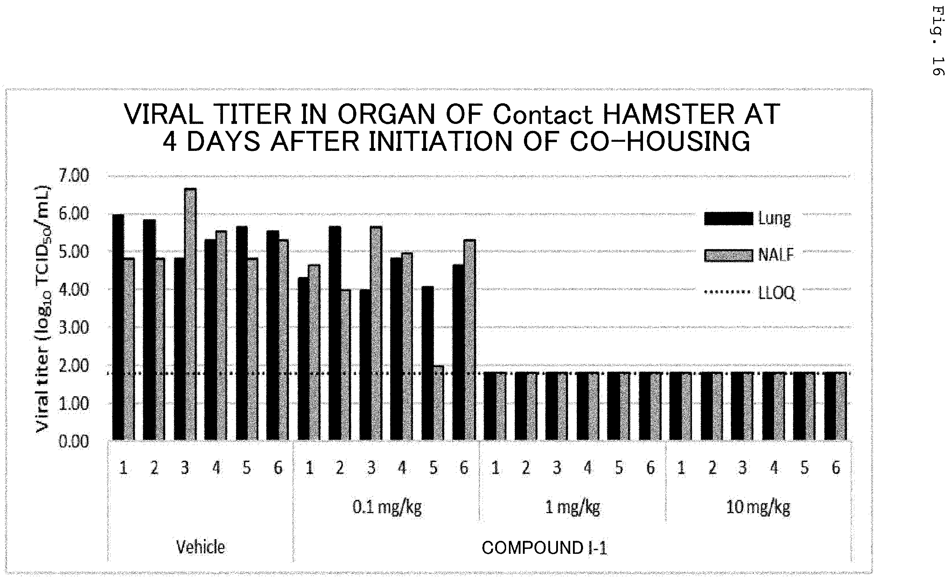

[Fig. 16] Fig. 16 shows viral titers in lung homogenates and nasal lavage fluid (NALF) of non-infected hamsters (Contact) at 4 days after initiation of co-housing, wherein compound was administered to hCoV-19/Japan/TY11-927/2021-infected hamsters ($1.00 \times 10^3$ $TCID_{50}$ per hamster, intranasally inoculated; Index), and non-infected hamsters (Contact) were co-housed with the infected hamsters for a 12-hour nighttime period from 1 to 2 days after infection. The infected hamsters (Index) received oral administration of vehicle or the compound represented by Formula (I-1) twice daily beginning 8 hours after infection. The vertical axis represents viral titers in lung homogenates and NALF, and the horizontal axis represents each administration group.

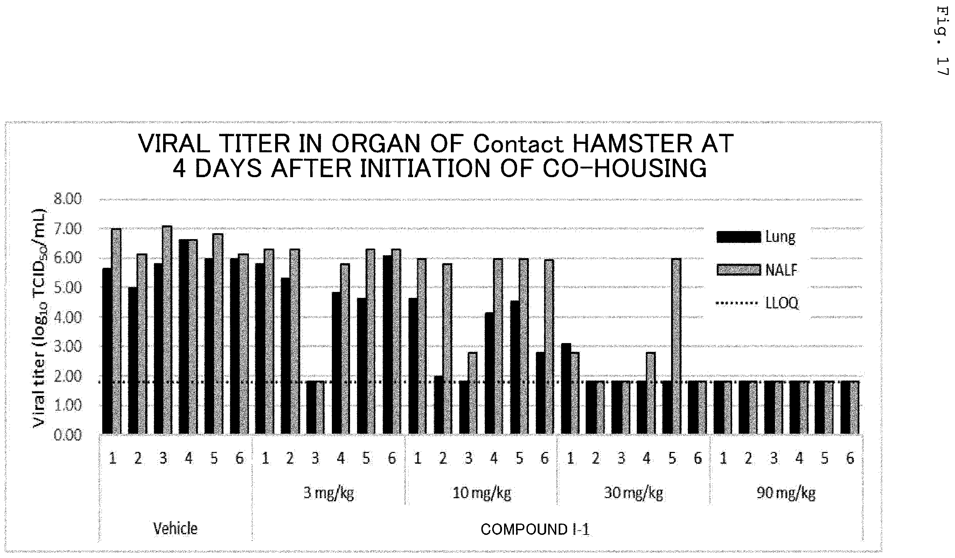

[Fig. 17] Fig. 17 shows viral titers in lung homogenates and nasal lavage fluid (NALF) of non-infected hamsters (Contact) at 4 days after initiation of co-housing with hCoV-19/Japan/TY11-927/2021-infected hamsters ($1.00 \times 10^3$ $TCID_{50}$ per hamster, intranasally inoculated; Index), wherein co-housing was conducted for a 12-hour nighttime period from 1 to 2 days after infection. The non-infected hamsters (Contact) received subcutaneous administration of

vehicle or the compound represented by Formula (I-1) 12 hours prior to initiation of co-housing. The vertical axis represents viral titers in lung homogenates and NALF, and the horizontal axis represents each administration group.

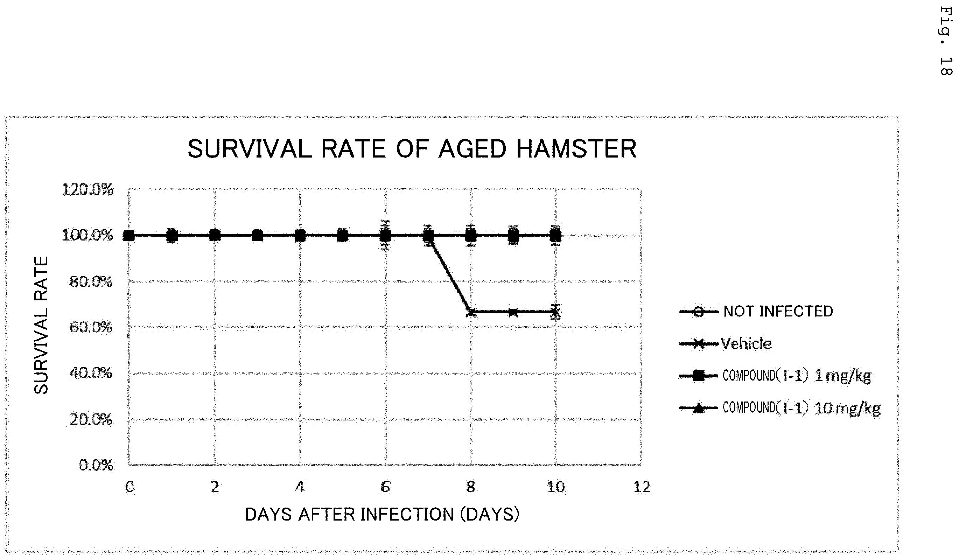

[Fig. 18] Fig. 18 shows the survival rate up to 10 days after infection in aged hamsters (11-month-old) infected with hCoV-19/Japan/TY11-927/2021 ($1.00 \times 10^4$ $TCID_{50}$ per hamster, intranasally inoculated), following oral administration of vehicle or the compound represented by Formula (I-1) twice daily for 5 days beginning 1 day after infection. The vertical axis represents the survival rate (%), and the horizontal axis represents the number of days after infection.

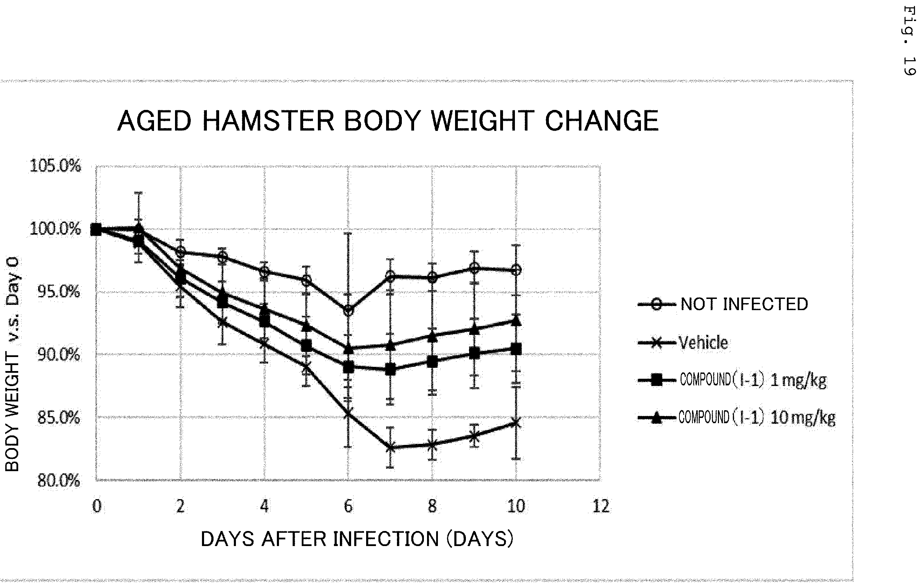

[Fig. 19] Fig. 19 shows body weight changes up to 10 days after infection in aged hamsters (11-month-old) infected with hCoV-19/Japan/TY11-927/2021 ($1.00 \times 10^4$ $TCID_{50}$ per hamster, intranasally inoculated), following oral administration of vehicle or the compound represented by Formula (I-1) twice daily for 5 days beginning 1 day after infection. The vertical axis represents the body weight change (%) relative to body weight on the day of infection defined as 100%, and the horizontal axis represents the number of days after infection.

MODE FOR CARRYING OUT THE INVENTION

Hereinafter, the meaning of each term used in the present specification will be described. Unless particularly stated otherwise, each term is used in the same sense, either alone or in combination with other terms.

The term "consist of" means having only the constituent elements.

The term "comprise" and the term "encompass" mean that elements are not limited to the constituent elements, and elements that are not described are not excluded.

The present invention will be described below with reference to embodiments. Throughout this specification, it should be understood that the representation of the singular also includes the concept of the plural unless stated otherwise. Thus, unless stated otherwise, singular articles (e.g., "a", "an", "the", and the like in English) can be understood to include the concepts of the plural forms.

Unless otherwise defined, it should be understood that the terms used in the present specification are used in a meaning normally used in the art. Accordingly, unless otherwise defined, all terminologies and scientific and technical terms used in the present specification have the same meanings as commonly understood by those having ordinary skill in the art to which the present invention belongs. In the event of any inconsistency, the present specification (including the definitions) shall prevail.

In the compound represented by Formula (II) of the present specification, the definitions of each term used for $R^1$, $R^2$, $R^{3c}$, $R^{5a}$, and $R^{5b}$ may refer to International Publication WO2023/195529, and the disclosure of which is incorporated herein by reference. With respect to the definitions of substituents used in each term, reference may be made to International Publication WO2023/195529, and the disclosure of which is incorporated herein by reference.

The compound represented by Formula (I-1) or (II) is not limited to specific isomers, but includes all possible isomers (e.g., keto-enol isomer, imine-enamine isomers, diastereoisomers, optical isomers, rotational isomers, etc.), racemates or mixtures thereof.

One or more hydrogen atoms, carbon atoms, and/or other atoms of the compound represented by Formula (I-1) or (II) may be replaced with isotopes of the corresponding hydrogen atoms, carbon atoms, and/or other atoms. Examples of such isotopes include isotopes of hydrogen, carbon, nitrogen, oxygen, phosphorus, sulfur, fluorine, iodine, and chlorine, such as $^2H$, $^3H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$, and $^{36}Cl$. The compound represented by Formula (I-1) or (II) also encompasses compounds substituted with such isotopes (for example, deuterated forms thereof). Compounds substituted with such isotopes are also useful as medicaments. The compound represented by Formula (I-1) or (II) encompasses all radiolabeled forms substituted with radioactive isotopes among such isotopes. Furthermore, a "radiolabeling method" for producing the "radiolabeled forms" is also included in the present invention, and the "radiolabeled forms" are useful as tools for metabolic pharmacokinetics studies, studies on binding assay, and/or diagnostics.

The deuterated forms of the compound represented by Formula (I-1) include the following structures:

[Chemical formula 42]

[Chemical formula 43]

[Chemical formula 44]

[Chemical formula 45]

Radiolabeled forms of the compound represented by Formula (I-1) or (II) can be prepared by methods well known in the art. For example, a tritium-labeled compound of Formula (I-1) or (II) can be prepared by introducing tritium into certain compound of Formula (I-1) or (II) by a catalytic dehalogenation reaction using tritium. The method involves the reaction of tritium gas with an appropriately halogensubstituted precursor of compound represented by Formula (I-1) or (II) in the presence or absence of a base, in the presence of a suitable catalyst, e.g. Pd/C. Other suitable methods for preparing a tritium-labeled compound are described in "Isotopes in the Physical and Biomedical Sciences, Vol. 1, Labeled Compounds (Part A), Chapter 6 (1987)". $^{14}C$-labeled compounds can be prepared by using raw material having a $^{14}C$ carbon.

The compound represented by Formula (I-1) or (II) as used herein may form a prodrug, and the present invention includes such various prodrugs. A prodrug is a derivative of the compound of the present invention having a chemically or metabolically cleavable group, and is a compound that becomes a pharmaceutically active compound of the present invention by solvolysis or in vivo under physiological conditions. Prodrugs encompass compounds that are enzymatically oxidized, reduced, hydrolyzed, or the like under physiological conditions in vivo to be converted into the compound represented by Formula (I-1) or (II), as well as compounds that are hydrolyzed by gastric acid or the like to be converted into the compound represented by Formula (I-1) or (II). Methods for selecting and preparing suitable prodrug derivatives are described in, for example, "Design of Prodrugs, Elsevier, Amsterdam, 1985". A prodrug may have activity per se.

As used herein, the "compound represented by Formula (I-1) or (II)" may form salts, co-crystals, or solvates thereof.

As used herein, the "compound represented by Formula (I-1) or (II), a pharmaceutically acceptable salt thereof, or a solvate thereof" encompasses such various salts, co-crystals, and solvates thereof.

"Salt" as used in the present specification means that, for example, the "compound represented by Formula (I-1) or (II)" and a counter molecule are regularly arranged in the same crystal lattice, and may include any number of counter molecules. The salt refers to a form in which proton transfer occurs between the compound and the counter molecule in the crystal lattice, resulting in formation via ionic bonding.

As used herein, the term "co-crystal" refers to a form in which counter molecules (co-former molecules) are regularly arranged within the same crystal lattice, and may include any number of counter molecules (co-former molecules). In addition, a co-crystal refers to a form in which intermolecular interactions between a compound and counter molecules (co-former molecules) are mediated by non-covalent and non-ionic chemical interactions, such as hydrogen bonding and van der Waals forces.

Generally, salts are considered to cause proton transfer between the compound and the counter molecule, but in some cases, it is also known that proton transfer may not be complete. This state may also be referred to as co-crystals because it is not a true salt. It is also known that proton transfer may vary continuously with temperature.

Thus, the "pharmaceutically acceptable salt of the compound represented by Formula (I-1) or (II)" as used herein encompass a co-crystal and refers to a pharmaceutically acceptable salt or co-crystal of the compound represented by Formula (I-1) or (II).

One aspect herein is a pharmaceutically acceptable salt or co-crystal of the compound represented by Formula (I-1) or (II) in combination with hydrofluoric acid, hydrochloric acid, hydrobromic acid, orthophosphoric acid, hydroiodic acid, nitric acid, phosphoric acid, boric acid, methanesulfonic acid, ethanesulfonic acid, p-toluenesulfonic acid, benzenesulfonic acid, trifluoromethylbenzenesulfonic acid, chlorobenzenesulfonic acid, methoxybenzenesulfonic acid, acetic acid, propionic acid, lactic acid, citric acid, fumaric acid, malonic acid, malic acid, succinic acid, salicylic acid, maleic acid, glycerophosphoric acid, tartaric acid, benzoic acid, glutamic acid, aspartic acid, 2-naphthalenesulfonic acid, hexanoic acid, acetylsalicylic acid, and the like.

Studies of salt formation and co-crystal formation provide a means for modifying the physicochemical properties and resulting biological properties of a drug without altering its chemical structure. Salt formation and co-crystal formation can have a dramatic impact on the properties of the drug. In the selection of an appropriate salt or co-crystal, hygroscopicity, stability, solubility and processing properties are also important perspectives. Solubility of the salt or co-crystal may affect their suitability for use as a drug. When the aqueous solubility is low, the dissolution rate in in vivo administration may be limited to the absorption process, resulting in low bioavailability. Also, their low water solubility may make administration by injection difficult, thus limiting the selection of the appropriate routes of administration.

The "compound represented by Formula (I-1) or (II)" can form a solvate with water (i.e., a hydrate) or a solvate with a common organic solvent. The "pharmaceutically acceptable salt of the compound represented by Formula (I-1) or (II)" can also form a solvate with water (i.e., a hydrate) or a solvate with a common organic solvent.

The "solvate" as used in the present specification means that, for example, with regard to the compounds represented by Formula (I-1) or (II), the compounds and any number of solvent molecules are arranged with regularity.

Examples of the solvent molecule include ethyl acetate, water, ethanol, acetone, 1,1-diethoxypropane, 1,1-dimethoxymethane, 2,2-dimethoxypropane, isooctane, isopropyl ether, methylisopropylketone, methyltetrahydrofuran, petroleum ether, trichloroacetic acid, trifluoroacetic acid, acetic acid, anisole, 1-butanol, 2-butanol, n-butyl acetate, t-butyl methyl ether, cumene, dimethyl sulfoxide, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, tetrahydrofuran, acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methylbutyl ketone, methylcyclohexane, N-methylpyrrolidone, nitromethane, pyridine, sulfolane, tetralin, toluene, 1,1,2-trichloroethene, xylene, and t-butanol.

The solvent molecule can be preferably acetic acid, water, ethanol, acetone, 1,1-diethoxypropane, 1,1-dimethoxymethane, 2,2-dimethoxypropane, isooctane, isopropyl ether, methylisopropylketone, methyltetrahydrofuran, petroleum ether, trichloroacetic acid, trifluoroacetic acid, acetic acid, anisole, 1-butanol, 2-butanol, n-butyl acetate, t-butyl methyl ether, cumene, dimethyl sulfoxide, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, or tetrahydrofuran.

The solvent molecule is more preferably ethyl acetate, water, ethanol, acetone, 1,1-diethoxypropane, 1,1-dimethoxymethane, 2,2-dimethoxypropane, isooctane, isopropyl ether, methylisopropylketone, methyltetrahydrofuran, petroleum ether, trichloroacetic acid, or trifluoroacetic acid. When the "compound represented by Formula (I-1) or (II)" is allowed to stand in the atmosphere, the compound may absorb water, resulting in attachment of adsorbed water or formation of a hydrate.

"Crystal" as used in the present specification means a solid in which constituent atoms, ions, molecules, and the

like are three-dimensionally arranged with regularity, and is distinguished from an amorphous solid that does not have such a regular internal structure. The crystal as used herein may be a single crystal, a twin crystal, a polycrystal, or the like.

Furthermore, the "crystal" may include "crystalline polymorphs" that have the same composition but different arrangements in the crystal, and crystals including those crystalline polymorphs are referred to as "crystalline forms".

The "compound represented by Formula (I-1) or (II), a pharmaceutically acceptable salt thereof, or a solvate thereof" encompasses a crystal polymorph.

The crystal of the compound according to the present invention may be a deuterated form. The crystal as used herein may be labeled with isotopes (for example, $^{2}H$, $^{3}H$, $^{14}C$, $^{35}S$, $^{125}I$, and the like).

The crystal form and/or degree of crystallinity can be confirmed, for example, by spectroscopy such as X-ray powder diffraction, Raman spectroscopy, infrared absorption spectroscopy, and solid-state NMR. In addition, the physical properties of the crystal can be confirmed by various techniques such as differential scanning calorimetry, moisture sorption/desorption measurements, and dissolution characteristics.

One aspect herein is an anhydrous crystal of the compound represented by Formula (I-1).

As used herein, "anhydride" is synonymous with "nonsolvated form", "unsolvated form", "non-hydrated form", and "anhydrous form".

In the anhydrous crystal of the compound represented by Formula (I-1), the theoretical content of crystalline water is 0 wt%. However, in the analysis of water content and/or solvent content, the measured value may be higher than the theoretical content of crystalline water due to the influence of adsorbed water and/or adsorbed solvent adhering to the crystal surface.

One aspect herein is an anhydrous crystal of the compound represented by Formula (I-1), having characteristic peaks at diffraction angles (2θ) of 6.5°±0.2°, 15.6°±0.2°, 17.4°±0.2°, 19.9°±0.2°, and 20.3°±0.2° in an X-ray powder diffraction pattern (CuKα radiation, λ=1.5418 Å).

One aspect herein is an anhydrous crystal of the compound represented by Formula (I-1), wherein when the anhydride crystal is subjected to single crystal structure analysis using CuKα radiation (λ=1.5418 Å) at 298 K (25°C), it is characterized by having
the following crystallographic data:

space group: Pbca

$$a=14.67\text{Å}\pm0.05\text{Å}$$

$$b=11.83\text{Å}\pm0.05\text{Å}$$

$$c=27.10\text{Å}\pm0.05\text{Å}$$

α=90°
β=90°
γ=90°.

One aspect herein is an anhydrous crystal of the compound represented by Formula (I-1), having a melting point of 261.3°C±2°C in differential scanning calorimetry (DSC).

One aspect herein is an anhydrous crystal of the compound represented by Formula (I-1), having a melting point of 265.6°C±2°C in simultaneous thermogravimetric-differential thermal analysis (TG/DTA).

One aspect herein is an anhydrous crystal of the compound represented by Formula (I-1), having characteristic peaks at 415.2 $cm^{-1}$±2 $cm^{-1}$, 502.7 $cm^{-1}$±2 $cm^{-1}$, 1431.4 $cm^{-1}$±2 $cm^{-1}$, 1714.8 $cm^{-1}$±2 $cm^{-1}$, and 3065.4 $cm^{-1}$±2 cm-1 in a Raman spectrum.

(X-Ray Powder Diffraction (XRPD))

X-ray Powder diffraction (XRPD) is one of the most sensitive analytical techniques for measuring crystal forms and crystallinity of solids. When the crystal is irradiated with X-rays, they are reflected by crystal lattice planes and interfere with each other, resulting in ordered diffracted lines corresponding to the periodicity of the crystal structure. In contrast, amorphous solids generally lack an ordered repeating periodicity in their structure, and therefore do not exhibit diffraction phenomena, instead showing a broad and featureless XRPD pattern (also referred to as a halo pattern).

The crystal form of the compound represented by Formula (I-1) or (II) can be identified by an X-ray powder diffraction pattern and characteristic diffraction peaks. The crystal form of the compound represented by Formula (I-1) or (II) can be distinguished from other crystal forms by the presence of characteristic diffraction peaks.

Characteristic diffraction peaks used in the present specification are peaks selected from an observed diffraction pattern. Characteristic diffraction peaks are preferably selected from about 10 peaks, more preferably about 5 peaks, and even more preferably about 3 peaks, in a diffraction pattern.

When distinguishing a plurality of crystals, a peak that is identified in the relevant crystal and is not identified in other crystals becomes a characteristic peak preferable for characterizing the crystal, rather than the intensity of a peak. With such characteristic peaks, even one or two peaks can characterize the crystal. When the charts obtained by measurement are compared and these characteristic peaks are found to coincide, it can be said that the X-ray powder diffraction patterns substantially coincide.

Generally, since the diffraction angle ($2\theta$) in X-ray powder diffraction may cause an error within the range of $\pm 0.2°$, it should be understood that a value of the diffraction angle of X-ray powder diffraction also includes numerical values within the range of about $\pm 0.2°$. Therefore, not only crystals in which the diffraction angles of peaks in X-ray powder diffraction perfectly coincide, but also crystals in which the diffraction angles of peaks coincide with an error of about $\pm 0.2°$, are included in the present invention.

It is known that generally, the intensity of a peak indicated in the following tables and drawings may fluctuate due to many factors, for example, the effect of selective orientation of crystals with respect to an X-ray beam, the influence of coarse particles, the purity of the substance to be analyzed, or the crystallinity of a sample. In addition, the peak positions may shift depending on height variations in sample. Furthermore, when measurements are performed using different wavelengths, different shifts are obtained in accordance with Bragg's equation ($n\lambda = 2d \sin\theta$); however, such XRPD patterns obtained using different wavelengths are also included within the scope of the present invention.

(Single Crystal Structure Analysis)

Single crystal structure analysis is one method for identifying a crystal and enables the determination of crystallographic parameters in the crystal, as well as atomic coordinates (values indicating the spatial relationships of individual atoms) and a three-dimensional structural model. For single crystal structure analysis, reference may be made to, for example, Toshio Sakurai, Guide to X-ray Structure Analysis, Shokabo Publishing (1983), and Stout & Jensen, X-Ray Structure Determination: A Practical Guide, Macmillan Co., New York (1968). When the crystal structures of a complex, a salt, an optical isomer, a tautomer, and a geometrical isomer such as the present invention are identified, single crystal structure analysis is useful.

(Raman Spectroscopy)

Raman spectroscopy shows the characteristics of the oscillation of a molecular or composite system. Its origin lies in the inelastic collision between molecules and photons, which are particles of light including light rays. Collision between molecules and photons results in the exchange of energy, which results in a change in energy, and thereby the wavelength of the photons changes. That is, since the Raman spectrum is a spectral line of very narrow wavelengths, which is emitted when photons are incident on a molecule of interest, lasers and the like are used as light sources. The wavelength of each Raman line is indicated by the wavenumber shift from incident light, and this is the difference between the Raman line and the reciprocal of the wavelength of incident light. Raman spectroscopy is to measure the state of oscillation of molecules, and this is determined by the molecular structure thereof.

Generally, since Raman spectral peaks ($cm^{-1}$) can cause errors within the range of $\pm 2$ $cm^{-1}$, it should be understood that the values of the above-described Raman spectral peaks also include numerical values within the range of about $\pm 2$ $cm^{-1}$. Therefore, not only the crystals whose Raman spectral peaks in the Raman spectra perfectly coincide, but also the crystals whose Raman spectral peaks coincide with errors of about $\pm 2$ $cm^{-1}$, are included in the present invention.

(Differential Scanning Calorimetry (DSC))

DSC is one of important measurement methods for thermal analysis and is a method of measuring the thermal properties of a substance as an aggregate of atoms and molecules.

A differential scanning calorimetry curve is obtained by measuring the temperature-related or time-related change in the calorific value of an active pharmaceutical ingredient by DSC and plotting the obtained data with respect to temperature or time. From the differential scanning calorimetry curve, information on the onset temperature at the time of melting of the active pharmaceutical ingredient, the maximum value of the endothermic peak curve associated with melting, and the enthalpy can be obtained.

With regard to DSC, it is known that the temperature to be observed may depend on the rate of temperature change as well as the sample preparation technique and the particular apparatus used. Therefore, the "melting point" in DSC refers to the onset temperature that is not likely to be affected by the sample preparation technique. The error range for the onset temperature obtainable from the differential scanning calorimetry curve is approximately $\pm 2°C$. For the

recognition of identity of crystals, not only the melting point but also the overall pattern are important, and there may be some variation depending on the measurement conditions and the measuring equipment.

(Simultaneous Thermogravimetric-Differential Thermal Analysis (TG/DTA))

TG/DTA is one of important measurement methods for thermal analysis and is a method of measuring the weight and thermal properties of a substance as an aggregate of atoms and molecules.

TG/DTA is a method of measuring the temperature-related or time-related changes in weight and calorific value of an active pharmaceutical ingredient, and a TG (thermogravimetric) and DTA (differential thermal) curve is obtained by plotting obtained data with respect to temperature or time. From the TG/DTA curve, information on the changes in weight and calorific value in relation to degradation, dehydration, oxidation, reduction, sublimation, and evaporation of an active pharmaceutical ingredient can be obtained.

With regard to TG/DTA, it is known that the temperature to be observed and the weight change may depend on the rate of temperature change as well as the sample preparation technique and the particular apparatus used. Therefore, the "melting point" in TG/DTA refers to the onset temperature that is not likely to be affected by the sample preparation technique. For the recognition of identity of crystals, not only the melting point but also the overall pattern are important, and there may be some variation depending on the measurement conditions and the measuring equipment.

Since the compound according to the present invention has coronavirus 3CL protease inhibitory activity, the compound is useful as a therapeutic agent and/or prophylactic agent for a disease associated with coronavirus 3CL proteases.

When the term "therapeutic agent and/or prophylactic agent" is used in the present invention, this also encompasses a symptom ameliorating agent.

In the present invention, the term "prevention" encompasses suppression of onset after exposure to SARS-CoV-2.

As used herein, the term "prevention" encompasses suppressing the onset of symptoms caused by SARS-CoV-2 by administering the pharmaceutical composition of the present invention to an individual who has been exposed to SARS-CoV-2 and is in need of suppression of the onset.

In the present invention, the term "prevention" encompasses prevention before exposure to SARS-CoV-2.

In the present invention, the term "prevention" encompasses suppressing the onset of symptoms caused by SARS-CoV-2 by administering the pharmaceutical composition of the present invention to an individual who has not been exposed to SARS-CoV-2 and is in need of suppression of the onset.

In the present invention, the term "prevention" encompasses suppression of onset and exacerbation after exposure to SARS-CoV-2.

The disease associated with coronavirus 3CL proteases may be viral infections, and preferably coronavirus infections.

According to an aspect, the coronavirus may be a coronavirus that infects human beings. The coronavirus that infects human beings may be HCoV-229E, HCoV-NL63, HCoV-HKU1, HCoV-OC43, SARS-CoV, MERS-CoV, and/or SARS-CoV-2.

As one aspect, as the coronavirus, alphacoronavirus and/or betacoronavirus, more preferably betacoronavirus, and further preferably sarbecovirus are exemplified.

According to an aspect, the alphacoronavirus may be HCoV-229E and HCoV-NL63. The alphacoronavirus may be particularly preferably HCoV-229E.

According to an aspect, the betacoronavirus may be HCoV-HKU1, HCoV-OC43, SARS-CoV, MERS-CoV, and/or SARS-CoV-2. The betacoronavirus may be HCoV-OC43 or SARS-CoV-2, and particularly preferably SARS-CoV-2.

According to an aspect, the betacoronavirus may be betacoronavirus lineage A (β-coronavirus lineage A), betacoronavirus lineage B (β-coronavirus lineage B), and betacoronavirus lineage C (β-coronavirus lineage C).The betacoronavirus may be more preferably betacoronavirus lineage A (β-coronavirus lineage A) and betacoronavirus lineage B (β-coronavirus lineage B) and particularly preferably betacoronavirus lineage B (β-coronavirus lineage B).

The betacoronavirus lineage A (β-coronavirus lineage A) may be, for example, HCoV-HKU1 or HCoV-OC43, preferably HCoV-OC43. The betacoronavirus lineage B (β-coronavirus lineage B) may be, for example, SARS-CoV or SARS-CoV-2, preferably SARS-CoV-2. The betacoronavirus lineage C (β-coronavirus lineage C) may be preferably MERS-CoV.

According to an aspect, the coronavirus may be HCoV-229E, HCoV-OC43, and/or SARS-CoV-2, and particularly preferably SARS-CoV-2.

It is generally known that viruses undergo mutations as they repeatedly replicate and infect. The coronavirus includes not only variants known in the art but also variants that may emerge in the future, so long as the compound according to the present invention is a strain capable of exhibiting coronavirus 3CL protease inhibitory activity. Examples of known variants of SARS-CoV-2 include, for example, the variants used in the Examples of the present specification.

The coronavirus infections may be infections caused by HCoV-229E, HCoV-NL63, HCoV-OC43, HCoV-HKU1, SARS-CoV, MERS-CoV, and/or SARS-CoV-2. Preferably, the coronavirus infections may be infections caused by HCoV-229E, HCoV-OC43, and/or SARS-CoV-2, and particularly preferably infection caused by SARS-CoV-2.
The coronavirus infections may be particularly preferably novel coronavirus infection (COVID-19).
The severity classification of individuals infected with the novel coronavirus is exemplified, for example, as follows (Reference: Clinical Management Guidelines for COVID-19, Version 10.0, Ministry of Health, Labour and Welfare).

(Mild)

The individual has an oxygen saturation of 96% or higher. The individual has no respiratory symptoms, or has cough alone without dyspnea, and in either case has no evidence of pneumonia.

(Moderate I)

The individual has an oxygen saturation of less than 96% and greater than 93%. The individual has dyspnea and evidence of pneumonia.

(Moderate II)

The degree of oxygen saturation is less than 93%. Respiratory failure is present, requiring oxygen therapy.

(Severe)

The individual is admitted to the intensive care unit (ICU) or requires mechanical ventilation.
The above classification is based on the severity classification defined in Japan; however, for example, severity classifications established in China or by the U.S. National Institutes of Health (NIH) may likewise be incorporated by reference.
Further, an asymptomatic SARS-CoV-2-infected individual refers to an asymptomatic carrier. For example, an individual in whom none of the 14 or 12 symptoms of COVID-19 are observed may be included.
(14 symptoms of COVID-19: malaise (fatigue), myalgia or body aches, headache, chills/sweating, feeling feverish or fever, taste disorder, olfactory disorder, runny nose or nasal congestion, sore throat, cough, shortness of breath (dyspnea), nausea, vomiting, and diarrhea)
As used herein, the 12 symptoms of COVID-19 include (malaise (fatigue), myalgia or body aches, headache, chills/sweating, feeling feverish or fever, runny nose or nasal congestion, sore throat, cough, shortness of breath (dyspnea), nausea, vomiting, and diarrhea).
As used herein, "suppression of exacerbation" includes suppression of an asymptomatic SARS-CoV-2-infected individual from being progressed to a severity classification of mild, moderate I, moderate II, or severe.
As used herein, "suppression of exacerbation" includes suppressing an asymptomatic or mild SARS-CoV-2-infected individual from progressing to a severity classification of moderate I, moderate II, or severe.
As used herein, "suppression of exacerbation" includes suppressing an asymptomatic, mild, or moderate I SARS-CoV-2-infected individual from progressing to a severity classification of moderate II or severe.
As used herein, "suppression of exacerbation" includes suppressing an asymptomatic, mild, moderate I, or moderate II SARS-CoV-2-infected individual from progressing to a severity classification of severe.
As used herein, "suppression of exacerbation" includes a reduction in the risk of hospitalization or death in a SARS-CoV-2-infected individual through the viral proliferation-suppression effect of the present agent.
As used herein, "suppression of exacerbation" includes a reduction in inflammation in the lungs of a SARS-CoV-2-infected individual through the viral proliferation-suppression effect of the present agent.
As used herein, "suppression of exacerbation" includes suppression of pneumonia caused by SARS-CoV-2 virus infection through the viral proliferation-suppression effect of the present agent.
As used herein, "suppression of exacerbation" includes suppression of an excessive immune response of the host, caused by SARS-CoV-2 virus infection, through the viral proliferation-suppression effect of the present agent.
In one aspect, the pharmaceutical composition of the present invention may be administered to a SARS-CoV-2-infected individual who has at least one exacerbation risk factor. Information regarding underlying medical conditions associated with exacerbation may be found, for example, in the summary provided by the U.S. Centers for Disease Control and Prevention (CDC) (https://www.cdc.gov/coronavirus/2019-ncov/hcp/clinical-care/underlyingconditions.html).
In one aspect, the pharmaceutical composition of the present invention may be administered to a SARS-CoV-2-infected individual who has at least one of the following exacerbation risk factors.
Exacerbation risk factors: malignant tumor, metabolic disease, cardiovascular mortality, respiratory disease, liver disease,

renal disease, neuropsychiatric disorder, physical inactivity, pregnancy, smoking, pediatric age, genetic disease, and immunodeficiency

In one aspect, the pharmaceutical composition of the present invention may be administered to an individual who has at least one exacerbation risk factors, and is used to suppress exacerbation of COVID-19 symptoms.

In one aspect, the pharmaceutical composition of the present invention is used in individuals having pneumonia with SARS-CoV-2.

As used herein, "prevention" includes suppression of the onset after exposure to SARS-CoV-2.

For example, the pharmaceutical composition of the present invention may be administered to a household member or cohabitant of an individual who has developed novel coronavirus infection.

For example, the pharmaceutical composition of the present invention may be administered to a contact promptly (e.g., within 72 hours) after contact with an individual with novel coronavirus infection.

For example, the pharmaceutical composition of the present invention may be administered to an asymptomatic SARS-CoV-2-infected individual to suppress the onset.

Further, the term "prevention" includes prevention before exposure to SARS-CoV-2.

For example, during a COVID-19 outbreak or in situations where there is a risk of SARS-CoV-2 infection, the pharmaceutical composition of the present invention may be administered to healthcare workers, elderly individuals, or individuals having exacerbation risk factors.

(Method for Producing Compound Represented by Formula (I-1))

The compound represented by Formula (I-1) can be produced by, for example, the general synthesis method described below. Regarding extraction, purification, and the like, the treatments carried out in ordinary experiments of organic chemistry may be carried out. The compound can be synthesized with reference to methods known in the field. Regarding extraction, purification, and the like, the treatments carried out in ordinary experiments of organic chemistry may be carried out.

The compound represented by Formula (I-1) can be produced with reference to techniques known in the art. The compound can be produced, for example, with reference to WO2012/020742, WO2013/118855, WO2023/195529, WO2023/195530, and the like.

(Method for Producing Compound Represented by Formula (II))

The compound represented by Formula (II) can be produced with reference to, for example, WO2023/195529.

Since the compound according to the present invention (for example, the compound represented by Formula (I-1), the compound represented by Formula (I-2), and the compound represented by Formula (II)) has coronavirus 3CL protease inhibitory activity, the compound is useful as a therapeutic agent and/or prophylactic agent for virus infections.

Furthermore, the compound according to the present invention has utility as a medicine, and preferably, the compound has any or a plurality of the following excellent features.

a) Inhibitory activity against CYP enzymes (for example, CYP1A2, CYP2C9, CYP2C19, CYP2D6, and CYP3A4) is weak.
b) Satisfactory pharmacokinetics such as high bioavailability and adequate clearance are exhibited.
c) Metabolic stability is high.
d) Irreversible inhibitory activity is not exhibited against CYP enzymes (for example, CYP3A4) within the concentration range of the measurement conditions described in the present specification.
e) Mutagenicity is not exhibited.
f) The cardiovascular risk is low.
g) High solubility is exhibited.
h) The protein unbinding rate (fu value) is high.
i) High coronavirus 3CL protease selectivity is exhibited.
j) High coronavirus replication inhibitory activity is exhibited. For example, high coronavirus proliferation inhibitory activity is exhibited when human blood serum (HS) or human serum albumin (HSA) is added.
k) High proliferation inhibitory activity is exhibited against 3CL protease inhibitor-resistant viruses.

Regarding the coronavirus proliferation inhibitor, for example, an aspect in which in the CPE effect (SARS-CoV-2) that will be described below, for example, $EC_{50}$ is 10 $\mu$M or less, preferably 1 $\mu$M or less, and more preferably 100 nM or less, may be mentioned.

The pharmaceutical composition according to the present invention can be administered by either an oral method or a parenteral method. Examples of a parenteral administration method include percutaneous, subcutaneous, intrave-

nous, intraarterial, intramuscular, intraperitoneal, transmucosal, inhalation, transnasal, ocular instillation, ear instillation, and intravaginal administration.

In the case of oral administration, the pharmaceutical composition may be prepared into any dosage form that is commonly used, such as a solid preparation for internal use (for example, a tablet, a powder preparation, a granular preparation, a capsule, a pill, or a film preparation), or a liquid preparation for internal use (for example, a suspension, an emulsion, an elixir, a syrup, a limonade, a spirit preparation, an aromatic water preparation, an extraction, a decoction, or a tincture) and administered. The tablet may be a sugar-coated tablet, a film-coated tablet, an enteric-coated tablet, a sustained release tablet, a troche, a sublingual tablet, a buccal tablet, a chewable tablet, or an orally disintegrating tablet; the powder preparation and granular preparation may be dry syrups; and the capsule may be a soft capsule, a microcapsule, or a sustained release capsule.

In the case of parenteral administration, the pharmaceutical composition can be suitably administered in any dosage form that is commonly used, such as an injectable preparation, an infusion, or a preparation for external use (for example, an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder preparation for external use, or a suppository). The injectable preparation may be an O/W, W/O, O/W/O, or W/O/W type emulsion, or the like.

A pharmaceutical composition can be obtained by mixing an effective amount of the compound according to the present invention with various pharmaceutical additives appropriate for the dosage form, such as a filler, a binder, a disintegrating agent, and a lubricant, as necessary. Furthermore, the pharmaceutical composition can also be provided as a pharmaceutical composition for pediatric use, elderly use, severely ill patients, or surgical use by appropriately modifying the effective amount, dosage form, and/or various pharmaceutical additives of the compound according to the present invention. For example, the pharmaceutical composition for pediatric use can be administered to neonates (less than 4 weeks after birth), infants (4 weeks after birth to younger than 1 year old), young children (1 year old or older to younger than 7 years old), children (7 years old or older to younger than 15 years old), or patients aged 15 to 18 years. For example, a pharmaceutical composition for an elderly may be administered to a patient 65 years of age or older.

It is desirable to set the amount of administration of the pharmaceutical composition of the present invention, after considering the age and body weight of the patient, the type and degree of the disease, the route of administration, and the like; however, in the case of oral administration, the amount of administration of the pharmaceutical composition as the compound according to the present invention is usually 0.01 to 100 mg/kg/day and is preferably in the range of 0.05 to 50 mg/kg/day. In the case of parenteral administration, the amount of administration may vary greatly depending on the route of administration; however, the amount of administration as the compound according to the present invention is usually 0.005 to 200 mg/kg/day and is preferably in the range of 0.01 to 100 mg/kg/day. This may be administered once a day or several times a day.

The compound of the present invention may be used in combination with, for example, another therapeutic drug for novel coronavirus infection (COVID-19) (the therapeutic drug includes an approved drug and a drug that is under development or to be developed in the future) (hereinafter, referred to as concomitant drug), for the purpose of enhancing the action of the compound, reducing the amount of administration of the compound, or the like. At this time, the timing of administration for the compound according to the present invention and the concomitant drug is not limited, and these may be administered simultaneously to the target of administration or may be administered with a time difference. Furthermore, the compound according to the present invention and the concomitant drug may be administered as two or more kinds of preparations each including active ingredients, or may be administered as a single preparation including those active ingredients.

The amount of administration of the concomitant drug can be appropriately selected based on the clinically used dosage. Furthermore, the blending ratio of the compound according to the present invention and the concomitant drug can be appropriately selected according to the target of administration, the route of administration, the target disease, symptoms, combination, and the like. For example, when the target of administration is a human being, 0.01 to 100 parts by weight of the concomitant drug may be used with respect to 1 part by weight of the compound according to the present invention.

## Examples

Hereinafter, the present invention will be described in more detail by way of Examples, Reference Examples, and Test Examples; however, the present invention is not intended to be limited by these.

Furthermore, abbreviations used in the present specification denote the following meanings.

FBS: Fetal bovine serum
mM: mmol/L
nM: nmol/L
μM: μmol/L

(Method for Identifying Compound)

The NMR analysis obtained in each Example was performed at 400 MHz, and measurement was made using DMSO-$d_6$ or $CDCl_3$. Furthermore, when NMR data are shown, there are occasions in which not all the measured peaks are described.

The term RT in the specification indicates retention time in an LC/MS: liquid chromatography/mass spectrometry, and the retention time was measured under the following conditions.

(Measurement Conditions A)

Column: ACQUITY UPLC (registered trademark) BEH C18 (1.7 μm, i.d. 2.1 × 50 mm) (Waters)
Flow rate: 0.8 mL/min
UV detection wavelength: 254 nm
Mobile phase: [A] is 0.1% formic acid-containing aqueous solution, and [B] is 0.1% formic acid-containing acetonitrile solution.
Gradient: A linear gradient of 5% to 100% solvent [B] was carried out for 3.5 minutes, and then 100% solvent [B] was maintained for 0.5 minutes.

X-Ray Powder Diffraction (XRPD) Experiment

X-ray Powder diffraction measurement of solid forms (crystals) obtained in each Example was performed according to the X-ray powder diffraction measurement method described in the General Testing Methods of the Japanese Pharmacopoeia. Measurement conditions are shown below.

Measurement Conditions 1:

X-ray Powder diffraction: SmartLab manufactured by Rigaku Corporation
Measuring method: Reflection method
Wavelength used: CuKα radiation (A = 1.5418Å)
Tube current: 200 mA
Tube voltage: 45 kV
Sample plate: Aluminum
Incident angle of X-rays: 2.5°
Sampling width: 0.02°
Detector: HyPix-3000 (two-dimensional detection mode)

Measurement and Analysis Method for Single Crystal Structure Analysis

The crystals obtained in the Examples were subjected to single crystal structure analysis. The measurement conditions and analysis method will be described below.

(Apparatus)

XtaLAB P200 MM007 manufactured by Rigaku Corporation

(Measurement Conditions)

Measurement temperature: 25°C
Temperature controller: Sample spraying low temperature apparatus manufactured by Rigaku Corporation
Wavelength used: CuKα radiation (A = 1.5418Å)
Software: CrysAlisPro 1.171.39.46e (Rigaku Oxford Diffraction, 2018)

(Data Processing)

Software: CrysAlisPro 1.171.39.46e (Rigaku Oxford Diffraction, 2018)

The data were subjected to Lorentz and polarization correction and absorption correction.

(Crystal Structure Analysis)

Phase determination was performed using a direct method program, ShelXT (Sheldrick, G.M., 2015), and regarding refinement, a full-matrix least squares method was carried out using ShelXL (Sheldrick, G.M., 2015). The temperature factors of non-hydrogen atoms were all subjected to refinement with anisotropy. Hydrogen atoms were computationally introduced using the default parameters of ShelXL and were treated as riding atoms. The hydrogen atoms were subjected to refinement with isotropic parameters.

PLATON (Spek, 1991)/ORTEP (Johnson, 1976) was used to draw the following structural drawing (30% PROBABILITY level).

Measurement of Raman Spectrum

Measurement of the Raman spectrum of crystals obtained in Examples and the measurement condition in which the baseline correction was performed are shown below.

Measurement Conditions 1

Measurement method: Microscopic laser Raman spectrometry
Laser wavelength: 671 nm
Cumulative number: Once
Exposure time: 1 second

Differential Scanning Calorimetry (DSC)

The DSC measurement of the crystals obtained in the Examples was performed. The sample was weighed into an aluminum pan and measured by simple sealing. The measurement conditions are shown below. In the measurement using the differential scanning calorimetry (DSC), an error may occur in the range of ±2°C.

Apparatus: Discovery DSC/TA Instrument
Measured temperature range: -10°C to 270°C
Rate of temperature increase: 10°C/min
Atmosphere: $N_2$ 50 mL/min

Simultaneous Thermogravimetric-Differential Thermal Analysis (TG/DTA)

The solid form (crystal) obtained in each Example was subjected to simultaneous thermogravimetric-differential thermal analysis (TG/DTA). Samples obtained in each Example were weighed and packed into an aluminum pan, and measurement was performed in an open system. Measurement conditions are as follows.

Apparatus: Hitachi High-Technologies TG/DTA STA7200RV
Measured temperature range: Room temperature tso -350°C
Rate of temperature increase: 10°C/min

HPLC Measurement

Instrument: Agilent 1290 Infinity VL
Column: Waters Acquity UPLC BEH C18, 1.7 μm, 2.1 × 100 mm
Column temperature: Constant temperature around 40°C
UV detection wavelength: 300 nm

Mobile phase: [A] is 0.1% formic acid-containing aqueous solution, and [B] is 0.1% formic acid-containing acetonitrile solution.
Gradient: Gradient was held at 20% solvent [B] for 1 minute, followed by a linear gradient from 20% to 80% solvent [B] over 32 minutes, and then held at 20% solvent [B] for 7 minutes.
Flow rate: 0.4 mL/min
Injection amount: 3 μL

Example 1

Synthesis of Compound (I-1)

[Chemical formula 46]

Step 1: Synthesis of Compound 3

To a mixture solution of a 2.64 mol/L n-butyllithium solution in hexane (31 mL, 82.4 mmol) and tetrahydrofuran (20 mL), a solution of Compound 1 (12 g, 68.7 mmol) in tetrahydrofuran (70 mL) was added dropwise over 15 minutes at -78°C,

and the resultant was stirred at -78°C for 1 hour. A solution of 1.9 mol/L zinc chloride in 2-methyltetrahydrofuran (43 mL, 82.4 mmol) was added dropwise over 5 minutes. The resultant was stirred at room temperature for 2 hours. Compound 2 (9.1 mL, 75.6 mmol) and tetrakis(triphenylphosphine)palladium (4.0 g, 3.44 mmol) were added and stirred at 80°C for 1.5 hours. The reaction solution was cooled to room temperature, water (80 mL) and 2 mol/L hydrochloric acid (40 mL) were added and subjected to extraction with ethyl acetate. The organic layer was concentrated under reduced pressure and to the resulting residue, isopropanol (40 mL) was added. The precipitate was filtered off and washed with isopropanol. After air drying, Compound 3 (14.8 g, 49 mmol) was obtained.

$^1$H-NMR($CDCl_3$)δ:3.95(s,3H), 4.05(s,3H), 7.18-7.23(m,2H), 7.35(d,J=6.8Hz,1H)
LC/MS(ESI):m/z=303, RT=2.70min, LC/MS Measurement Conditions A

Step 2: Synthesis of Compound 4

To Compound 3 (14.8 g, 48.8 mmol) acetic acid (40 mL) and concentrated hydrochloric acid (41 mL) were added and stirred at 110°C for 5 hours. The reaction solution was cooled to room temperature and then water (80 ml) was added. The precipitate was filtered off and washed with water. After air drying, Compound 4 (11.6 g, 42.2 mmol) was obtained.

$^1$H-NMR(DMSO-$d_6$)δ:7.31(ddd,J=8.8,4.9,2.1Hz,1H), 7.45(t,J=8.8Hz,1H), 7.53(dd,J=7.3,2.1Hz,1H), 11.57(s,1H), 12.24(brs,1H)
LC/MS (ESI):m/z=275, RT=1.81min, LC/MS Measurement Conditions A

Step 3: Synthesis of Compound 5

Compound 4 (1.00 g, 3.64 mmol), 5-chloropyridine-3-boronic acid (1.14 g, 7.27 mmol), copper (II) acetate (0.99 g, 5.45 mmol), acetonitrile (10 mL), triethylamine (5.04 mL, 36.4 mmol) and pyridine (7.34 mL, 91.0 mmol) were mixed, and the solution was stirred at room temperature overnight. A saturated aqueous sodium bicarbonate solution (5 mL) was added to the reaction solution, and the mixture was extracted with ethyl acetate. The mixture was extracted with ethyl acetate. The organic layer was washed with water, dried over sodium sulfate, and filtrated. The filtrate was concentrated, and the resulting residue was purified by silica gel column chromatography (chloroform:methanol = 100:0 to 90:10). The solvent was distilled off under reduced pressure. The resulting residue was dried under reduced pressure to give Compound 5 (1.15 g, 2.97 mmol, yield: 82%).

$^1$H-NMR(DMSO-$d_6$)
δ:7.35-7.37(1H,m),7.49(1H,t,J=9.0Hz),7.54-7.56(1H,m),8.07(1H,t,J=2.1Hz),8.54(1H,d,J=2.0Hz),8.70(1H,d,J=2 .3-Hz).
LC/MS (ESI):m/z=386, RT=1.98min, LC/MS Measurement Conditions A

Step 4: Synthesis of Compound 6

To a solution obtained by mixing Compound 5 (520 mg, 1.345 mmol), N,N-diisopropylethylamine (0.705 mL, 4.04 mmol), and DMF (5.2 mL) was added 2-bromoacetonitrile (269 μL, 4.04 mmol) and stirred overnight at room temperature. 2 mol/L hydrochloric acid (2 mL) was added to the reaction solution under ice-cooling, and the mixture was subjected to extraction with ethyl acetate. The organic layer was washed with water, dried over sodium sulfate and filtered. The filtrate was concentrated and the obtained residue was purified by silica gel column chromatography (chloroform:methanol=100:0 to 99:1), and the solvent was distilled off under reduced pressure. The resulting residue was dried in vacuo to yield Compound 6 (291 mg, 0.684 mmol, yield: 51%).

$^1$H-NMR($CDCl_3$)δ:5.13(2H,s),7.23-7.24(2H,m),7.42(1H,d,J=7.3Hz),7.67(1H,t,J=2.1Hz),8.45(1H,d, J=2.3Hz),8.67(1H,d,J=2.3Hz).
LC/MS (ESI):m/z=425, RT=2.17min, LC/MS Measurement Conditions A

Step 5: Synthesis of Compound (I-1)

Compound 6 (25.0 mg, 0.059 mmol), 6,6-difluoro-2-azaspiro[3.3]heptane trifluoroacetate (17.4 mg, 0.070 mmol), N,N-diisopropylethylamine (20.5 μL, 0.117 mmol), and DMF (0.5 mL) were mixed and the solution was stirred at 60°C for 2 hours. Water (2 mL) was added to the reaction solution and subjected to extraction with ethyl acetate. The organic layer was washed with water, dried over sodium sulfate, and filtered. The filtrate was concentrated and ethyl acetate (0.05 mL), hexane (0.125 mL) and diisopropylether (0.125 mL) were added. The resulting precipitate was filtered

off and washed with diisopropylether. The resulting solid was dried under reduced pressure to give an anhydrous crystal of Compound (I-1) (22.0 mg, 0.042 mmol, yield: 72%).

$^{1}$H-NMR($CDCl_3$)
δ:2.75(4H,t,J=12.0Hz),4.02(4H,s),4.74(2H,s),7.16-7.18(2H,m),7.32-7.35(1H,m),7.65(1H,t,J=2.1Hz),8.43(1H,d,-J=2.3Hz),8.61(1H,d,J=2 .3Hz).
LC/MS (ESI):m/z=522, RT=2.27min, LC/MS Measurement Conditions A

(Pulverization of Anhydrous Crystal of Compound Represented by Formula (I-1))

The anhydrous crystal of the compound represented by Formula (I-1) obtained in Example 1 was sieved through a 1000 μM mesh and then pulverized under the following conditions.

Apparatus: A-O jet mill (Seishin Kigyo Co., Ltd.)
Feeding method: Feeder
Feed rate: 20 g/hour
Pulverizing pressure: 0.30 MPa
Supply pressure: 0.40 MPa

(X-Ray Powder Diffraction Experiment of Anhydrous Crystal of Compound Represented by Formula (I-1))

An X-ray powder diffraction experiment was performed on the anhydrous crystal of the compound represented by Formula (I-1) after pulverization under measurement conditions described above. The X-ray powder diffraction pattern is shown in Fig. 7, and the peak list of the X-ray powder diffraction pattern is shown in Fig. 8. Hereinafter, in the table of the peak list of the X-ray powder diffraction pattern, "Position" indicates 2θ (°), and "Intensity" indicates intensity.
The X-ray powder diffraction pattern has peaks at diffraction angles (2θ) of 6.5°±0.2°, 10.1°±0.2°, 13.0°±0.2°, 14.1°±0.2°, 15.3°±0.2°, 15.6°±0.2°, 16.2°±0.2°, 17.4°±0.2°, 18.9°±0.2°, 19.9°±0.2°, 20.3°±0.2°, 21.7°±0.2°, 23.0°±0.2°, 23.8°±0.2°, 25.8°±0.2°, 28.8°±0.2°, and 30.6°±0.2°.
For the anhydrous crystal of the compound represented by Formula (I-1), the X-ray powder diffraction pattern has characteristic peaks at diffraction angles (2θ) of 6.5°±0.2°, 15.6°±0.2°, 17.4°±0.2°, 19.9°±0.2°, and 20.3°±0.2°.

(Single Crystal Structure Analysis of Anhydrous Crystal of Compound Represented by Formula (I-1))

<Method of Preparing Single Crystal>

To 1 mg of the crystal of the compound represented by Formula (I-1), 400 μL of methanol was added, and the mixture was heated to 50°C to dissolve the crystal. The solution was dispensed into a 1.5 mL HPLC vial, the HPLC vial was capped, a syringe needle was inserted through the cap, and the HPLC vial was allowed to stand at room temperature. Single crystals were prepared by solvent evaporation.

<Single Crystal Structure Analysis>

The single-crystal diffraction experiment and analysis were performed according to the method described above. It should be noted that Cl1 and Cl7C, and H5CA and H6CA are in a disorder relationship; therefore, the structure was analyzed with occupancies of Cl1:Cl7C = 0.75:0.25 and H5CA:H6CA = 0.25:0.75.
The results of the single crystal structure analysis are shown below. The $R_1$ (I>2.00s(I)) was 0.0555, and it was confirmed from the final difference Fourier map that no missing or misplaced electron density was present.
Crystallographic data are shown in Table 1.

[Table 1]

| Space group | Pbca |
|---|---|
| a (Å) | 14.6653 (7) |
| b (Å) | 11.8338 (5) |
| c (A) | 27.1024 (10) |
| α (°) | 90 |

(continued)

| Space group | Pbca |
|---|---|
| β (°) | 90 |
| γ (°) | 90 |
| V (Å$^3$) | 4703.5 (3) |
| Z | 8 |
| Density (calculated value) (g/cm$^3$) | 1.475 |
| Measurement temperature (K) | 298 |

Wherein V indicates the unit lattice volume, Z indicates chemical unit number per unit cell.

The fractional atomic coordinates x, y, and z (Å×10$^4$) and the equivalent isotropic displacement parameters U(eq)(Equivalent Isotropic Displacement Parameters, Å$^2$×10$^3$) for non-hydrogen atoms are shown in Table 2. Here, U(eq) is defined as one third of the trace of the orthogonalized $U_{ij}$ tensor.

The numbers of non-hydrogen atoms in Table 2 correspond to the numbers shown in Fig. 9, respectively.

[Table 2]

| Atom | x | y | z | U(eq) |
|---|---|---|---|---|
| Cl0A | 1162.1(10) | 9350.8(10) | 9005.2(4) | 134.2(5) |
| Cl1 | 754.6(13) | 6240.2(15) | 5416.9(8) | 158.6(8) |
| O0AA | 4165.4(12) | 8688.3(14) | 7965.5(7) | 62.4(5) |
| N4AA | 3011.5(14) | 8849.3(16) | 7408.6(8) | 55.0(5) |
| N1AA | 4332.8(13) | 7814.7(16) | 7227.4(8) | 53.0(5) |
| N2AA | 4551.4(15) | 6837.4(17) | 6491.3(8) | 59.9(5) |
| O1AA | 1886.9(15) | 9038(2) | 6844.4(8) | 97.5(8) |
| N0AA | 2003.5(18) | 11399.8(18) | 7952.6(10) | 76.3(7) |
| C1AA | 2516.9(17) | 9553.6(18) | 7746.5(9) | 52.9(6) |
| C2AA | 3850.1(17) | 8463.9(19) | 7561.7(10) | 53.1(6) |
| C3AA | 3996.7(17) | 7532.2(19) | 6757.1(9) | 54.5(6) |
| F4AA | 6218(2) | 3242(2) | 5837.2(11) | 158.4(11) |
| C5AA | 4960(2) | 5752(2) | 6663.9(10) | 62.6(7) |
| C6AA | 3179.9(18) | 7974(2) | 6609.7(9) | 61.2(7) |
| C7AA | 4998.9(18) | 5350(2) | 6131.3(10) | 61.4(7) |
| C8AA | 2142.6(19) | 9118(2) | 8169.5(10) | 63.0(7) |
| F9AA | 1731(3) | 7503(2) | 4713.3(10) | 186.6(16) |
| C0BA | 2430(2) | 10692(2) | 7651.1(11) | 66.1(7) |
| C1BA | 2644(2) | 8653(2) | 6937.4(10) | 65.7(7) |
| C2BA | 5281.1(18) | 7639(2) | 7371.6(12) | 69.6(8) |
| C3BA | 2787(2) | 7819(2) | 6105(1) | 70.5(8) |
| C4BA | 1677(2) | 9846(2) | 8473.2(11) | 70.1(7) |
| C5BA | 1632(2) | 10977(2) | 8357.7(12) | 73.9(8) |
| F6BA | 5565(3) | 3960(3) | 5212.6(9) | 180.9(14) |
| C7BA | 5403(2) | 6691(3) | 7713.5(13) | 79.8(9) |
| N3AA | 5508(2) | 5941(3) | 7971.4(13) | 114.7(12) |

(continued)

| **Atom** | **x** | **y** | **z** | **U(eq)** |
|---|---|---|---|---|
| C8BA | 4770(2) | 4133(3) | 5977.5(13) | 83.8(9) |
| C9BA | 2054(3) | 7103(3) | 6022.4(13) | 89(1) |
| C0CA | 4369(3) | 6326(3) | 6001.4(11) | 89.6(11) |
| C1CA | 5647(3) | 4080(3) | 5706.8(12) | 92.1(11) |
| C2CA | 5924(2) | 5216(3) | 5873.1(14) | 94.9(11) |
| C3CA | 3122(3) | 8436(3) | 5721.9(12) | 94.8(11) |
| C4CA | 2070(4) | 7603(4) | 5176.6(16) | 120.8(18) |
| C5CA | 1702(3) | 7000(4) | 5550.8(18) | 113.8(15) |
| C6CA | 2768(4) | 8325(4) | 5246.0(14) | 121.0(17) |
| CI7C | 2963(4) | 9116(5) | 4816.4(15) | 140.4(18) |

Next, the atomic coordinates x, y, and z (Å×$10^4$) and the isotropic displacement parameters U(eq) (Isotropic Displacement Parameters, $Å^2 \times 10^3$) for hydrogen atoms are shown in Table 3.

[Table 3]

| Atom | x | y | z | U(eq) |
|---|---|---|---|---|
| H5AA | 4557.64 | 5307.5 | 6871.27 | 75 |
| H5AB | 5554.99 | 5838.46 | 6816.05 | 75 |
| H8AA | 2201.83 | 8356.07 | 8247.86 | 76 |
| H0BA | 2680.93 | 10977.51 | 7361.78 | 79 |
| H2BA | 5508.61 | 8323.16 | 7525.87 | 84 |
| H2BB | 5642.53 | 7502.71 | 7077.87 | 84 |
| H5BA | 1329.65 | 11462.88 | 8572.27 | 89 |
| H8BA | 4731.05 | 3609.27 | 6252.06 | 101 |
| H8BB | 4237.54 | 4073.65 | 5766.58 | 101 |
| H9BA | 1801.24 | 6695.23 | 6281.91 | 107 |
| H0CA | 4582.75 | 6779.79 | 5726.84 | 108 |
| H0CB | 3738.83 | 6101.51 | 5953.94 | 108 |
| H2CA | 6021.1 | 5746.94 | 5605.7 | 114 |
| H2CB | 6439.24 | 5211.91 | 6097.8 | 114 |
| H3CA | 3596.24 | 8942.43 | 5778.24 | 114 |
| H5CA | 1213.16 | 6517.45 | 5491.34 | 137 |
| H6CA | 3008.16 | 8737.79 | 4984.33 | 145 |

The structure in the asymmetric unit of the crystal structure is shown in Fig. 9.

It should be noted that the label numbers of non-hydrogen atoms shown in Fig. 9 correspond to the numbers of non-hydrogen atoms in Table 2.

Since only one molecule of the compound represented by Formula (I-1) was present in the asymmetric unit of the crystal structure, the crystal was identified as an anhydrous crystal of the compound represented by Formula (I-1).

It was confirmed that the X-ray powder diffraction pattern (λ=1.5418 Å) calculated from the crystal structure using Mercury (The Cambridge Crystallographic Data Centre, Ver. 4.0.0) generally corresponds to the X-ray powder diffraction pattern (Fig. 7).

(Differential Scanning Calorimetry of Anhydrous Crystal of Compound Represented by Formula (I-1))

About 2 mg of the pulverized anhydrous crystal of the compound represented by Formula (I-1) was weighed into an aluminum pan, and measurement was performed according to the method described above. The results are shown in Fig. 10. An endothermic peak with an onset temperature of about 261.3°C was observed.

(Simultaneous Thermogravimetric-Differential Thermal Analysis of Anhydrous Crystal of Compound Represented by Formula (I-1))

For the pulverized anhydrous crystal of the compound represented by Formula (I-1), measurement was performed according to the method described above. The results are shown in Fig. 11. An endothermic peak with an onset temperature of about 265.6°C was observed. No weight loss was observed.

(Measurement of Raman Spectrum of Anhydrous Crystal of Compound Represented by Formula (I-1))

For the pulverized anhydrous crystal of the compound represented by Formula (I-1), Raman spectroscopy was performed under measurement conditions 1 described above. The results are shown in Fig. 12. The main Raman peaks are shown below.

[Table 4]

| Raman peak($cm^{-1}$) | Raman peak($cm^{-1}$) | Raman peak($cm^{-1}$) | Raman peak($cm^{-1}$) |
|---|---|---|---|
| 415.2 | 729.1 | 1100.1 | 1400.8 |
| 439.0 | 742.7 | 1109.7 | 1431.4 |
| 466.2 | 768.7 | 1133.3 | 1468.0 |
| 502.7 | 801.4 | 1145.0 | 1502.3 |
| 526.2 | 827.2 | 1162.0 | 1572.4 |
| 543.7 | 864.0 | 1194.9 | 1595.2 |
| 565.8 | 892.9 | 1213.9 | 1641.0 |
| 582.1 | 902.9 | 1227.6 | 1714.8 |
| 589.1 | 922.7 | 1241.2 | 2249.0 |
| 615.7 | 945.9 | 1275.8 | 2952.9 |
| 654.9 | 1024.5 | 1287.2 | 3065.4 |
| 681.2 | 1041.9 | 1307.0 | |
| 696.1 | 1059.2 | 1332.9 | |

For the anhydrous crystal of the compound represented by Formula (I-1), the Raman spectrum showed characteristic peaks at 415.2 $cm^{-1} \pm 2$ $cm^{-1}$, 502.7 $cm^{-1} \pm 2$ $cm^{-1}$, 1431.4 $cm^{-1} \pm$ 2 $cm^{-1}$, 1714.8 $cm^{-1} \pm 2$ $cm^{-1}$, and 3065.4 $cm^{-1}$ $\pm$ 2 $cm^{-1}$.

(HPLC Measurement of Anhydrous Crystal of Compound Represented by Formula (I-1))

For the pulverized anhydrous crystal of the compound represented by Formula (I-1), measurement was performed according to the method described above. The results are shown in Fig. 13 (about 96.5 pa%).

Example 2

Synthesis of Compound (I-2)

[Chemical formula 47]

Step 1: Synthesis of Compound 8

Compound 7 (2.6 g, 14.2 mmol), DMF (13 mL), and methylalcohol-$O^{18}$ (1.18 mL, 29.1 mmol) were mixed, and sodium hydride (1.42 g, 35.4 mmol) was slowly added under ice cooling. The reaction solution was warmed to room temperature and stirred for 2 hours. The reaction solution was cooled in an ice bath, and quenched by adding water (26 mL) and ethyl acetate (26 mL), followed by extraction with ethyl acetate. The organic layer was concentrated under reduced pressure, and the obtained residue was purified by silica gel column chromatography (hexane:ethyl acetate = 10:0 to 9:1). The solvent was distilled off under reduced pressure to give Compound 8 (1.57 g, 8.79 mmol, yield 62%).

LC/MS (ESI) m/z = 179, RT = 1.57 min, LC/MS measurement conditions A

$^1$H-NMR($CDCl_3$)δ:3.97(s,3H), 4.00(s,3H), 6.41(s,1H)

Step 2: Synthesis of Compound 10

Compound 9 (0.5 g, 3.43 mmol), deuterated water (17.1 mL, 945 mmol), and 5 mol/L DCl (0.69 mL, 3.43 mol) were mixed and heated at 180°C for 30 minutes using microwave. The same operation was repeated a total of 10 times. All reaction solutions were mixed, and 50 mL of ethyl acetate and 1 mol/L aqueous sodium hydroxide (51.5 mL) were added, followed by extraction with ethyl acetate. The resultant was washed with water and brine. The organic layer was concentrated under reduced pressure to give Compound 10 (5.02 g, 34 mmol, yield 99%).
$^1$H-NMR($CDCl_3$)δ:3.59(brs,2H), 6.87~6.95(m,1H)

Step 3: Synthesis of Compound 11

Compound 10 (5 g, 33.9 mmol) and water (11.5 mL) were mixed, and hydrogen bromide (11.5 mL, 102 mmol) was added under ice cooling. Thereafter, $NaNO_2$ (2.384 g, 34.6 mmol) dissolved in water (7.5 mL) was slowly added. The reaction solution was heated to 30°C, and CuBr (6.32 g, 44.0 mmol) dissolved in hydrogen bromide (13.42 mL, 119 mmol) was added while maintaining the internal temperature at 30 to 40°C. After stirring for 30 minutes, hexane (200 mL) and 5 mol/L aqueous sodium hydroxide solution (67.8 mL, 339 mmol) were added to the reaction solution. The reaction solution was filtered through Celite, and the filtrate was extracted with hexane. The organic layer was washed with water and brine, dried over magnesium sulfate, and filtered. The organic layer was concentrated under reduced pressure, and the obtained residue was purified by silica gel column chromatography (hexane:ethyl acetate = 10:0 to 9:1). The solvent was distilled off under reduced pressure to give Compound 11 (4.35 g, 15.1 mmol, 73.5 wt%). Since Compound 11 has a low boiling point and was used in the next reaction without distilling the solvent off.
$^1$H-NMR($CDCl_3$)δ:7.00-7.07(m,2H)

Step 4: Synthesis of Compound 12

A solution of Compound 8 (0.75 g, 4.2 mmol) in tetrahydrofuran (70 mL) was slowly added dropwise over 5 minutes at -78°C to a mixture solution obtained by mixing a solution of 2.76 mol/L n-butyllithium in hexane (1.8 mL, 5.04 mmol) and tetrahydrofuran (2.6 mL). The reaction solution was stirred at -78°C for 1 hour. To the reaction solution, a 1.9 mol/L solution of zinc chloride in 2-methyltetrahydrofuran (2.6 mL, 82.4 mmol) was added dropwise. The reaction solution was then stirred at room temperature for 2 hours. Compound 11 (1.2 g, 4.2 mmol) and tetrakis(triphenylphosphine) palladium (243 mg, 0.21 mmol) were then added to the reaction solution, and the mixture was stirred at 90°C for 1.5 hours. The reaction solution was cooled to room temperature, water (7.5 mL) was added, and the mixture was extracted with ethyl acetate. The organic layer was washed with water and brine, dried over magnesium sulfate, and filtered. The organic layer was concentrated under reduced pressure, and the obtained residue was purified by (hexane:ethyl acetate = 10:0 to 9:1). The solvent was distilled off under reduced pressure to give Compound 12 (374 mg, 1.2 mmol, 29% yield).

$^1$H-NMR($CDCl_3$)δ:3.95(s,3H), 4.05(s,3H), 7.20(d, J=9.0Hz,2H)

LC/MS (ESI):m/z=309, RT=2.51min, LC/MS Measurement Conditions A

Step 5: Synthesis of Compound 13

Compound 12 (374 mg, 1.2 mmol), acetonitrile (7.5 mL), NaI (181 mg, 1.2 mmol), and TMSCl (464 μL) were mixed, and the reaction solution was heated at 40°C. NaI (181 mg, 1.2 mmol) and TMSCl (464 μL) were added at approximately 30-minute intervals until the starting material was almost completely consumed. After completion of the reaction, water (15 mL) was added to the reaction solution, whereby a solid was precipitated. After partially concentrating the reaction solution, the solid was collected by filtration. The solid was washed with cold acetonitrile to give Compound 13 (246 mg, 0.88 mmol).

$^1$H-NMR(DMSO-$d_6$)δ:7.43(d,J=9.5Hz,2H), 7.53(dd,J=7.3,2.1Hz,1H), 11.53(s,1H), 12.23(br s,1H)

LC/MS(ESI):m/z=281, RT=1.44min, LC/MS Measurement Conditions A

Step 6: Synthesis of Compound 14

Compound 13 (250 mg, 0.89 mmol), 5-chloropyridine-3-boronic acid (280 mg, 1.78 mmol), copper(II) acetate

(242 mg, 1.33 mmol), acetonitrile (7.5 mL), triethylamine (1.23 mL, 8.89 mmol), and pyridine (1.8 mL, 22.2 mmol) were mixed, and the resulting solution was stirred at room temperature overnight. A 2 mol/L hydrochloric acid solution (44.5 mL) was added to the reaction solution, followed by extraction with ethyl acetate. The organic layer was washed with water, dried over magnesium sulfate, and filtrated. The filtrate was concentrated, and the obtained residue was used in the subsequent step without further purification.

Step 7: Synthesis of Compound 15

To a solution obtained by mixing Compound 14 (330 mg, 0.84 mmol), DIPEA (440 μL, 2.52 mmol), and DMF (3.3 mL), 2-bromoacetonitrile (168 μL, 2.52 mmol) was added. The resulting solution was stirred at room temperature overnight. Water (14 mL) was added to the reaction solution, followed by extraction with ethyl acetate. The organic layer was washed with water and brine, dried over magnesium sulfate, and filtered. The filtrate was concentrated, and the obtained residue was purified by silica gel column chromatography (hexane:ethyl acetate = 10:0 to 2:3). The solvent was distilled off under reduced pressure to give Compound 15 (307 mg, 0.71 mmol, 85% yield).

$^{1}$H-NMR($CDCl_3$)δ:5.13(s,2H),7.21-7.25(m,1H),

7.67(1H,t,J=2.3Hz),8.45(d,J=2.1Hz,1H),8.66(d, J=2.1Hz,1H). LC/MS (ESI):m/z=431, RT=2.13min, LC/MS Measurement Conditions A

Step 8: Synthesis of Compound (I-2)

Compound 15 (48.8 mg, 0.113 mmol), 6,6-difluoro-2-azaspiro[3.3]heptane trifluoro hydrochloride (33.5 mg, 0.136 mmol), DMF (0.5 mL), and N,N-diisopropylethylamine (59.2 μL, 0.339 mmol) were mixed, and the mixture was stirred at 60°C for 2 hours. Water (2 mL) was added to the reaction solution, followed by extraction with ethyl acetate. The organic layer was washed with water, dried over magnesium sulfate, and filtered. The filtrate was concentrated under reduced pressure, and the obtained residue was purified by silica gel column chromatography (hexane:ethyl acetate = 10:0 to 3:7). The solvent was distilled off under reduced pressure, and diisopropyl ether was added. The resulting precipitate was collected by filtration and washed with diisopropyl ether. The obtained solid was dried under reduced pressure to give Compound (I-2) (34.3 mg, 0.065 mmol, 57% yield).

$^{1}$H-NMR(DMSO-$d_6$)δ:2.83(t,J=12.5Hz,4H)4.07(s,4H), 4.86(s,2H),7.44(d,J=9.4Hz,1H), 8.01(t,J=2.2Hz,1H), 8.50(d,J=2.0Hz,1H), 8.73(d,J=2.0Hz,1H).
LC/MS (ESI):m/z=528, RT=2.19min, LC/MS Measurement Conditions A

(Reference Example 1)

Synthesis of Compound (II-1)

[Chemical formula 48]

16

II-1

Step 1: Synthesis of Compound (II-1)

Propargyl bromide (90 μL, 1.13 mmol) was added to a solution obtained by mixing Compound 16 (150 mg, 0.376 mmol; synthesis method described in International Publication WO2023/195529), N,N-diisopropylethylamine (0.197 mL, 1.13 mmol), and DMF (1.2 mL), and the mixture was stirred at 80°C for 4 hours. 6,6-difluoro-2-azaspiro[3.3]heptane trifluoro hydrochloride (186 mg, 0.753 mmol) and N,N-diisopropylethylamine (0.197 mL, 1.13 mmol) were added, and the solution was stirred at 80°C for 3 hours. A 0.1 mol/L aqueous hydrochloric acid solution (10 mL) was added to the reaction solution, followed by extraction with ethyl acetate. The organic layer was washed with water, dried over sodium sulfate, and filtered. The filtrate was concentrated, and the obtained residue was purified by silica gel column chromatography (hexane:ethyl acetate = 9:1 to 3:7). The solvent was distilled off under reduced pressure to give Compound (II-1) (57 mg, 0.109 mmol, 29% yield).

$^1$H-NMR($CDCl_3$)
δ:2.73-2.79(4H,m),3.45(1H,s),4.06(4H,s),4.57(2H,s),7.18-7.21(2H,m),7.36-7.43(2H,m),7.97(1H,t,J=2.0Hz),8.44(1H,d,J=2.0Hz),8.66(1H,d,J=2 .4Hz).
LC/MS (ESI):m/z=521, RT=2.37min, LC/MS Measurement Conditions A

(Reference Example 2)

The following compounds can be synthesized in the same manner as in Reference Example 1 and International Publication WO2023/195529.

[Chemical formula 49]

[Chemical formula 50]

, , , , , , , , , and

Biological test examples for the compounds according to the present invention will be described below.

It is desirable that the compound according to the present invention is a compound having coronavirus 3CL protease inhibitory activity and inhibiting coronavirus 3CL proteases.

Specifically, in the evaluation method described below, the $IC_{50}$ is preferably 50 μM or less, more preferably 1 μM or less, and even more preferably 100 nM or less. $EC_{50}$ is preferably 10 μM or less, more preferably 1 μM or less, and even more preferably 100 nM or less.

Test Example 1: Cytopathic Effect (CPE) Suppression Effect Confirmation Test Using HEK293T Cells Expressing Human TMPRSS2, ACE2 (HEK293T/ACE2-TMPRSS2 Cells)

<Operational Procedure>

Dilution and dispensing of sample to be tested

The sample to be tested is diluted in advance to an appropriate concentration with DMSO, and a 2- to 5-fold series of serial dilutions is prepared and then dispensed into a 384-well plate.

Dilution and dispensing of cells and SARS-CoV-2

HEK293T/ACE2-TMPRSS2 cells (GCP-SL222, $5\times10^3$ cells/well) and SARS-CoV-2 (200-600 $TCID_{50}$/well) are mixed in a medium (MEM, 2% FBS, penicillin-streptomycin), the mixture is dispensed into the wells in which the sample to be tested has been introduced, and then the cells are cultured for 3 days in a $CO_2$ incubator.

· Dispensing of CellTiter-Glo (registered trademark) 2.0 and measurement of luminescent signals

The plate that has been cultured for 3 days is returned to room temperature, subsequently CellTiter-Glo (registered trademark) 2.0 is dispensed into each well, and the plate is mixed using a plate mixer. After standing for a predetermined period of time, the luminescent signal (Lum) is measured using a plate reader.

<Calculation of Each Measurement Item Value>

· Calculation of 50% SARS-CoV-2 infected cell death inhibitory concentration ($EC_{50}$)

When x denotes the logarithmic value of the compound concentration and y denotes %Efficacy, the inhibition curve is approximated by the following Logistic regression equation, and the value of x when y = 50 (%) is inputted is calculated as $EC_{50}$.

$$y = min + (max - min)/\{1 + (X50/x)\ \hat{}Hill\}$$

%Efficacy = {(Sample - virus control) /(cell control - virus control)} * 100%

cell control: the average of Lum of cell control wells
virus control: the average of Lum of virus control wells
min: lower limit of y axis, max: upper limit of y axis, X50: x coordinate of inflection point, Hill: Slope of the curve at the midpoint between min and max

The compounds according to the present invention were tested essentially as described above. The $EC_{50}$ values are shown in the following.

(Results)

Compound I-1: 1.66 nM:
Compound I-2: 2.36 nM:

Test Example 2-1: Inhibitory Activity Test on SARS-CoV-2 3CL Protease

<Materials>

· Commercially available Recombinant SARS-CoV-2 3CL Protease

· Commercially available substrate peptide

Dabcyl-Lys-Thr-Ser-Ala-Val-Leu-Gln-Ser-Gly-Phe-Arg-Lys-Met-Glu(Edans)-$NH_2$ (SEQ ID NO: 1)

· Internal Standard peptide

Dabcyl-Lys-Thr-Ser-Ala-Val-Leu($^{13}C_6$,$^{15}N$)-Gln (SEQ ID NO: 2)

Dabcyl-Lys-Thr-Ser-Ala-Val-Leu($^{13}C_6$,$^{15}N$)-Gln can be synthesized with reference to documents (Atherton, E.; Sheppard, R. C., "In Solid Phase Peptide Synthesis, A Practical Approach", IRL Press at Oxford University Pres, 1989.; Bioorg.Med.Chem., Vol. 5, No. 9, 1997, pp. 1883-1891; and the like). An example will be shown below.

H-Lys-Thr-Ser-Ala-Val-Leu($^{13}C_6$,$^{15}N$)-Glu(resin)-OαOtBu (the Lys-side chain is Boc-protected, the Thr-side chain is protected with a tert-butyl group, the Ser-side chain is protected with a tert-butyl group, the C-terminal OH of Glu is protected with a tert-butyl group, and the carboxylic acid of the Glu-side chain is condensed into the resin) is synthesized by Fmoc solid-phase synthesis using a Rink amide resin. N-terminal Dabcyl group modification was carried out by coupling 4-dimethylaminoazobenzene-4'-carboxylic acid (Dabcyl-OH) on the resin using EDC/HOBT. Final deprotection and cleavage from the resin were carried out by treatment with TFA/EDT (95:5). Thereafter, purification is performed by reverse phase HPLC.

· RapidFire Cartridge C4 type A

<Operational Procedure>

· Preparation of assay buffer

In this test, an assay buffer consisting of 20 mM Tris-HCl, 1 mM EDTA, 10 mM DTT, and 0.01% BSA is used.

Dilution and dispensing of sample to be tested

The sample to be tested is diluted in advance to an appropriate concentration with DMSO, and a 2- to 5-fold series of serial dilutions is prepared and then dispensed into a 384-well plate.

Addition of enzyme and substrate, and enzymatic reaction

8 μM substrate and 6 nM or 0.6 nM enzymatic solution were added to the prepared compound plate, and the mixture was incubated at room temperature for 3 hours to 5 hours. Thereafter, a reaction quenching solution (0.067 μM, Internal Standard, 0.1% formic acid, and 10 or 25% acetonitrile) was added to stop the enzymatic reaction.

· Measurement of reaction product

The plate in which the reaction has been completed is measured using RapidFire System 360 and a mass analyzer (Agilent, 6550 iFunnel Q-TOF), or Rapid Fire System 365 and a mass analyzer (Agilent, 6495C Triple Quadrupole). Solution A (75% isopropanol, 15% acetonitrile, 5 mM ammonium formate) and solution B (0.01% trifluoroacetic acid, 0.09% formic acid) are used as a mobile phase at the measurement.

The reaction product detected by the mass analyzer is calculated using RapidFire Integrator or an equivalent program capable of analysis and is taken as Product area value. Furthermore, the Internal Standard that has been detected at the same time is also calculated and is designated as Internal Standard area value.

<Calculation of Each Measurement Item Value>

·Calculation of P/IS

The area values obtained in the previous items are calculated by the following formula, and P/IS is calculated.

P/IS = Product area value/Internal Standard area value · Calculation of 50% SARS-CoV-2 3CL protease inhibitory concentration ($IC_{50}$)

When x denotes the logarithmic value of the compound concentration and y denotes %Inhibition, the inhibition curve is approximated by the following Logistic regression equation, and the value of x obtainable when y = 50 (%) is inputted is calculated as $IC_{50}$.

$$y = min + (max - min)/\{1 + (X50/x)\ \hat{}Hill\}$$

%Inhibition = {1-(Sample - Control(-)) / Control(+)-Control(-))} * 100

Control(-):the average of P/IS of enzyme inhibited condition wells
Control(+):the average of P/IS of DMSO control wells
min: lower limit of y axis, max: upper limit of y axis, X50: x coordinate of inflection point, Hill: Slope of the curve at the midpoint between min and max

The compounds of the present invention were tested essentially as described above. The $IC_{50}$ values are shown in the following.

(Results)

Compound I-1: 0.00036 μM
Compound I-2: 0.00033 μM
Compound II-1: 0.00068 μM

Test Example 2-2: Inhibitory Activity Test on SARS-CoV-2 3CL Protease

<Materials>

· Commercially available Recombinant SARS-CoV-2 3CL Protease
· SARS-CoV-2 3CL Protease P132H
· Commercially available substrate peptide
Dabcyl-Lys-Thr-Ser-Ala-Val-Leu-Gln-Ser-Gly-Phe-Arg-Lys-Met-Glu(Edans)-$NH_2$ (SEQ ID NO: 1)
· Internal Standard peptide
Dabcyl-Lys-Thr-Ser-Ala-Val-Leu($^{13}C_6$,$^{15}N$)-Gln (SEQ ID NO: 2)

Dabcyl-Lys-Thr-Ser-Ala-Val-Leu($^{13}C_6$,$^{15}N$)-Gln can be synthesized with reference to documents (Atherton, E.; Sheppard, R. C., "In Solid Phase Peptide Synthesis, A Practical Approach", IRL Press at Oxford University Pres, 1989.; Bioorg.Med.Chem., Vol. 5, No. 9, 1997, pp. 1883-1891; and the like). An example will be shown below.

H-Lys-Thr-Ser-Ala-Val-Leu(13C6,15N)-Glu(resin)-OαOtBu (the Lys-side chain is Boc-protected, the Thr-side chain is protected with a tert-butyl group, the Ser-side chain is protected with a tert-butyl group, the C-terminal OH of Glu is protected with a tert-butyl group, and the carboxylic acid of the Glu-side chain is condensed into the resin) is synthesized by Fmoc solid-phase synthesis using a Rink amide resin. N-terminal Dabcyl group modification was carried out by coupling 4-dimethylaminoazobenzene-4'-carboxylic acid (Dabcyl-OH) on the resin using EDC/HOBT. Final deprotection and cleavage from the resin were carried out by treatment with TFA/EDT (95:5). Thereafter, purification is performed by reverse phase HPLC.

· RapidFire Cartridge C4 type A

<Operational Procedure>

Preparation of assay buffer

In this test, an assay buffer consisting of 20 mM Tris-HCl, 1 mM EDTA, 10 mM DTT, and 0.01% BSA is used.

· Dilution and dispensing of sample to be tested

The sample to be tested is diluted in advance to an appropriate concentration with DMSO, and a 3-fold series of serial dilutions is prepared and then dispensed into a 384-well plate.

Addition of enzyme and substrate, and enzymatic reaction

Substrate was added to the prepared compound plate to a final concentration of 4 μM, together with enzyme at 0.3 nM, and the mixture was incubated at room temperature for 4 hours. Thereafter, a reaction quenching solution (7.2 nM Internal Standard, 0.1% formic acid, and 10% acetonitrile) was added to stop the enzymatic reaction.

Measurement of reaction product

The plate in which the reaction has been completed is measured using Rapid Fire System 365 and a mass analyzer (Agilent, 6495C Triple Quadrupole). Solution A (75% isopropanol, 15% acetonitrile, 5 mM ammonium formate) and solution B (0.01% trifluoroacetic acid, 0.09% formic acid) are used as a mobile phase at the measurement.
The reaction product detected by the mass analyzer is calculated using RapidFire Integrator and is taken as Product area value. Furthermore, the Internal Standard that has been detected at the same time is also calculated and is designated as Internal Standard area value.

<Calculation of Each Measurement Item Value>

· Calculation of P/IS

The area values obtained in the previous items are calculated by the following formula, and P/IS is calculated.

P/IS = Product area value / Internal Standard area value · Calculation of 50% SARS-CoV-2 3CL protease inhibitory concentration ($IC_{50}$)

When x denotes the logarithmic value of the compound concentration and y denotes %Inhibition, the inhibition curve is approximated by the following Logistic regression equation, and the value of x obtainable when y = 50 (%) is inputted is calculated as $IC_{50}$.

$$y = min + (max - min)/\{1 + (X50/x)\ ^\wedge Hill\}$$

%Inhibition = {1-(Sample - Control(-)) / Control(+)-Control(-))} * 100

Control(-):the average of P/IS ratio in the wells without SARS-CoV-2 3CL protease and test substance
Control(+):the average of P/IS ratio in the wells with SARS-CoV-2 3CL protease and without test substance
min: lower limit of y axis, max: upper limit of y axis, X50: x coordinate of inflection point, Hill: Slope of the curve at the midpoint between min and max

The compounds according to the present invention were tested essentially as described above. The results are shown in the following.

[Table 5]

| 3CL protease mutation | Compound I - 1 $EC_{50}$ (nM) |
|---|---|
| WT | 0.655±0.062 |
| P132H | 0.767±0.041 |

Test Example 3: Inhibitory Activity of Cytopathic Effect (CPE) in VeroE6 Cells Expressing Human TMPRSS2 (VeroE6/TMPRSS2 Cells)

<Operational Procedure>

Dilution and dispensing of sample to be tested

The sample to be tested is diluted in advance to an appropriate concentration with DMSO, and a 3-fold series of serial dilutions is prepared and then dispensed into a 96-well plate.

Dilution and dispensing of cells and SARS-CoV-2

VeroE6/TMPRSS2 cells (JCRB1819, $1.5 \times 10^4$ cells/well) were mixed with SARS-CoV-2 strains hCoV-19/Japan/TY/WK-521/2020 (Ancestral), hCoV-19/Japan/QHN001/2020 (Alpha), hCoV-19/Japan/TY8-612/2021 (Beta), hCoV-19/Japan/TY7-501/2021 (Gamma), hCoV-19/Japan/TY11-927/2021 (Delta), hCoV-19/Japan/TY38-871/2021 (Omicron BA.1.1), hCoV-19/Japan/TY40-385/2022 (Omicron BA.2), hCoV-19/Japan/TY41-721/2022 (Omicron BA.2.12.1), hCoV-19/Japan/TY41-716/2022 (Omicron BA.2.75), hCoV-19/Japan/TY41-703/2022 (Omicron BA.4.1), hCoV-19/Japan/TY41-763/2022 (Omicron BA.4.6), hCoV-19/Japan/TY41-702/2022 (Omicron BE.1/BA.5-like), hCoV-19/Japan/TY41-704/2022 (Omicron BA.5.2.1), hCoV-19/Japan/TY41-820/2022 (Omicron BF.7), hCoV-19/Japan/TY41-828/2022 (Omicron BF.7.4.1), hCoV-19/Japan/TY41-796/2022 (Omicron BQ.1.1), hCoV-19/Japan/TY41-832/2022 (Omicron CH.1.1.11), hCoV-19/Japan/TY41-795/2022 (Omicron XBB.1), hCoV-19/Japan/23-018/2022 (Omicron XBB.1.5), hCoV-19/Japan/TY-41951/2023 (Omicron XBB.1.9.1), hCoV-19/Japan/TY41-984/2023 (Omicron XBB.1.16), hCoV-19/Japan/TY41-831/2022 (Omicron XBF), and hCoV-19/Japan/TY41-686/2022 (Omicron XE) (30-3000 $TCID_{50}$/well) in medium (MEM supplemented with 2% FBS and penicillin-streptomycin). The mixture was dispensed into wells containing the test sample and incubated in a $CO_2$ incubator for 3 or 4 days.

· Dispensing of CellTiter-Glo (registered trademark) 2.0 and measurement of luminescent signals

The plate that has been cultured for 3 days is returned to room temperature, subsequently CellTiter-Glo (registered trademark) 2.0 is dispensed into each well, and the plate is mixed using a plate mixer. After standing for a predetermined period of time, the luminescent signal (Lum) is measured using a plate reader.

<Calculation of Each Measurement Item Value>

· Calculation of 50% SARS-CoV-2 infected cell death inhibitory concentration ($EC_{50}$)

When x denotes the logarithmic value of the compound concentration and y denotes %Efficacy, the inhibition curve is approximated by the following Logistic regression equation, and the value of x when y = 50 (%) is inputted is calculated as $EC_{50}$.

$$y = min + (max - min)/\{1 + (X50/x)\ \hat{}Hill\}$$

%Efficacy = {(Sample - virus control) / (cell control - virus control)} * 100%

cell control: the average of Lum of cell control wells
virus control: the average of Lum of virus control wells
min: lower limit of y axis, max: upper limit of y axis, X50: x coordinate of inflection point, Hill: Slope of the curve at the midpoint between min and max

The compounds according to the present invention were tested essentially as described above. The results are shown in the following.

[Table 6]

| SARS-CoV-2 strain | Pango lineage | Strain name | Compound I - 1 $EC_{50}$ (nM) |
|---|---|---|---|
| Ancestral | A | WK-521 | 8.77±1.92 |
| Alpha | B. 1. 1. 7 | QHN002 | 3.85±1.41 |
| Beta | B. 1. 351 | TY8-612 | 9.64±1.20 |
| Gamma | P. 1 | TY7-501 | 8.91±1.62 |
| Delta | AY. 122 | TY11-927 | 4.91±1.29 |
| Omicron | BA. 1. 1 | TY38-871 | 2.63+0.18 |
| Omicron | BA. 2 | TY40-385 | 7.38±3.82 |

(continued)

| SARS-CoV-2 strain | Pango lineage | Strain name | Compound I - 1 $EC_{50}$ (nM) |
|---|---|---|---|
| Omicron | BA. 2. 12. 1 | TY41-721 | 2.27±0.26 |
| Omicron | BA. 2. 75 | TY41-716 | 8.44±1.25 |
| Omicron | BA. 4. 1 | TY41-703 | 2.45±0.40 |
| Omicron | BA. 4. 6 | TY41-763 | 6.91±1.21 |
| Omicron | BE. 1/BA. 5-like | TY41-702 | 6.54±3.49 |
| Omicron | BA. 5. 2. 1 | TY41-704 | 6.80±1.38 |
| Omicron | BF. 7 | TY41-820 | 9.49±0.56 |
| Omicron | BF. 7. 4. 1 | TY41-828 | 7.39±1.97 |
| Omicron | BQ. 1. 1 | TY41-796 | 11.0±1.7 |
| Omicron | CH. 1. 1. 11 | TY41-832 | 5.69±2.44 |
| Omicron | XBB. 1 | TY41-795 | 5.11±2.15 |
| Omicron | XBB. 1. 5 | 23-018 | 7.40±1.08 |
| Omicron | XBB. 1. 9. 1 | TY41-951 | 12.6±0.9 |
| Omicron | XBB. 1. 16 | TY41-984 | 7.06±2.00 |
| Omicron | XBF | TY41-831 | 6.27±3.30 |
| Omicron | XE | TY41-686 | 7.10±2.59 |

Test Example 5: Antiviral Effect Against HCoV-OC43

<Operational Procedure>

· Dilution and dispensing of sample to be tested

The sample to be tested is diluted in advance to an appropriate concentration with DMSO, and a 3-fold series of serial dilutions is prepared and then dispensed into a 96-well plate and diluted with maintenance medium (MEM supplemented with 2% FBS and penicillin-streptomycin).

Dilution and dispensing of cells and HCoV-OC43

MRC-5 cells ($2\times10^4$ cells/well) suspended in growth medium (DMEM supplemented with 10% FBS and penicillin-streptomycin) were seeded into 96-well plates on the day before infection. On the following day, HCoV-OC43 (300 $TCID_{50}$/well) suspended in maintenance medium (MEM supplemented with 2% FBS and penicillin-streptomycin) was added to infect the cells for 1 hour. The viral fluid is then removed, maintenance medium containing the reagent to be tested is added, and cultured for 72 hours in a $CO_2$ incubator. To evaluate the cytotoxicity of the reagent to be tested, the same procedure was performed in the absence of virus.

· Dispensing of CellTiter-Glo (registered trademark) 2.0 and measurement of luminescent signals

The plate that has been cultured for 72 hours is returned to room temperature, subsequently CellTiter-Glo (registered trademark) 2.0 is dispensed into each well, and the plate is mixed using a plate mixer. After standing for a predetermined period of time, the luminescent signal (Lum) is measured using a plate reader.

<Calculation of Each Measurement Item Value>

· Calculation of 50% HCoV-OC43 infected cell death inhibitory concentration ($EC_{50}$)

When x denotes the logarithmic value of the compound concentration and y denotes %Efficacy, the inhibition curve is approximated by the following Logistic regression equation, and the value of x when y = 50 (%) is inputted is calculated as $EC_{50}$.

$$y = min + (max - min)/\{1 + (X50/x)\ ^{\wedge}Hill\}$$

%Efficacy = {(Sample - virus control)/(cell control - virus control)} * 100%

cell control: the average of Lum of cell control wells
virus control: the average of Lum of virus control wells
min: lower limit of y axis, max: upper limit of y axis, X50: x coordinate of inflection point, Hill: Slope of the curve at the midpoint between min and max

· Calculation of 50% cytotoxicity concentration

When x denotes the logarithmic value of the compound concentration and y denotes %Cytotoxicity, the inhibition curve is approximated by the following Logistic regression equation, and the value of x when y = 50 (%) is inputted is calculated as $CC_{50}$.

$$y = min + (max - min)/\{1 + (X50/x)\ ^{\wedge}Hill\}$$

%Cytotoxicity = {(Sample - medium control) / (cell control - medium control)} * 100%

cell control: the average of Lum of cell control wells
medium control: the average of Lum of medium control wells
min: lower limit of y axis, max: upper limit of y axis, X50: x coordinate of inflection point, Hill: Slope of the curve at the midpoint between min and max

The compounds according to the present invention were tested essentially as described above. The results are shown in the following.

Compound I-1: $EC_{50}$ 92.0±21.0 nM, $CC_{50}$ > 100 μM

Test Example 6: Antiviral Activity Against HCoV-229E

<Operational Procedure>

Dilution and dispensing of sample to be tested

The sample to be tested is diluted in advance to an appropriate concentration with DMSO, and a 3-fold series of serial dilutions is prepared and then dispensed into a 96-well plate and diluted with maintenance medium (MEM supplemented with 2% FBS and penicillin-streptomycin).

Dilution and dispensing of cells and HCoV-229E

MRC-5 cells ($2\times10^4$ cells/well) suspended in growth medium (DMEM supplemented with 10% FBS and penicillin-streptomycin) were seeded into 96-well plates on the day before infection. On the following day, HCoV-229E (1000 $TCID_{50}$/well) suspended in maintenance medium (MEM supplemented with 2% FBS and penicillin-streptomycin) was added to infect the cells for 1 hour. The viral fluid is then removed, maintenance medium containing the reagent to be tested is added, and cultured for 72 hours in a $CO_2$ incubator.

· Dispensing of CellTiter-Glo (registered trademark) 2.0 and measurement of luminescent signals

The plate that has been cultured for 72 days is returned to room temperature, subsequently CellTiter-Glo (registered trademark) 2.0 is dispensed into each well, and the plate is mixed using a plate mixer. After standing for a predetermined period of time, the luminescent signal (Lum) is measured using a plate reader.

<Calculation of Each Measurement Item Value>

· Calculation of 50% HCoV-229E infected cell death inhibitory concentration ($EC_{50}$)

When x denotes the logarithmic value of the compound concentration and y denotes %Efficacy, the inhibition curve is approximated by the following Logistic regression equation, and the value of x when y = 50 (%) is inputted is calculated as $EC_{50}$.

$$y = min + (max - min)/\{1 + (X50/x)\ \hat{}Hill\}$$

%Efficacy = {(Sample - virus control)/(cell control - virus control)} * 100%

cell control: the average of Lum of cell control wells
virus control: the average of Lum of virus control wells
min: lower limit of y axis, max: upper limit of y axis, X50: x coordinate of inflection point, Hill: Slope of the curve at the midpoint between min and max

The compounds according to the present invention were tested essentially as described above. The results are shown in the following.

Compound I-1: $EC_{50}$ 3570±510 nM

Test Example 7: Effect of Human, Mouse, and Hamster Sera on Anti-SARS-CoV-2 Activity

<Operational Procedure>

Dilution and dispensing of sample to be tested

The sample to be tested is diluted in advance to an appropriate concentration with DMSO, and a 3-fold series of serial dilutions is prepared and then dispensed into a 96-well plate.

Addition of serum addition medium

Media (MEM, 2% FBS, penicillin-streptomycin) were prepared so that the final concentrations of human serum or mouse serum or hamster serum was 0, 12.5, 25, or 50%, and the prepared media were dispensed into wells containing the test sample, followed by incubation at room temperature for 1 hour.

· Dilution and dispensing of cells and SARS-CoV-2

VeroE6/TMPRSS2 cells (JCRB1819, $1.5 \times 10^4$ cells/well) were seeded into a 96-well plate on the day prior to infection. On the following day, the cells were infected for 1 hour with SARS-CoV-2 hCoV-19/Japan/TY7-501/2021 (1000$TCID_{50}$/well) suspended in maintenance medium (MEM, 2% FBS, penicillin-streptomycin). Thereafter, the virus-containing fluid was removed, and medium containing the test compound and human serum, mouse serum, or hamster serum was added, followed by incubation for 1 day in a $CO_2$ incubator.

Determination of viral load

After incubation for 1 day, the supernatant was removed from the plate, and a cell lysis solution prepared by mixing Trizol LS and maintenance medium at a ratio of 3:1 was added. RNA was extracted using a Direct-zol-96 RNA Kit (ZYMO RESEARCH). The extracted RNA solution was then quantified by real-time PCR (Applied Biosystems Quant-Studio5). The following probes and primers were used.

[Table 7]

| Role | Primer name | Sequence (5’ →3’ ) |
|---|---|---|
| Probe | 2019-nCoV_N1-P | FAM-ACCCCGCATTACGTTTGGTGGACC-TAMRA |
| Forward primer | 2019-nCoV_N1-F | GACCCCAAAATCAGCGAAAT |

(continued)

| Role | Primer name | Sequence (5' →3' ) |
|---|---|---|
| Reverse primer | 2019-nCoV_N1-R | TCTGGTTACTGCCAGTTGAATCTG |

<Calculation of Each Measurement Item Value>

· Calculation of 90% SARS-CoV-2 virus replication inhibitory concentration ($EC_{90}$)

In a concentration-dependent curve in which x represents the logarithmic value of compound concentration and y represents the viral copy number ($Log_{10}$ copies/mL), the value corresponding to a 1 $log_{10}$ reduction from the virus control was calculated by a two-point interpolation method using the copy numbers at the two concentrations immediately above and below the target value. In other words,

X = the lowest concentration at the mean reduction of viral RNA copy number from that of virus control is less than z
x = the highest concentration at the mean reduction of viral RNA copy number from that of virus control is z or more.
Y = the mean of logarithmic reduction of viral RNA copy number from that of virus control at X
y = the mean of logarithmic reduction of viral RNA copy number from that of virus control at x
$EC_{90}$ values were calculated by the following formula.

$$EC_{90} = 10[\log(x) + (\log(X) - \log(x)) \times (y - z)/(y - Y)]$$

$$z = \log_{10}(10/100)$$

· Calculation of Protein-Adjusted $EC_{90}$ (PA-$EC_{90}$)

After calculation of the $EC_{90}$ at each serum concentration, the PA-$EC_{90}$ value under the 100% serum condition was calculated by linear regression analysis.

The compounds according to the present invention were tested essentially as described above. The results are shown in the following.

Compound I-1:

Human serum PA-$EC_{90}$ 7.90 nM

Mouse serum PA-$EC_{90}$ 19.8 nM

Hamster serum PA-$EC_{90}$ 8.17 nM

Test Example 8: Evaluation Test of Viral Proliferation-Suppression Effect Using Human Airway Epithelial Cells

<Operational Procedure>

Dilution and dispensing of sample to be tested

The sample to be tested was first diluted to an appropriate concentration with DMSO and subjected to a 3-fold serial dilution. The resulting dilution series was then diluted 200-fold with MucilAir™ culture medium and dispensed into a 24-well plate.

· SARS-CoV-2 infection

MucilAir™ (Nasal cavity, about $5.0 \times 10^5$ cells/well) cultured on a Transwell insert was inoculated with SARS-CoV-2 hCoV-19/Japan/TY41-702/2022 (Omicron BE.1/BA.5-like) (5000 $TCID_{50}$/well) and incubated in a $CO_2$ incubator for 2 hours. Subsequently, the transwell insert was washed with MucilAir™ culture solution, transferred to wells containing the test sample, and cultured in a $CO_2$ incubator. At 2 days after infection, MucilAir™ culture solution was added to the transwells, and the resulting supernatant was collected.

Measurement of viral titers in supernatants

The collected supernatants were subjected to 10-fold serial dilutions in medium (MEM supplemented with 2% FBS and penicillin-streptomycin), mixed with VeroE6/TMPRSS2 cells (JCRB1819, $1.5 \times 10^4$ cells/well), and seeded into 96-well plates. After incubation in a $CO_2$ incubator for 4 days, Cytopathic effects (CPE) were observed, and the viral titers present in the supernatants were determined.

<Calculation of Each Measurement Item Value>

· Calculation of 90% SARS-CoV-2 viral production inhibitory concentration ($EC_{90}$)

In a concentration-dependent curve where x represents the logarithm of the compound concentration and y represents the viral titer ($Log_{10}$ $TCID_{50}$/mL), the concentration corresponding to a 1 $log_{10}$ reduction relative to the virus control was determined by a two-point interpolation method based on the viral titers at the two concentrations flanking the calculated value.

[Math. 1]

X = the lowest conc. at the average of reduction from control viral titer of $<-1$

x = the highest conc. at the average of reduction from control viral titer of $\geq-1$

Y = the average of reduction from control viral titer at x

y = the average of reduction from control viral titer at X

$EC_{90}$ value were calculated by the following formula.

$$EC_{90} = 10^{[\log(x) + (-1 - Y) \times (\log(x) - \log(X)) / (Y - y)]}$$

The compounds according to the present invention were tested essentially as described above. The results are shown in the following.

[Table 8]

| SARS-CoV-2 strain | Pango lineage | Strain name | Compound I-1 $EC_{90}$ (nM) |
|---|---|---|---|
| Omicron | BE.1/BA.5-like | TY41-702 | $2.41 \pm 1.61$ |

Test Example 9: Test on Suppression of Increase in Viral Titer in Lung Homogenates of SARS-CoV-2-Infected Mice by Delayed Administration of Compound I-1

<Materials and Methods>

Compound

Compound I-1 according to the present invention was used as a test sample using N,N-dimethylacetamide (DMA) and polyethylene glycol 400 (PEG400) containing 0.5 w/v% poly(1-vinylpyrrolidone-co-vinyl acetate) (PVPVA) (DMA: PEG400 containing 0.5 w/v% PVPVA = 1:9). The dose volume was 5 mL/kg.

· Virus

SARS-CoV-2 hCoV-19/Japan/TY7-501/2021 strain isolated at the National Institute of Infectious Diseases was used.

· Lung infection in mice, drug administration, and lung collection

Specific pathogen-free 5-week-old female BALB/c mice (CLEA Japan, Inc.) were used in this study. For viral

inoculation, the mice were anesthetized by intramuscular administration of 100 μL of an anesthetic solution in Saline containing 0.03 mg/mL medetomidine hydrochloride, 0.4 mg/mL midazolam, and 0.5 mg/mL butorphanol tartrate. Under anesthesia, 50 μL of hCoV-19/Japan/TY7-501/2021 ($1.00 \times 10^4$ $TCID_{50}$) was intranasally inoculated into the mice. Beginning 1 day after viral infection, Compound I-1 was orally administered to the mice (n=5 per group) twice daily at doses of 0.1, 0.3, 1, 3, and 10 mg/kg. DMA/0.5 w/v% PVPVA in PEG400 was orally administered to control mice twice daily. Compound administration was 2 days from the start of administration. At 3 days after infection, the lungs of infected mice were collected and supplemented with 2 mL of PBS, followed by homogenization and recovery of the supernatant after centrifugation.

Measurement of viral titers in lung homogenates

Lung homogenates were subjected to 10-fold serial dilutions in medium (MEM supplemented with 2% FBS and penicillin-streptomycin), mixed with VeroE6/TMPRSS2 cells (JCRB1819, $1.5 \times 10^4$ cells/well), and seeded into 96-well plates. After incubation in a $CO_2$ incubator for 4 days, Cytopathic effects (CPE) were observed, and the viral titers present in the lung homogenates were determined.

The compounds according to the present invention were tested essentially as described above. The results are shown in the following.

At 3 days after infection, viral titers in lung homogenates were 6.45-$log_{10}$ $TCID_{50}$/mL in the DMA/0.5 w/v% PVPVA in PEG400 administration group and 6.27, 5.39, 3.97, 2.51, and 2.47-$log_{10}$ $TCID_{50}$/mL in the Compound I-1-administered groups at doses of 0.1, 0.3, 1, 3, and 10 mg/kg, respectively. In the Compound I-1-administered groups, the viral titers in lung homogenates were dose-dependently lower than those in the DMA/0.5 w/v% PVPVA in PEG400-administered group. These results suggest that Compound I-1 reduces viral titers in lung homogenates even when there is a delay between infection and administration (Fig. 1).

Test Example 10: Tests on Suppression of Increase in Viral Titer and on Suppression of Increase in Lung Weight in Lung and Nasal Turbinate Homogenates of SARS-CoV-2-Infected Hamsters by Delayed Administration of Compound I-1

<Materials and Methods>

Compound

Compound I-1 according to the present invention was used as a test sample using N,N-dimethylacetamide (DMA) and polyethylene glycol 400 (PEG400) containing 0.5 w/v% poly(1-vinylpyrrolidone-co-vinyl acetate) (PVPVA) (DMA: PEG400 containing 0.5 w/v% PVPVA = 1:9). The dose volume was 2.5 mL/kg.

· Virus

SARS-CoV-2 hCoV-19/Japan/TY41-702/2022 strain (Omicron BE.1/BA.5-like) isolated at the National Institute of Infectious Diseases was used.

· Lung infection in hamsters, drug administration, and collection of lung and nasal turbinate

Specific pathogen-free 6-week-old male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 μL of hCoV-19/Japan/TY41-702/2022 ($1.00 \times 10^4$ $TCID_{50}$) was intranasally inoculated. In infected hamsters, oral administration of Compound I-1 was initiated 1 day after viral inoculation and performed twice daily at doses of 0.1, 1, and 10 mg/kg. DMA/0.5 w/v% PVPVA in PEG400 was orally administered twice daily to infected control hamsters and non-infected hamsters. Compound administration was five days after the start of administration. At 3 or 4 days after infection, the lungs and nasal turbinates of infected hamsters were collected. 5 mL or 1 mL of PBS was added thereto, followed by homogenization, and the supernatant was collected after centrifugation. At 7 days after infection, lungs were collected from both infected and non-infected hamsters, and the lung weights were determined.

· Measurement of viral titers in lung and nasal turbinate homogenates

Lung and nasal turbinate homogenates were subjected to 10-fold serial dilutions in medium (MEM supplemented with 2% FBS and penicillin-streptomycin), mixed with VeroE6/TMPRSS2 cells (JCRB1819, $1.5 \times 10^4$ cells/well), and

seeded into 96-well plates. After incubation in a $CO_2$ incubator for 4 days, Cytopathic effects (CPE) were observed, and the viral titers present in the nasal turbinate homogenates were determined.

The compounds according to the present invention were tested essentially as described above. The results are shown in the following.

The viral titers in lung homogenates at 3 days after infection were 5.4 $log_{10}$ $TCID_{50}$/mL in the DMA/0.5 w/v% PVPVA in PEG400-administered group and 5.5, 3.5, and 2.5-$log_{10}$ $TCID_{50}$/mL in the Compound I-1-administered groups at doses of 0.1, 1, and 10 mg/kg, respectively. At 4 days after infection, the viral titers were 5.6-$log_{10}$ $TCID_{50}$/mL in the DMA/0.5 w/v% PVPVA in PEG400-administered group and 4.6, 2.4, and 1.9-$log_{10}$ $TCID_{50}$/mL in the Compound I-1-administered groups at doses of 0.1, 1, and 10 mg/kg, respectively.

The viral titers in nasal turbinate homogenates at 3 days after infection were 5.0-$log_{10}$ $TCID_{50}$/mL in the DMA/0.5 w/v% PVPVA in PEG400-administered group and 4.8, 3.2, and 2.7-$log_{10}$ $TCID_{50}$/mL in the Compound I-1-administered groups at doses of 0.1, 1, and 10 mg/kg, respectively. At 4 days after infection, the viral titers were 3.8-$log_{10}$ $TCID_{50}$/mL in the DMA/0.5 w/v% PVPVA in PEG400-administered group and 4.0, 2.2, and 1.8-$log_{10}$ $TCID_{50}$/mL in the Compound I-1-administered groups at doses of 0.1, 1, and 10 mg/kg, respectively.

In the Compound I-1-administered groups, the viral titers in lung and nasal turbinate homogenates were dose-dependently lower than those in the DMA/0.5 w/v% PVPVA in PEG400-administered group. These results suggest that Compound I-1 reduces viral titers in lung and nasal turbinate homogenates even when there is a delay between infection and administration (Figs. 2-1 and 2-2).

At 7 days after infection, the lung weight/body weight was 5.26 mg/g in non-infected hamsters, 8.96 mg/g in the DMA/0.5 w/v% PVPVA in PEG400-administered group, and 8.24, 5.75, and 5.57 mg/g in the Compound I-1-administered groups at doses of 0.1, 1, and 10 mg/kg, respectively.

In the Compound I-1-administered groups, the lung weight/body weight was decreased in a dose-dependent manner compared with the DMA/0.5 w/v% PVPVA in PEG400-administered group. These results suggest that administration of Compound I-1 suppresses the increase in lung weight induced by SARS-CoV-2 infection (Fig. 3).

Test Example 11: Body Weight Loss Suppression Test for SARS-CoV-2 Infected Hamsters by Administration of Compound (I-1)

<Materials and Methods>

· Compound

Compound I-1 according to the present invention was used as a test sample using N,N-dimethylacetamide (DMA) and polyethylene glycol 400 (PEG400) containing 0.5 w/v% poly(1-vinylpyrrolidone-co-vinyl acetate) (PVPVA) (DMA: PEG400 containing 0.5 w/v% PVPVA = 1:9). The dose volume was 2.5 mL/kg.

· Virus

SARS-CoV-2 hCoV-19/Japan/TY41-702/2022 strain (Omicron BE.1/BA.5-like) isolated at the National Institute of Infectious Diseases was used.

· Hamster lung infection, drug administration, and body weight measurement

Specific pathogen-free 6-week-old male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 $\mu$L of hCoV-19/Japan/TY41-702/2022 ($1.00 \times 10^4$ $TCID_{50}$) was intranasally inoculated. In infected hamsters, oral administration of Compound I-1 was initiated 1 day after viral inoculation and performed twice daily at doses of 0.1, 1, and 10 mg/kg. DMA/0.5 w/v% PVPVA in PEG400 was orally administered twice daily to infected control hamsters and non-infected hamsters. Compound administration was five days after the start of administration. Body weight was monitored once daily.

The compounds according to the present invention were tested essentially as described above. The results are shown in the following.

Non-infected hamsters exhibited an increase in body weight, while infected hamsters showed a decrease in body weight beginning 3 days after infection, reaching the lowest level at 6 days after infection. In the Compound I-1-administered groups at doses of 1 and 10 mg/kg, all hamsters survived until 10 days after infection, and the decrease in body weight was suppressed (Fig. 4).

The above results suggest that administration of Compound I-1 suppresses body weight loss.

Test Example 12: Test on Suppression of Body Weight Loss and Inhibition of Viral Proliferation in Lung and Nasal Turbinate Homogenates in SARS-CoV-2-Infected Hamsters by Prophylactic Administration of Compound I-1

<Materials and Methods>

Compound

Compound I-1 was formulated as a test sample in a 0.5% methylcellulose (0.5% MC) solution. The dose volume was 10 mL/kg.

· Virus

SARS-CoV-2 hCoV-19/Japan/TY41-702/2022 strain (Omicron BE.1/BA.5-like) isolated at the National Institute of Infectious Diseases was used.

· Hamster lung infection, drug administration, and body weight measurement

Specific pathogen-free 6-week-old male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 $\mu$L of hCoV-19/Japan/TY41-702/2022 ($1.00 \times 10^4$ $TCID_{50}$) was intranasally inoculated. In the test on suppression of body weight loss, Compound I-1 was subcutaneously administered once at 3 mg/kg or 10 mg/kg one day or three days before infection. In the test on suppression of viral proliferation in lung and nasal turbinate homogenates, Compound I-1 was subcutaneously administered once at 10 mg/kg or 30 mg/kg one day before viral infection. 0.5% MC was subcutaneously administered to control hamsters once, 1 day prior to viral infection. Body weight was monitored once daily. At 1 and 2 days after infection, the lungs and nasal turbinates of infected hamsters were collected. 5 mL or 1 mL of PBS was added thereto, followed by homogenization, and the supernatant was collected after centrifugation.

· Measurement of viral titers in lung and nasal turbinate homogenates

Lung and nasal turbinate homogenates were subjected to 10-fold serial dilutions in medium (MEM supplemented with 2% FBS and penicillin-streptomycin), mixed with VeroE6/TMPRSS2 cells (JCRB1819, $1.5 \times 10^4$ cells/well), and seeded into 96-well plates. After incubation in a $CO_2$ incubator for 4 days, Cytopathic effects (CPE) were observed, and the viral titers present in the nasal turbinate homogenates were determined.

The compounds according to the present invention were tested essentially as described above. The results are shown in the following. In the infected hamsters, the body weight decreased beginning 3 days after infection and reached a minimum at 6 days after infection. In the Compound I-1-administered groups at doses of 3 and 10 mg/kg one day prior to infection, and at 10 mg/kg three days prior to infection, all hamsters survived until 7 days after infection, and suppression of the body weight loss was observed (Fig. 5).

The viral titers in lung homogenates at 1 day after infection were 5.59-$\log_{10}$ $TCID_{50}$/mL in the 0.5% MC-administered group and 3.34 and 1.84-$\log_{10}$ $TCID_{50}$/mL in the Compound I-1-administered groups at doses of 10 and 30 mg/kg, respectively. At 2 days after infection, the viral titers were 6.18-$\log_{10}$ $TCID_{50}$/mL in the 0.5% MC-administered group and 5.29 and 3.55-$\log_{10}$ $TCID_{50}$/mL in the Compound I-1-administered groups at doses of 10 and 30 mg/kg, respectively (Fig. 6-1).

The viral titers in nasal turbinate homogenates at 1 day after infection were 5.71-$\log_{10}$ $TCID_{50}$/mL in the 0.5% MC-administered group and 4.80 and 3.36-$\log_{10}$ $TCID_{50}$/mL in the Compound I-1-administered groups at doses of 10 and 30 mg/kg, respectively. At 2 days after infection, the viral titers were 5.80-$\log_{10}$ $TCID_{50}$/mL in the 0.5% MC-administered group and 5.38 and 5.01-$\log_{10}$ $TCID_{50}$/mL in the Compound I-1-administered groups at doses of 10 and 30 mg/kg, respectively (Fig. 6-2). When plasma concentrations were measured immediately before infection, the values were 6.1 ng/mL and 12.5 ng/mL in the 3 mg/kg and 10 mg/kg groups administered one day prior to infection, respectively, and 5.9 ng/mL and 3.0 ng/mL in the 3 mg/kg and 10 mg/kg groups administered three days prior to infection, respectively.

The above results indicate that prophylactic administration of Compound I-1 exhibits the effect of suppressing body weight loss at plasma concentrations of about 6 ng/mL or greater at the time of viral infection, and the effect of suppressing viral proliferation in lung and nasal turbinate homogenates at plasma concentration of about 12 ng/mL or greater.

The above results indicate that prophylactic subcutaneous administration of Compound I-1 suppresses progression of disease and viral proliferation associated with viral infection, and support the usefulness of Compound I-1 as a prophylactic agent against SARS-CoV-2 infection.

Test Example 13: Test on Clearance of Viral Titers in Nasal Lavage Fluid of SARS-CoV-2-Infected Hamsters by Delayed Administration of Compound I-1

<Materials and Methods>

Compound

Compound I-1 according to the present invention was used as a test sample using N,N-dimethylacetamide (DMA) and polyethylene glycol 400 (PEG400) containing 0.5 w/v% poly(1-vinylpyrrolidone-co-vinyl acetate) (PVPVA) (DMA:PEG400 containing 0.5 w/v% PVPVA = 1:9). The dose volume was 2.5 mL/kg.

· Virus

SARS-CoV-2 hCoV-19/Japan/TY41-702/2022 strain (Omicron BE.1/BA.5-like) isolated at the National Institute of Infectious Diseases was used.

· Intranasal infection in hamsters, drug administration, and nasal lavage fluid collection

Specific pathogen-free 6-week-old male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 μL of hCoV-19/Japan/TY41-702/2022 ($1.00 \times 10^4$ $TCID_{50}$) was intranasally inoculated. In infected hamsters, oral administration of Compound I-1 was initiated 2 or 3 day after viral inoculation and performed twice daily at doses of 1 and 10 mg/kg. DMA/0.5 w/v% PVPVA in PEG400 was administered to the infected control hamsters twice daily. Compound administration was five days after the start of administration. At 2 to 6 days after infection, infected hamsters were anesthetized with isoflurane, and nasal lavage fluid was collected with 400 μL of DPBS, followed by recovery of the supernatant after centrifugation. Each group consisted of n = 4.

Measurement of viral titers in nasal lavage fluids

Supernatants of lung homogenates were subjected to 10-fold serial dilutions in medium (MEM supplemented with 2% FBS and penicillin-streptomycin), mixed with VeroE6/TMPRSS2 cells (JCRB1819, $1.5 \times 10^4$ cells/well), and seeded into 96-well plates. After incubation in a $CO_2$ incubator for 4 days, Cytopathic effects (CPE) were observed, and the viral titers present in the supernatants of lung homogenates were determined.

The compounds of the present invention were tested essentially as described above. The results are shown in the following.

In the group in which administration of the compound was initiated at 2 days after infection, the viral titers in nasal lavage fluid in the DMA/0.5 w/v% PVPVA in PEG400-administered group and the Compound I-1-administered groups at doses of 1 and 10 mg/kg were 4.80, 4.90, and 4.88-$\log_{10}$ $TCID_{50}$/mL, respectively, at the start of administration (2 days after infection, Day 1 of administration); 3.75, 3.63, and 3.68-$\log_{10}$ $TCID_{50}$/mL, respectively, at 3 days after infection (Day 2 of administration); and 2.96, <1.80, and <1.80-$\log_{10}$ $TCID_{50}$/mL, respectively, at 5 days after infection (Day 4 of administration) (Fig. 14). In the group in which administration of the compound was initiated at 3 days after infection, the viral titers in nasal lavage fluid in the DMA/0.5 w/v% PVPVA in PEG400-administered group and the Compound I-1-administered groups at doses of 1 and 10 mg/kg were 3.40, 2.93, and 3.13-$\log_{10}$ $TCID_{50}$/mL, respectively, at the start of administration (3 days after infection, Day 1 of administration); 2.97, 1.84, and 2.13-$\log_{10}$ $TCID_{50}$/mL, respectively, at 4 days after infection (One day after start of administration); and 2.43, <1.80, and <1.80-$\log_{10}$ $TCID_{50}$/mL, respectively, at 6 days after infection (4 days after start of administration) (Fig. 15).

In the Compound I-1-administered groups at doses of 1 and 10 mg/kg, the viral titers in nasal lavage fluid were reduced compared with those in the DMA/0.5 w/v% PVPVA in PEG400 administration group, irrespective of whether treatment was initiated at 2 or 3 days after infection. These findings indicate that Compound I-1 exerts an in vivo antiviral effect even when administration is initiated after a certain interval following infection.

Test Example 14: Test on Suppression of Viral Transmission From SARS-CoV-2-Infected Animals to Non-Infected Animals by Delayed Administration of Compound I-1

<Materials and Methods>

Compound

Compound I-1 according to the present invention was used as a test sample using N,N-dimethylacetamide (DMA) and polyethylene glycol 400 (PEG400) containing 0.5 w/v% poly(1-vinylpyrrolidone-co-vinyl acetate) (PVPVA) (DMA: PEG400 containing 0.5 w/v% PVPVA = 1:9). The dose volume was 2.5 mL/kg.

· Virus

SARS-CoV-2 hCoV-19/Japan/TY11-927/2021 isolated at the National Institute of Infectious Diseases was used.

· Intranasal infection in hamsters, drug administration, co-housing, nasal lavage fluid collection, and lung collection

Specific pathogen-free 6-week-old male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 μL of hCoV-19/Japan/TY11-927/2021 ($1.00 \times 10^3$ $TCID_{50}$) was intranasally inoculated. In infected hamsters (Index), oral administration of Compound I-1 was initiated 8 hours after viral inoculation and performed twice daily at doses of 0.1, 1, or 10 mg/kg. To the infected control hamsters (Index), DMA/0.5 w/v% PVPVA in PEG400 was orally administered twice daily. Compound administration was carried out a total of three times from the start of administration. From 1 to 2 days after infection, during a 12-hour nighttime period, one infected hamster (Index) and one non-infected hamster (Contact) that had not been inoculated with virus were co-housed in the same cage, while being placed in separate stainless-steel enclosures positioned 2 cm apart to prevent direct contact. After completion of the co-housing period, the hamsters were housed individually. Four days after the initiation of co-housing (3 days after separation), nasal lavage fluid and lungs were collected from the non-infected hamster (Contact). Nasal lavage fluid was collected under isoflurane anesthesia using 400 μL of DPBS, and the supernatant was recovered after centrifugation. The lungs were homogenized in 5 mL of DPBS, and the supernatant was collected after centrifugation. Each group consisted of n = 6.

· Measurement of viral titers in nasal lavage fluid and lung homogenate supernatants

Nasal lavage fluids or supernatants of lung homogenates were subjected to 10-fold serial dilutions in medium (MEM supplemented with 2% FBS and penicillin-streptomycin), mixed with VeroE6/TMPRSS2 cells (JCRB1819, $1.5 \times 10^4$ cells/well), and seeded into 96-well plates. After incubation in a $CO_2$ incubator for 4 days, Cytopathic effects (CPE) were observed, and the viral titers present in the nasal lavage fluids or supernatants of lung homogenates were determined.

The compounds of the present invention were tested essentially as described above. The results are shown in the following.

At 4 days after initiation of co-housing with infected hamsters (Index) (3 days after separation), the numbers of non-infected hamsters (Contact) in which viral titers in nasal lavage fluid or supernatants of lung homogenate reached the detection limit (1.8 $log_{10}$ $TCID_{50}$/mL) or higher were 6/6 in the DMA/0.5 w/v% PVPVA in PEG400-administered group, 6/6 in the Compound I-1 0.1 mg/kg-administered group, 0/6 in the Compound I-1 1 mg/kg-administered group, and 0/6 in the Compound I-1 10 mg/kg-administered group, demonstrating a dose-dependent suppression of viral transmission (Fig. 16). These findings indicate that administration of Compound I-1 to infected hamsters (Index) after infection is effective in suppressing viral transmission to non-infected hamsters (Contact).

Test Example 15: Test on Prevention of Viral Transmission From SARS-CoV-2-Infected Animals by Prophylactic Administration of Compound I-1 to Non-Infected Animals

<Materials and Methods>

Compound

Compound I-1 was formulated as a test sample in a 0.5% methylcellulose (0.5% MC) solution. The dose volume was 5 mL/kg.

· Virus

SARS-CoV-2 hCoV-19/Japan/TY11-927/2021 (Delta) isolated at the National Institute of Infectious Diseases was used.

· Intranasal infection in hamsters, drug administration, co-housing, nasal lavage fluid collection, and lung collection

Specific pathogen-free 6-week-old male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 μL of hCoV-19/Japan/TY11-927/2021 (1.00 $\times$ $10^3$ $TCID_{50}$) was intranasally inoculated. Compound I-1 was subcutaneously administered to non-infected hamsters (Contact) at doses of 3, 10, 30, or 90 mg/kg as a single dose 12 hours prior to co-housing. Non-infected control hamsters (Contact) received a single subcutaneous administration of 0.5% MC. From 1 to 2 days after infection in infected hamsters (Index), during a 12-hour nighttime period, one infected hamster (Index) and one non-infected hamster (Contact) into which the compound was administered were co-housed in the same cage, while being placed in separate stainless-steel enclosures positioned 2 cm apart to prevent direct contact. After completion of the co-housing period, the hamsters were housed individually. Four days after the initiation of co-housing (3 days after separation), nasal lavage fluid and lungs were collected from the non-infected hamster (contact). Nasal lavage fluid was collected under isoflurane anesthesia using 400 μL of DPBS, and the supernatant was recovered after centrifugation. Lungs were supplemented with 3 mL DPBS, homogenized and centrifuged supernatants collected and diluted 2-fold in DPBS. Each group consisted of n = 6.

· Measurement of viral titers in nasal lavage fluid and lung homogenate supernatants

Nasal lavage fluids or supernatants of lung homogenates were subjected to 10-fold serial dilutions in medium (MEM supplemented with 2% FBS and penicillin-streptomycin), mixed with VeroE6/TMPRSS2 cells (JCRB1819, 1.50 $\times$ $10^4$ cells/well), and seeded into 96-well plates. After incubation in a $CO_2$ incubator for 4 days, Cytopathic effects (CPE) were observed, and the viral titers present in the nasal lavage fluids or supernatants of lung homogenates were determined.

The compounds of the present invention were tested essentially as described above. The results are shown in the following.

At 4 days after initiation of co-housing with infected hamsters (Index) (3 days after separation), the numbers of non-infected hamsters (Contact) to which Compound was administered and in which viral titers in nasal lavage fluid or supernatants of lung homogenate reached the detection limit (1.8 $\log_{10}$ $TCID_{50}$/mL) or higher were 6/6 in the 0.5% MC-administered group, 5/6 in the Compound I-1 3 mg/kg-administered group, 6/6 in the Compound I-1 10 mg/kg-administered group, 3/6 in the Compound I-1 30 mg/kg-administered group, and 0/6 in the Compound I-1 90 mg/kg-administered group, demonstrating a dose-dependent preventive effect against infection (Fig. 17). These findings indicate that prophylactic administration of Compound I-1 is effective in preventing viral transmission from infected hamsters (Index).

Test Example 16: Test on Suppression of Mortality and Body Weight Loss in SARS-CoV-2-Infected Aged Hamsters by Delayed Administration of Compound I-1

<Materials and Methods>

Compound

Compound I-1 according to the present invention was used as a test sample using N,N-dimethylacetamide (DMA) and polyethylene glycol 400 (PEG400) containing 0.5 w/v% poly(1-vinylpyrrolidone-co-vinyl acetate) (PVPVA) (DMA: PEG400 containing 0.5 w/v% PVPVA = 1:9). The dose volume was 1.25 mL/kg.

· Virus

SARS-CoV-2 hCoV-19/Japan/TY11-927/2021 isolated at the National Institute of Infectious Diseases was used.

· Intranasal infection in hamsters, drug administration, co-housing, nasal lavage fluid collection, and lung collection

Specific pathogen-free 11-month-old (aged) male Syrian hamsters (Japan SLC, Inc.) were used in this study. For viral inoculation, the hamsters were anesthetized by subcutaneous administration of an 3 mL/kg anesthetic solution containing 0.07 mg/mL medetomidine hydrochloride, 6.98 mg/mL alfaxalone, and 1.16 mg/mL butorphanol tartrate. Subsequently, 100 μL of hCoV-19/Japan/TY11-927/2021 (1.00 $\times$ $10^4$ $TCID_{50}$) was intranasally inoculated. Oral administration of Compound I-1 was initiated 1 day after viral inoculation and performed twice daily at doses of 0.1, 1, and 10 mg/kg. DMA/0.5 w/v% PVPVA in PEG400 was administered to the infected control hamsters twice daily. Compound administration was five days after the start of administration. The body weight was monitored once per day, and for

purposes of survival assessment, animals were deemed to have died when their body weight fell below 80% of the body weight immediately before infection. Each group consisted of n = 6.

The compounds of the present invention were tested essentially as described above. The results are shown in the following.

In the group of infected hamsters to which DMA/0.5 w/v% PVPVA in PEG400 was administered, two hamsters became moribund and died at 8 days after infection, yielding a survival rate of 66.7%. In the administration groups of Compound I-1 at 1 and 10 mg/kg, all hamsters survived until 10 days after infection, and suppression of the body weight loss was observed (Figs. 18 and 19).

The above results suggest that administration of Compound I-1 in aged hamsters exerts the effect of suppressing mortality and body weight loss.

In rodents, aging has been reported to increase expression of the ACE2 receptor, known to serve as a receptor for SARS-CoV-2, as in humans (Scientific Reports (2020) 10:22401; Molecular Therapy Methods & Clinical Development, Vol. 18, pp. 1-6, 2020). Furthermore, since aging is associated with a decline in normal immune responses that protect against and eliminate infectious agents, aged rodents are presumed to be more prone to severe disease following viral infection. As shown above, in a SARS-CoV-2 infection model employing aged hamsters, administration of Compound I-1 exhibited suppressive effects on body weight loss and mortality. Non-clinical test using aged hamsters is regarded as a non-clinical evaluation system that models progression to exacerbation in high-risk individuals with underlying medical conditions. Therefore, the results support the usefulness of Compound I-1 as a therapeutic option for individuals at elevated risk of exacerbation.

In addition, the above test results suggest that in the Compound I-1-administered group, exacerbation associated with viral infection was suppressed, thereby supporting the usefulness of Compound I-1 not only as an antiviral agent against SARS-CoV-2 but also as a pharmaceutical for preventing exacerbation of SARS-CoV-2 infection.

Test Example 17: In Vitro Combined Effects Confirmation Test

<Operational Procedure>

Test samples

Ensitrelvir fumarate, nirmatrelvir, remdesivir, EIDD-1931, sotrovimab, and tixagevimab/cilgavimab were employed as samples to be tested, used in combination with Compound I-1.

· Dilution and dispensing of sample to be tested

Each test sample was diluted to an appropriate concentration using DMSO or DPBS and medium (MEM, 2% FBS, penicillin-streptomycin), and serial dilutions were prepared in a 96-well plate.

· Dilution and dispensing of cells and SARS-CoV-2

A549/ACE2-TMPRSS2 cells (Invivogen, a549-hace2tpsa, $1.5\times10^4$ cells/well) and SARS-CoV-2 hCoV-19/Japan/TY11-927/2021(100 $TCID_{50}$/well) were mixed in a medium (MEM, 2% FBS, penicillin-streptomycin), the mixture was dispensed into the wells in which the sample to be tested has been introduced, and then the cells are cultured for 2 days in a $CO_2$ incubator.

· Dispensing of CellTiter-Glo (registered trademark) 2.0 and measurement of luminescent signals

The plate that had been cultured for 3 days was returned to room temperature, subsequently CellTiter-Glo (registered trademark) 2.0 was dispensed into each well, and the plate was mixed using a plate mixer. After standing for a predetermined period of time, the luminescent signal (Lum) was measured using a plate reader.

<Analysis of Combined Effects>

Synegy and Antagonism volumes were determined using MacSynergy II. The calculation of these values may be performed in accordance with methods described in the literature, such as Antiviral Research, 1990, Vol. 14, pp. 181-206.

Determination of combined effects

The combined effect was determined on the criteria shown below for Synegy volume and Antagonism volume at

99% confidence of MacSynergy II.

synergy volume ≦ 25: additive

25 < synergy volume ≦ 50: minor synergy

50 < synergy volume ≦ 100: moderate synergy

100 < synergy volume: strong synergy

-25 ≦ antagonism volume: additive

-50 ≦ antagonism volume < -25: minor antagonism

-100 ≦ antagonism volume < -50: moderate antagonism

antagonism volume < -100: strong antagonism

The compounds of the present invention were tested essentially as described above. The results are shown in the table below.

[Table 9]

| Substance | Study No. | Synergy volume / Combination effect | Antagonism volume / Combination effect | Summarized combination effect |
|---|---|---|---|---|
| Compound I-1 + Ensitrelvir fumaric acid | 1 | 71.60 / moderate | -5. 72 / additive | additive to moderate synergy |
| | 2 | 56.67 / moderate | -2.08 / additive | |
| | 3 | 12.12 / additive | -12.20 / additive | |
| Compound I-1 + Nirmatrelvir | 1 | 52.02 / moderate | -11.22 / additive | additive to strong synergy |
| | 2 | 114.85 / strong | -9.32 /additive | |
| | 3 | 14.11 / additive | -7.74 / additive | |
| Compound I-1 + Remdesivir | 1 | 200.70 / strong | -1.42 / additive | moderate to strong synergy |
| | 2 | 234.06 / strong | -1.13 / additive | |
| | 3 | 72.57 / moderate | -1.64 / additive | |
| Compound I-1 + EIDD-1931 | 1 | 117.17 / strong | -0.32 / additive | additive to strong synergy |
| | 2 | 162.37 / strong | -1.13 / additive | |
| | 3 | 24.75 / additive | -19.65/ additive | |
| Compound I-1 + Sotrovimah | 1 | 97.51 / moderate | 0 / additive | moderate to strong synergy |
| | 2 3 | 121.56 / strong | -0.68 / additive | |
| | | 85.18 / moderate | -3. 20 / additive | |
| Compound I-1 + Tixagevimab/cilgavimab | 1 | 59.85 / moderate | -7.30 / additive | additive to strong synergy |
| | 2 | 114.04 / strong | -11.85 / additive | |
| | 3 | 6.65 / additive | -13.82 / additive | |

The above results demonstrate that combinations of Compound I-1 with an RNA-dependent RNA polymerase inhibitor (EIDD-1931 or remdesivir), a 3CL protease inhibitor (ensitrelvir or nirmatrelvir), or an anti-SARS-CoV-2 monoclonal antibody (sotrovimab or tixagevimab/cilgavimab) produced additive to synergistic suppression of SARS-CoV-2 proliferation without exhibiting antagonism.

The preparation examples shown below are only for illustrative purposes and are by no means intended to limit the scope of the invention.

The compound according to the present invention can be administered as a pharmaceutical composition by any

conventional route, particularly enterally, for example, orally, for example, in the form of a tablet or a capsule; parenterally, for example, in the form of an injectable preparation or a suspension; and topically, for example, in the form of a lotion, a gel, an ointment or a cream, or as a pharmaceutical composition in a transnasal form or a suppository form. A pharmaceutical composition comprising the compound according to the present invention in a free form or in the form of a pharmaceutically acceptable salt together with at least one pharmaceutically acceptable carrier or diluent can be produced by a mixing, granulating, or coating method in a conventional manner. For example, oral compositions can be a tablet, a granular preparation, or a capsule, each containing a filler, a disintegrating agent, a binder, a lubricant, and the like, as well as an active ingredient and the like. Furthermore, the composition for injection can be prepared as a solution or a suspension, may be sterilized, and may contain a preservative, a stabilizer, a buffering agent, and the like.

Industrial Applicability

The compound according to the present invention has inhibitory activity against coronavirus 3CL proteases, and the pharmaceutical composition comprising the compound according to the present invention is useful as a therapeutic agent and/or prophylactic agent for coronavirus infections.

## Claims

1. A pharmaceutical composition comprising a compound represented by Formula (I-1):

[Chemical formula 1]

(I-1)

, or a pharmaceutically acceptable salt thereof.

2. The pharmaceutical composition according to claim 1, wherein the pharmaceutical composition is a 3CL protease inhibitor.

3. The pharmaceutical composition according to claim 1 or 2, wherein the pharmaceutical composition is for use in inhibition of viral proliferation of SARS-CoV-2.

4. The pharmaceutical composition according to any one of claims 1 to 3, wherein the pharmaceutical composition is a therapeutic agent and/or prophylactic agent for novel coronavirus infection (COVID-19).

5. The pharmaceutical composition according to any one of claims 1 to 4, wherein the pharmaceutical composition is for use in suppression of exacerbation of SARS-CoV-2 infection.

6. The pharmaceutical composition according to any one of claims 1 to 4, wherein the pharmaceutical composition is used so that administration is initiated within 72 hours after the onset of symptoms of SARS-CoV-2 infection or after a positive diagnosis of SARS-CoV-2.

7. The pharmaceutical composition according to any one of claims 1 to 4, wherein the pharmaceutical composition is used so that administration is initiated within 24 hours after the onset of symptoms of SARS-CoV-2 infection or after a positive diagnosis of SARS-CoV-2.

8. The pharmaceutical composition according to any one of claims 1 to 4, wherein the pharmaceutical composition is for use in suppression of viral transmission of SARS-CoV-2.

Fig. 1

VIRAL TITER IN MOUSE LUNG HOMOGENATE
Lung viral titter (log10 TCID50/mL)
8.00
7.00
6.00
5.00
4.00
3.00
2.00
1.00
0.00
**
***
***
***
Vehicle
0.1 mg/kg
0.3 mg/kg
1 mg/kg
3 mg/kg
10 mg/kg
COMPOUND (I-1)

p** p < 0.01, *** p < 0.0001 vs vehicle (Dunnett's test)

Fig. 2-1

VIRAL TITER IN HAMSTER LUNG HOMOGENATE
Lung viral titter (log10 TCID50/mL)
7.00
6.00
5.00
4.00
3.00
2.00
1.00
0.00
**
***
***
***
***
Vehicle
0.1 mg/kg
1 mg/kg
10 mg/kg
Vehicle
0.1 mg/kg
1 mg/kg
10 mg/kg
COMPOUND (I-1)
3 DAYS AFTER INFECTION
COMPOUND (I-1)
4 DAYS AFTER INFECTION

** $p < 0.01$, *** $p < 0.0001$ vs vehicle (Dunnett's test)

Fig. 2-2

VIRAL TITER IN HAMSTER NASAL TURBINATE HOMOGENATE
Nasal turbinate viral titter (log10 TCID50/mL)
6.00
5.00
4.00
3.00
2.00
1.00
0.00
**
***
***
***
Vehicle
0.1 mg/kg
1 mg/kg
10 mg/kg
Vehicle
0.1 mg/kg
1 mg/kg
10 mg/kg
COMPOUND(I-1)
COMPOUND(I-1)
3 DAYS AFTER INFECTION
4 DAYS AFTER INFECTION

** $p < 0.01$, *** $p < 0.0001$ vs vehicle (Dunnett's test)

Fig. 3

HAMSTER LUNG WEIGHT/BODY WEIGHT
LUNG WEIGHT/BODY WEIGHT (mg/g)
10.00
9.00
8.00
7.00
6.00
5.00
4.00
3.00
2.00
1.00
0.00
*
***
***
NOT INFECTED
Vehicle
0.1 mg/kg
1 mg/kg
10 mg/kg
COMPOUND (I-1)
* p < 0.05, *** p < 0.0001 vs vehicle (Dunnett's test)

Fig. 4

HAMSTER BODY WEIGHT CHANGE
BODY WEIGHT v.s. Day 0
120.0%
115.0%
110.0%
105.0%
100.0%
95.0%
90.0%
85.0%
80.0%
0
2
4
6
8
10
12
DAYS AFTER INFECTION (DAYS)
NOT INFECTED
Vehicle
COMPOUND(I-1) 0.1 mg/kg
COMPOUND(I-1) 1 mg/kg
COMPOUND(I-1) 10 mg/kg

Fig. 5

HAMSTER BODY WEIGHT CHANGE
BODY WEIGHT v.s. Day 0
110.0%
105.0%
100.0%
95.0%
90.0%
85.0%
80.0%
0
2
4
6
8
DAYS AFTER INFECTION (DAYS)
NOT INFECTED
Vehicle
COMPOUND (I-1) -72hpi 3 mg/kg
COMPOUND (I-1) -72hpi 10 mg/kg
COMPOUND (I-1) -24hpi 3 mg/kg
COMPOUND (I-1) -24hpi 10 mg/kg

Fig. 6-1

VIRAL TITER IN HAMSTER LUNG HOMOGENATE
Lung viral titer ($log_{10}$ $TCID_{50}$/mL)
7.00
6.00
5.00
4.00
3.00
2.00
1.00
0.00
***
***
**
Vehicle
10 mg/kg
30 mg/kg
Vehicle
10 mg/kg
30 mg/kg
COMPOUND (I-1)
COMPOUND (I-1)
1 DAY AFTER INFECTION
2 DAYS AFTER INFECTION

** $p < 0.01$, *** $p < 0.0001$ vs vehicle (Dunnett's test)

Fig. 6-2

VIRAL TITER IN HAMSTER NASAL TURBINATE HOMOGENATE
Nasal turbinate viral titter (log10 TCID50/mL)
7.00
6.00
5.00
4.00
3.00
2.00
1.00
0.00
**
***
*
Vehicle
10 mg/kg
30 mg/kg
Vehicle
10 mg/kg
30 mg/kg
COMPOUND(I-1)
COMPOUND(I-1)
1 DAY AFTER INFECTION
2 DAYS AFTER INFECTION

* $p < 0.05$, ** $P < 0.01$, *** $p < 0.0001$ vs vehicle (Dunnett's test)

Fig. 7

2-theta
5
7.5
10
12.5
15
17.5
20
22.5
25
27.5
30
0
50
100
150
200
250

Fig. 8

| No. | Position (2θ) | Intensity |
|---|---|---|
| 1 | 6.5 | 177.0 |
| 2 | 10.1 | 76.8 |
| 3 | 13.0 | 51.6 |
| 4 | 14.1 | 68.8 |
| 5 | 15.3 | 85.0 |
| 6 | 15.6 | 168.1 |
| 7 | 16.2 | 117.8 |
| 8 | 17.4 | 278.6 |
| 9 | 18.9 | 77.6 |

| No. | Position (2θ) | Intensity |
|---|---|---|
| 10 | 19.9 | 219.1 |
| 11 | 20.3 | 230.8 |
| 12 | 21.7 | 218.4 |
| 13 | 23.0 | 115.1 |
| 14 | 23.8 | 186.3 |
| 15 | 25.8 | 79.7 |
| 16 | 28.8 | 78.1 |
| 17 | 30.6 | 96.0 |

Fig. 9

CL0A
C5BA
C4BA
N0AA
C8AA
C0BA
O1AA
C1AA
C1BA
O0AA
C2AA
N4AA
C3BA
C5CA
C3AA
C6AA
N1AA
C9BA
CL1
N2AA
N3AA
C2BA
C5AA
C0CA
C3CA
C6CA
F9AA
CL7C
C7AA
C2CA
C8BA
F4AA
F6BA

Fig. 10

261.33°C
Heat Flow (W/g)
2
0
-2
-4
-6
Exo Up
-50
0
50
100
150
200
250
300
Temperature (°C)
Universal V4.5A TA Instruments

Fig. 11

TG %
0.00
-5.00
-10.00
-15.00
-20.00
-25.00
-30.00
-35.00
TG
DTA
265.6Cel
21.56uV
50.0
100.0
150.0
200.0
250.0
300.0
Temp Cel
60.00
50.00
40.00
30.00
20.00
10.00
0.00
DTA uV

Fig. 12

Intensity
20000
10000
0
500
1000
1500
2000
2500
3000
Raman shift (cm-1)

Fig. 13

×10 3
2.1
2
1.9
1.8
1.7
1.6
1.5
1.4
1.3
1.2
1.1
1
0.9
0.8
0.7
0.6
0.5
0.4
0.3
0.2
0.1
0
mAU
0
2
4
6
8
10
12
14
16
18
20
22
24
26
28
30
32
34
36
38
40
TIME [min]

| RT [min] | AREA | HEIGHT | PEAK WIDTH (50%) | PEAK AREA% |
|---|---|---|---|---|
| 17.276 | 0.91303 | 0.22189 | 0.0640 | 0.01 |
| 18.705 | 2.28050 | 0.56120 | 0.0633 | 0.03 |
| 20.169 | 0.53358 | 0.10920 | 0.0767 | 0.01 |
| 20.996 | 1.56164 | 0.30532 | 0.0792 | 0.02 |
| 22.022 | 8480.14941 | 1984.69946 | 0.0661 | 96.52 |
| 22.370 | 113.75063 | 23.80575 | 0.0681 | 1.29 |
| 22.527 | 58.68169 | 13.14615 | 0.0683 | 0.67 |
| 22.721 | 91.57609 | 19.05317 | 0.0692 | 1.04 |
| 23.533 | 34.58274 | 8.74096 | 0.0617 | 0.39 |
| 26.482 | 1.41937 | 0.36425 | 0.0593 | 0.02 |

Fig. 14

VIRAL TITER IN NALF WITH ADMINISTRATION
BEGINNING AT 2 DAYS AFTER INFECTION
NALF viral titer ($log_{10}$ $TCID_{50}$/mL)
5.50
5.00
4.50
4.00
3.50
3.00
2.50
2.00
1.50
1.00
1
3
5
7
9
DAYS AFTER INFECTION (DAYS)
Vehicle
COMPOUND I-1 1 mg/kg
COMPOUND I-1 10 mg/kg

Fig. 15

VIRAL TITER IN NALF WITH ADMINISTRATION BEGINNING AT 3 DAYS AFTER INFECTION
NALF viral titer ($log_{10}$ $TCID_{50}$/mL)
4.00
3.50
3.00
2.50
2.00
1.50
1.00
1
3
5
7
9
DAYS AFTER INFECTION (DAYS)
Vehicle
COMPOUND I-1 1 mg/kg
COMPOUND I-1 10 mg/kg

Fig. 16

VIRAL TITER IN ORGAN OF Contact HAMSTER AT
4 DAYS AFTER INITIATION OF CO-HOUSING
Lung
NALF
LLOQ
Viral titer ($log_{10}$ $TCID_{50}$/mL)
7.00
6.00
5.00
4.00
3.00
2.00
1.00
0.00
1 2 3 4 5 6
1 2 3 4 5 6
1 2 3 4 5 6
1 2 3 4 5 6
Vehicle
0.1 mg/kg
1 mg/kg
10 mg/kg
COMPOUND I-1

Fig. 17

VIRAL TITER IN ORGAN OF Contact HAMSTER AT
4 DAYS AFTER INITIATION OF CO-HOUSING
Lung
NALF
LLOQ
Viral titer ($log_{10}$ $TCID_{50}$/mL)
8.00
7.00
6.00
5.00
4.00
3.00
2.00
1.00
0.00
1 2 3 4 5 6
1 2 3 4 5 6
1 2 3 4 5 6
1 2 3 4 5 6
1 2 3 4 5 6
Vehicle
3 mg/kg
10 mg/kg
30 mg/kg
90 mg/kg
COMPOUND I-1

Fig. 18

SURVIVAL RATE OF AGED HAMSTER
SURVIVAL RATE
120.0%
100.0%
80.0%
60.0%
40.0%
20.0%
0.0%
0
2
4
6
8
10
12
DAYS AFTER INFECTION (DAYS)
NOT INFECTED
Vehicle
COMPOUND(I-1) 1 mg/kg
COMPOUND(I-1) 10 mg/kg

Fig. 19

AGED HAMSTER BODY WEIGHT CHANGE
BODY WEIGHT v.s. Day 0
105.0%
100.0%
95.0%
90.0%
85.0%
80.0%
0
2
4
6
8
10
12
DAYS AFTER INFECTION (DAYS)
NOT INFECTED
Vehicle
COMPOUND (I-1) 1 mg/kg
COMPOUND (I-1) 10 mg/kg

**INTERNATIONAL SEARCH REPORT**

International application No. **PCT/JP2024/035560**

**A. CLASSIFICATION OF SUBJECT MATTER**

***A61K 31/513***(2006.01)i; ***A61K 31/506***(2006.01)i; ***A61P 31/14***(2006.01)i; ***A61P 43/00***(2006.01)i; ***C07D 401/14***(2006.01)i
FI: A61K31/513 ZNA; A61K31/506; A61P31/14; A61P43/00 111; C07D401/14

According to International Patent Classification (IPC) or to both national classification and IPC

**B. FIELDS SEARCHED**

Minimum documentation searched (classification system followed by classification symbols)

A61K31/513; A61K31/506; A61P31/14; A61P43/00; C07D401/14

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2022/138987 A1 (SHIONOGI & CO., LTD.) 30 June 2022 (2022-06-30) claims | 1-8 |
| A | WO 2022/138988 A1 (SHIONOGI & CO., LTD.) 30 June 2022 (2022-06-30) claims | 1-8 |
| A | JP 2023-519035 A (PFIZER INC.) 10 May 2023 (2023-05-10) claims, paragraph [0001] | 1-8 |
| A | JP 2022-534186 A (PFIZER INC.) 28 July 2022 (2022-07-28) claims, paragraph [0001] | 1-8 |
| P, X | WO 2023/195529 A1 (SHIONOGI & CO., LTD.) 12 October 2023 (2023-10-12) example 6 (I-077), test exmaples 1-2, tables 16, 92, 96 | 1-8 |
| P, X | WO 2023/195530 A1 (SHIONOGI & CO., LTD.) 12 October 2023 (2023-10-12) example 6 (I-077), test exmaples 1-2, tables 3, 5 | 1-8 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 October 2024** | **12 November 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/035560**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/035560**

| Patent document cited in search report | Publication date (day/month/year) | Patent family member(s) | Publication date (day/month/year) |
|---|---|---|---|
| WO 2022/138987 A1 | 30 June 2022 | US 2023/0128162 A1<br>claims<br>EP 4122926 A1<br>CN 115038696 A | |
| WO 2022/138988 A1 | 30 June 2022 | US 2023/0212154 A1<br>claims<br>KR 10-2022-0142999 A | |
| JP 2023-519035 A | 10 May 2023 | US 2022/0017548 A1<br>claims, paragraph [0003]<br>KR 10-2021-0151949 A<br>CN 114641485 A<br>WO 2021/205298 A1 | |
| JP 2022-534186 A | 28 July 2022 | US 2022/0062232 A1<br>claims, paragraph [0001]<br>WO 2021/250648 A1<br>EP 4166539 A1<br>KR 10-2022-0031547 A<br>CN 114466838 A | |
| WO 2023/195529 A1 | 12 October 2023 | CN 117203195 A<br>example 6 (I-077), test exmaples 1-2, tables 16, 92, 96 | |
| WO 2023/195530 A1 | 12 October 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021205298 A
- WO 2021250648 A
- WO 2012020742 A
- WO 2013118855 A
- CN 113620888
- CN 113666914
- CN 113735838 A
- CN 113773300
- CN 113801097
- WO 2022138987 A
- WO 2022138988 A
- WO 2023054292 A
- WO 2023054732 A
- WO 2023195529 A
- WO 2023195530 A


### Non-patent literature cited in the description


- *The NEW ENGLAND JOURNAL of MEDICINE*, 2020, vol. 382, 1564-1567
- Report of the WHO-China Joint Mission on Coronavirus Disease 2019 (COVID-19). WHO, 28 February 2020
- *Science*, 2003, vol. 300, 1763-1767
- A comparative analysis of SARS-CoV-2 antivirals characterizes 3CLpro inhibitor PF-00835231 as a potential new treatment for COVID-19. *Journal of Virology*, 10 March 2021, vol. 95 (7), e01819-20
- *Cell Research*, 2020, vol. 30, 678-692
- *Science*, 2020, vol. 368, 409-412
- *ACS Central Science*, 2021, vol. 7 (3), 467-475
- *261st Am Chem Soc (ACS) Natl Meet*, 05 April 2021
- *Science*, 2021, vol. 374, 1586-1593
- Pfizer's Novel COVID-19 Oral Antiviral Treatment Candidate Reduced Risk Of Hospitalization Or Death By 89% In Interim Analysis Of Phase 2/3 EPIC-HR Study. Pfizer Press, 05 November 2021
- *Synthetic Communications*, 2006, vol. 36 (19), 2913-2920
- Discovery and Development of PBI-0451. *35th International Conference on Antiviral Research (ICAR)*, 24 March 2022, https://ir.pardesbio.com/-static-files/fc7c4f8c-e0bd-4b97-8c9c-eff09bafd4db
- *Molecules*, 2020, vol. 25, 3193
- *Molecules*, 2020, vol. 25, 3920
- *European Journal of Medicinal Chemistry*, 2020, vol. 206, 112711
- *Journal of the American Chemical Society*, 2022, vol. 144, 2905-2920
- Labeled Compounds (Part A). Isotopes in the Physical and Biomedical Sciences. 1987, vol. 1
- Design of Prodrugs. Elsevier, 1985
- **TOSHIO SAKURAI**. Guide to X-ray Structure Analysis. Shokabo Publishing, 1983
- **STOUT** ; **JENSEN**. X-Ray Structure Determination: A Practical Guide. Macmillan Co., 1968
- **ATHERTON, E.** ; **SHEPPARD, R. C.** In Solid Phase Peptide Synthesis, A Practical Approach. IRL Press at Oxford University Pres, 1989
- *Bioorg.Med.Chem.*, 1997, vol. 5 (9), 1883-1891
- *Scientific Reports*, 2020, vol. 10, 22401
- *Molecular Therapy Methods & Clinical Development*, 2020, vol. 18, 1-6
- *Antiviral Research*, 1990, vol. 14, 181-206